# EUROPEAN PATENT APPLICATION

(11) **EP 4 212 166 A1**
(43) Date of publication of application: **19.07.2023**
(21) Application number: 22188250.9
(22) Date of filing: 02.12.2016
(51) Int. Cl.: A61K 38/04, C07K 16/28, A61K 38/17, C07K 16/30, C07K 7/04

(54) **COMPOSITIONS AND METHODS FOR REDUCING IMMUNE RESPONSES AGAINST CELL THERAPIES**

(30) Priority: 03.12.2015 US 201562262912 P; 09.06.2016 US 201662348126 P
(62) Divisional of application: 16825929.9
(71) Applicant: Juno Therapeutics, Inc., Seattle, WA 98109 (US)
(72) Inventor: BONYHADI, Mark L., Seattle, WA 98109 (US)
(74) Representative: Carpmaels & Ransford LLP

(57) **Abstract**

Provided are methods of administering peptides containing portions of or corresponding to an agent to be administered, such as a cell or molecule expressed by such cell such as a recombinant protein such as a chimeric receptor. In some embodiments, the administration is in conjunction with treatment methods employing the agent, e.g., cell or chimeric receptor, such as adoptive cell therapy methods. The chimeric receptor can be a chimeric antigen receptor (CAR). The peptides contain an epitope of the recombinant receptor and, in some embodiments, are capable of downregulating or reducing immune responses against the chimeric receptor, such as CAR. Also provided are such peptides and compositions that can induce tolerance to an agent such as a chimeric receptor, such as a CAR.

## Description

### Cross-Reference to Related Applications

This application claims priority from U.S. provisional application No. 62/262,912, filed December 3, 2015, entitled "Compositions and Methods for Reducing Immune Responses Against Cell Therapies," and U.S. provisional application No. 62/348,126, filed June 9, 2016, entitled "Compositions and Methods for Reducing Immune Responses Against Cell Therapies," the contents of each of which is incorporate by reference in its entirety.

### Incorporation by Reference of Sequence Listing

An electronic version of the Sequence Listing is provided herewith as a text file, the contents of which are incorporated by reference in their entirety. The electronic file was created on December 2, 2016, is 71,009 bytes in size, and is titled
735042001540SEQLIST.txt.

### Field

The present disclosure relates to peptides and methods of administering such peptides in conjunction with administering a therapeutic agent, such as an engineered cell expressing recombinant protein, such as a chimeric receptor to a subject, such as in conjunction with administering cells expressing a genetically engineered or recombinant protein, which is generally a recombinant receptor, such as a recombinant antigen receptor, a recombinant chimeric receptor. The chimeric receptor may include a chimeric antigen receptor (CAR). The isolated peptides generally contain one or more epitope of the chimeric receptor and, in some embodiments, the methods are capable of downregulating or reducing immune responses against the chimeric receptor, such as a CAR, expressed by the cells. Among the provided peptides and compositions are those that can induce tolerance to the cells or recombinant protein, such as the CAR, and/or to cells genetically engineered with the chimeric receptor, for example, in a subject to which the cells or protein is administered, e.g., is later administered. Features of the methods provide various advantages for adoptive cell therapies, such as improved efficacy and in some aspects the potential avoidance or reduction of the use of more toxic immunosuppressive approaches, such as chemotherapy. For example, the peptides, compositions and methods can minimize the risk of reduced exposure to cells expressing the chimeric receptors, which can otherwise result due to specific immune responses to the chimeric receptor in the subject.

### Background

Various methods are available for adoptive cell therapy using engineered cells expressing recombinant receptors, such as chimeric antigen receptor (CARs). Improved methods are needed, for example, to improve efficacy of such therapies, for example, by increasing exposure of the subject to the administered cells. Such methods are needed, for example, that can improve expansion and/or persistence of the administered cells, reduce the immune response to the receptors expressed on the cells or reduce other unwanted outcomes. Provided are methods, compositions, and articles of manufacture that meet such needs.

### Summary

In some embodiments, provided herein is a method of preventing or reducing or reducing risk of an immune response against an agent, or inducing tolerance to the agent, such as a therapeutic agent such as an engineered cell or molecule expressed therein or thereon or thereby such as a recombinant molecule, e.g., a receptor, e.g., an antigen and/or chimeric receptor. The methods generally include administering to a subject at least one peptide containing all or a portion of the agent or molecule, such as an immunogenic region thereof, such as of the chimeric receptor. In some embodiments, a plurality of peptides each containing all of a portion of the agent or molecule is administered. The peptide or plurality of peptides generally is administered under conditions that induces tolerance in the subject to the agent, e.g., to the molecule such as the chimeric receptor or cells expressing it. In some embodiments, the molecule such as the chimeric receptor is expressed by cells genetically engineered with nucleic acid encoding the chimeric receptor.

In some embodiments, the method further includes administering to the subject the agent, e.g., the chimeric receptor, and/or administering cells expressing the same, e.g., expressing the chimeric receptor, for treating a disease or condition in the subject. In some embodiments, the chimeric receptor is a chimeric antigen receptor.

In some embodiments, also provided herein is a method of treatment includes administering to a subject a peptide(s) containing all or a portion of an immunogenic region of the agent or portion thereof such as the chimeric antigen receptor, said peptide(s) being administered under conditions that induce tolerance in the subject to the agent or portion thereof e.g., chimeric antigen receptor; and administering to the subject cells expressing the chimeric antigen receptor, wherein the chimeric antigen receptor specifically binds to an antigen associated with a disease or condition in the subject. In some embodiments, the peptide(s) is administered prior to, subsequently or intermittently from administration of the chimeric receptor. In some embodiments, the peptide(s) is administered prior to administration of the chimeric receptor. In some embodiments, the peptide(s) is administered from or from about 7 to 28 days before administration of the chimeric receptor.

In some embodiments, the peptide(s) is administered in the absence of an adjuvant.

In some embodiments, the method further includes administering a tolerogenic agent. In some embodiments, the tolerogenic agent targets and/or specifically binds to, and optionally induces or reduces or agonizes or antagonizes signaling by or mediated by, one or more molecules that is expressed on T cells or other immune cells, such as a molecule selected from among CD4, CD40, CD40L, OX40, OX40L, CD26, CD44, CD28, PD1, BTLA, B7-1, ICOS, CTLA-4, B7-2 family, CD99, CD137 (4-1BBL), CD2, LFA3, CD27, CD70, CD3, CD8, ICAM1, LFA1, MHC class II and MHC class I molecule. In some embodiments, the tolerogenic agent targets and/or specifically binds to, and optionally induces signaling by or mediated by, one or more molecules that is expressed on a non-T cell, such as an antigen presenting cell (APC), e.g., a macrophage or a dendritic cell, e.g., a cell that serve as the ligand/receptor pair for any of the aforementioned molecules identified above expressed on the T cells, such as PD-L1, PD-L2 and B7 family members.

In some embodiments, the tolerogenic agent is an antibody or fragment thereof or is a soluble fusion protein. In some embodiments, for example, in which the tolerogenic agent is also administered, the peptide(s) is administered in the presence of adjuvant. In some embodiments, it is not administered in the presence of an adjuvant. In some embodiments, the peptide(s) is administered by, intravenous, intraperitoneal, intranasal, oral or intrathymic administration. In some embodiments, the peptide(s) is administered by intraperitoneal injection. In some embodiments, the peptide(s) is administered through implantation of a subcutaneous or similar pump (such as an Alzet pump), or through a topical patch. In some embodiments, the peptide(s) is administered in an amount that from or from about is 0.1 mg and 1000 mg. In some embodiments, the peptide(s) is administered in an amount that is from or from about 100 mg to 1000 mg. In some embodiments, the peptide(s) is administered in an amount that is from or from about 0.1 mg to 5 mg.

In some embodiments, the peptide(s) is administered a plurality of times by repeated administration. In some embodiments, the peptide(s) is administered at least two times, at least three times, at least four times, at least five times, at least six times or at least seven times. In some embodiments, the peptide(s) is administered at least once daily for two days, three days, four days, five days, six days or seven days. In some embodiments, each administration of the peptide(s) occurs prior to administration of the agent, e.g., the engineered cell or chimeric receptor.

In some embodiments, the peptide(s) includes a plurality of peptides, each of said peptides including all or a portion of an immunogenic region of the molecule or engineered cell or molecule expressed therein or thereon or thereby such as a recombinant molecule, e.g., a receptor, e.g., an antigen and/or chimeric receptor. In some embodiments, the plurality of peptides are peptides of two or more different immunogenic regions of said chimeric receptor. In some embodiments, the plurality of peptides are peptides of the same immunogenic region of said chimeric receptor, each of said peptides overlapping in sequence and including a portion of said immunogenic region. In some embodiments, the plurality of peptides includes at least two, three, four, five, six, seven, eight, nine or ten different peptides.

In some embodiments, the immunogenic region includes a T cell epitope. In some embodiments, the T cell epitope is an MHC class I or an MHC class II epitope. In some embodiments, the T cell epitope is an MHC class I epitope. In some embodiments, the subject is positive for an HLA allele that specifically binds to the administered peptide. In some embodiments, the method includes, prior to administering the peptide, selecting a subject that is positive for an HLA allele that specifically binds or is known to bind well to the administered peptide. In some embodiments, the HLA allele is selected from among HLA-A^{∗}02:01, HLA-A^{∗}03:01, HLA-A^{∗}11:01 and HLA-B^{∗}08:01. In some embodiments, the immunogenic region includes a region within one or more portions selected from the group consisting of an scFv portion, a linker portion, an amino acid sequence not endogenous to the subject, a sequence derived from a different species than that of the subject, and/or a junction between two CAR domains; and/or where the region of the chimeric receptor is a junction region including amino acids on each side of a junction between two domains.

In some embodiments, the region includes a framework region (FR) within the scFv portion, the region includes a heavy chain FR sequence, the region includes a heavy chain CDR sequence, the region includes a light chain FR sequence, and/or the region includes a light chain CDR sequence.

In some embodiments, the immunogenic region includes a contiguous sequence of amino acids of a junction region of the chimeric receptor, wherein the junction region includes up to 15 contiguous amino acids directly C-terminal of a junction that joins a first domain and a second domain of the chimeric receptor and/or up to 15 contiguous amino acids directly N-terminal of the junction, and optionally further includes the junction. In some embodiments, the first domain and second domain are directly linked or are indirectly linked via a linker or linkers. In some embodiments, each of the first and second domain include domain sequences of a protein derived from the same species as the subject and/or domain sequences of a protein endogenous to the subject.

In some embodiments, the subject is a human.

In some embodiments, the peptide or each of the peptides includes a contiguous sequence of amino acids of the junction region. In some embodiments, the peptide or each of the peptides individually is between 7 and 30 amino acids, 8 and 20 amino acids, 8 and 15 amino acids, 10 and 17 amino acids, 7 and 13 amino acids or 8 and 10 amino acids. In some embodiments, the peptide is between 10 and 17 amino acids. In some embodiments, the peptide or each of the peptides individually is or is about 10 amino acids, 11 amino acids, 12 amino acids, 13 amino acids, 14 amino acids, 15 amino acids, 16 amino acids or 17 amino acids.

In some embodiments, the chimeric receptor is a CAR and the CAR includes an extracellular antigen-recognition domain that specifically binds to a target antigen and an intracellular signaling domain including an activating cytoplasmic signaling domain. In some embodiments, the extracellular antigen-recognition domain includes an antibody or antigen-binding fragment. In some embodiments, the extracellular antigen-recognition domain includes an scFv.

In some embodiments, the activating cytoplasmic signaling domain includes a T cell receptor (TCR) component and/or includes an immunoreceptor tyrosine-based activation motif (ITAM); and/or the activating cytoplasmic signaling domain includes an intracellular domain of a CD3-zeta (CD3ζ) chain. In some embodiments, the method further includes a transmembrane domain linking the extracellular domain and the intracellular signaling domain. In some embodiments, the transmembrane domain includes a transmembrane portion of CD28. In some embodiments, the intracellular signaling domain further includes an intracellular costimulatory signaling domain of a T cell costimulatory molecule. In some embodiments, the T cell costimulatory molecule is selected from the group consisting of CD28 and 41BB.

In some embodiments, the first domain and second domain are, respectively, an extracellular ligand binding domain and a hinge domain, an extracellular ligand binding domain and a transmembrane domain, a transmembrane domain and an intracellular costimulatory signaling domain, and an intracellular costimulatory signaling domain and an ITAM signaling domain.

In some embodiments, the first domain is a transmembrane domain or a functional portion thereof and the second domain is a costimulatory signaling domain or a functional portion thereof. In some embodiments, the transmembrane domain is a CD28 transmembrane domain or a functional portion or variant thereof and the costimulatory signaling domain is a 4-1BB signaling domain or a functional portion or variant thereof. In some embodiments, the CD28 transmembrane domain includes the sequence of amino acids set forth in SEQ ID NO: 2, 103 or 104 or a functional portion or variant thereof including a sequence that exhibits at least 95% sequence identity to SEQ ID NO: 2, 103 or 104; and the 4-1BB costimulatory signaling domain includes the sequence of amino acids set forth in SEQ ID NO:3 or a functional portion or variant thereof including a sequence that exhibits at least 95% sequence identity to SEQ ID NO:3.

In some embodiments, the first domain and second domain together include the sequence of amino acids set forth in SEQ ID NO:5 or a functional portion or variant thereof including a sequence of amino acids that exhibits at least 85%, 86%, 87%, 88%, 89%, 90%, 91%, 92%, 93%, 94%, 95%, 96%, 97%, 98% or 99% sequence identity to SEQ ID NO:5. In some embodiments, the first domain and second domain together include the sequence of amino acids set forth in SEQ ID NO:5.

In some embodiments, the peptide or one or more peptides of the plurality of peptides include i) a sequence of amino acids set forth in any of SEQ ID NOS: 6, 7, 8-12, 16-22, 137, 160, 163, 165, 166, 167 or 168 or a variant thereof that retains activity to induce tolerance; or ii) a peptide epitope of a sequence of i) that binds to an HLA molecule.

In some embodiments, the peptide(s) has a binding affinity for a human leukocyte antigen (HLA) molecule that is less than 1000 nM, less than 500 nM or less than 50 nM. In some embodiments, the binding affinity is an IC50. In some embodiments, the peptide binds an MHC class I and the HLA allele is selected from among HLA-A^{∗}02:01, HLA-A^{∗}03:01, HLA-A^{∗}11:01 and HLA-B^{∗}08:01. In some embodiments, the peptide binds an MHC class II and the HLA allele comprises an alpha and/or beta chain selected from HLA-DPA1^{∗}0103, HLA-DPA1^{∗}0201, HLA-DPB1^{∗}0101, HLA-DPB1^{∗}0301, HLA-DPB1^{∗}0401, HLA-DPB^{∗}0402, HLA-DPB1^{∗}1501, HLA-DRA^{∗}0101, HLA-DRB1^{∗}1101.

In some embodiments, the peptide(s) is administered no more than one week prior to administering the chimeric receptor. In some embodiments, the method further includes administering an immunosuppressive compound. In some embodiments, the chimeric antigen receptor specifically binds to an antigen associated with the disease or condition. In some embodiments, the disease or condition is a cancer, and autoimmune disease or disorder, or an infectious disease.

In some embodiments, provided herein is use of a peptide(s) for formulation of a medicament for reducing an immune response associated with treatment with a chimeric receptor, wherein said peptide(s) is formulated for administration to induce tolerance in a subject to the chimeric receptor. In some embodiments, provided herein is a pharmaceutical composition including a peptide(s) for use in reducing an immune response associated with treatment with a chimeric receptor, wherein said peptide(s) is formulated for administration to induce tolerance in a subject to the chimeric receptor. In some embodiments, the peptide(s) is formulated for administration in the absence of an adjuvant.

In some embodiments, provided herein is use of a chimeric receptor for formulation of a medicament for treating a disease or condition in a subject that has received the peptide or a plurality of peptides to induce tolerance to the chimeric receptor. In some embodiments, a provided herein is a pharmaceutical composition including a cell, such as an engineered cell expressing the molecule expressed therein or thereon or thereby such as a recombinant molecule, e.g., a receptor, e.g., an antigen and/or chimeric receptor for use in treating a disease or condition in a subject that has received the peptide or a plurality of peptides to reduce tolerance to the chimeric receptor. In some embodiments, the chimeric receptor is a chimeric antigen receptor. In some embodiments, the chimeric receptor is expressed by cells genetically engineered with nucleic acid encoding the chimeric receptor. In some embodiments, the chimeric receptor binds to an antigen associated with the disease or condition. In some embodiments, the disease or condition is a cancer, and autoimmune disease or disorder, or an infectious disease.

In some embodiments, provided herein is an isolated peptide including a contiguous sequence of amino acids of a junction region of a chimeric receptor, wherein the junction region includes up to 15 contiguous amino acids directly C-terminal of a junction that joins a first domain of the chimeric receptor and a second domain of the chimeric receptor and/or up to 15 amino acids directly N-terminal of the junction, and optionally further includes the junction. In some embodiments, the first domain and second domain are directly linked or are indirectly linked via a linker or linkers. In some embodiments, each of the first and second domain include domain sequences of a protein derived from the same species as the subject and/or domain sequences of a protein endogenous to the subject. In some embodiments, the first domain and second domain are, respectively, an extracellular ligand binding domain and a hinge domain, an extracellular ligand binding domain and a transmembrane domain, a transmembrane domain and an intracellular costimulatory signaling domain, and an intracellular costimulatory signaling domain and an ITAM signaling domain. In some embodiments, the first domain is a transmembrane domain or a functional portion thereof and the second domain is a costimulatory signaling domain or a functional portion thereof.

In some embodiments, the transmembrane domain is a CD28 transmembrane domain or a functional portion or variant thereof and the costimulatory signaling domain is a 4-1BB signaling domain or a functional portion or variant thereof. In some embodiments, the CD28 transmembrane domain includes the sequence of amino acids set forth in SEQ ID NO:2, 103 or 104 or a functional portion or variant thereof including a sequence that exhibits at least 95% sequence identity to SEQ ID NO:2, 103 or 104; and the 4-1BB costimulatory signaling domain includes the sequence of amino acids set forth in SEQ ID NO:3 or a functional portion or variant thereof including a sequence that exhibits at least 95% sequence identity to SEQ ID NO:3. In some embodiments, the first domain and second domain together include the sequence of amino acids set forth in SEQ ID NO:5 or a functional portion or variant thereof including a sequence of amino acids that exhibits at least 85%, 86%, 87%, 88%, 89%, 90%, 91%, 92%, 93%, 94%, 95%, 96%, 97%, 98% or 99% sequence identity to SEQ ID NO:5. In some embodiments, the first domain and second domain together include the sequence of amino acids set forth in SEQ ID NO:5.

In some embodiments, the peptide or each of the peptides individually is between 7 and 30 amino acids, 8 and 20 amino acids, 8 and 15 amino acids, 10 and 17 amino acids, 7 and 13 amino acids or 8 and 10 amino acid; or the peptide or each of the peptides individually is or is about 10 amino acids, 11 amino acids, 12 amino acids, 13 amino acids, 14 amino acids, 15 amino acids, 16 amino acids or 17 amino acids.

In some embodiments, the isolated peptide(s) includes i) a sequence of amino acids set forth in any of SEQ ID NOS: 6, 7, 8-12, 16-22 and 137 or a variant thereof that retains activity to induce tolerance; or ii) a sequence of amino acids of a peptide epitope of a sequence of i) that binds to an HLA molecule.

In some embodiments, the peptide(s) has a binding affinity for a human leukocyte antigen (HLA) molecule that is less than 1000 nM, less than 500 nM or less than 50 nM. In some embodiments, the binding affinity is an IC50. In some embodiments, the HLA allele is selected from among HLA-A^{∗}02:01, HLA-A^{∗}03:01, HLA-A^{∗}11:01 and HLA-B^{∗}08:01. In some embodiments, the peptide contains or is the sequence of amino acids set forth in SEQ ID NO: 6, 7, 8-12, 16-22 or 137 or a peptidomimetic, analog or variant thereof.

In some embodiments, provided is a composition including any of the provided peptide or a plurality of peptides, including a plurality of any of the provided peptides. In some embodiments, provided is a composition including a plurality of peptides, wherein each of said peptides overlap in sequence and include all or a portion of an immunogenic region of a chimeric receptor. In some embodiments, the composition includes a pharmaceutically acceptable excipient. In some embodiments, provided is a combination including any of the provided peptides or a plurality of peptides including any of the provided peptides, and a chimeric receptor. In some embodiments, provided herein is a kit including any of the compositions or combinations described herein, and, optionally, instructions for use. In some embodiments, the chimeric receptor is expressed by cells genetically engineered with nucleic acid encoding the chimeric receptor. In some embodiments, the chimeric receptor is a chimeric antigen receptor.

### Brief Description of the Drawings

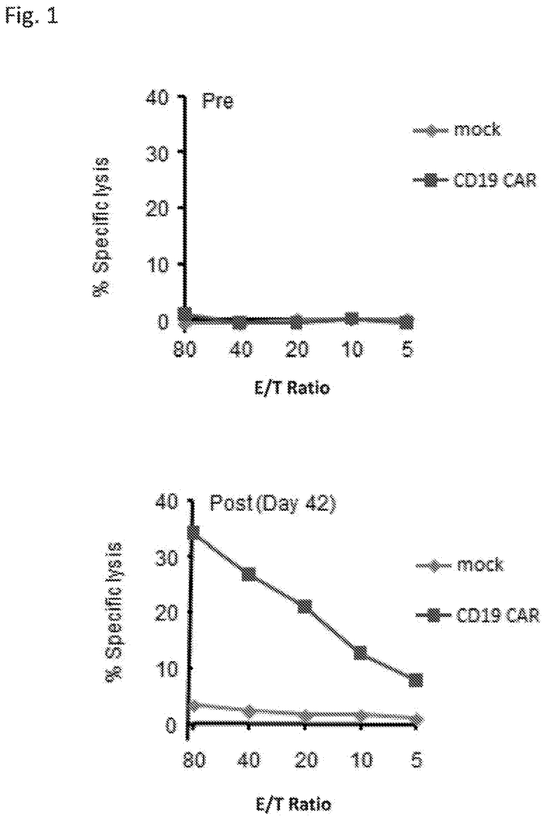

**Fig. 1** shows results from an exemplary chromium release assay detecting the presence of a cytolytic immune response specific for CAR-expressing cells following administration of anti-CD 19 CAR-expressing cells in a human subject. Results are shown for mixed-lymphocyte cultures containing peripheral blood mononuclear cells (PBMCs) derived from the subject pre-infusion (left panel) and post-infusion (right panel) with the CAR-expressing cells, in the presence of either CAR-expressing ("CD19-CAR") and non-CAR-expressing ("Mock"). "E/T"=effector to target cell ratio.

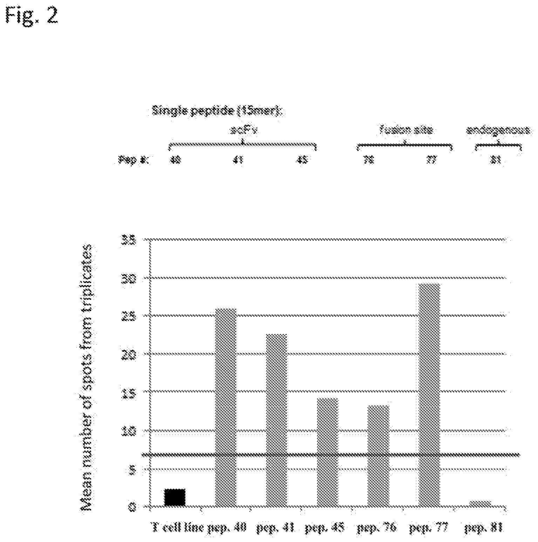

**Fig. 2** shows results from an exemplary ELISpot analysis confirming immune responses to certain overlapping peptides representing particular regions of a CAR in an exemplary human subject. Numbers labeled with "pep" represent various overlapping peptides along the length of the CAR sequence, with corresponding regions indicated above the chart.

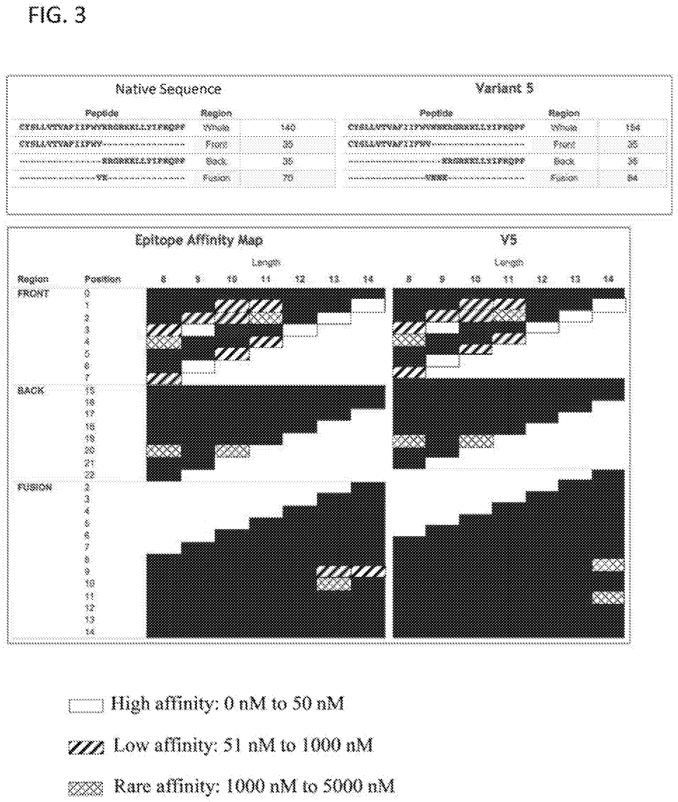

**Fig. 3** shows an epitope affinity map for predicted binding affinities of peptides of an exemplary region of a chimeric receptor for binding to HLA-A2:01, including a series of overlapping 8mer to 14mer peptides of an exemplary junction region having an amino acid sequence CYSLLVTVAFIIFWVKRGRKKLLYIFKQPF (SEQ ID NO: 6) where residues 1-15 correspond to an exemplary CD28 transmembrane domain and residues 16-30 correspond to an exemplary 4-1BB costimulatory domain. The figure also depicts predicted binding affinities of a series of overlapping 8mer to 14mer peptides of a variant junction region having an amino acid sequence CYSLLVTVAFIIFWVNNKRGRKKLLYIFKQPF (SEQ ID NO: 13), containing inserted asparagine residues between the CD28 transmembrane domain and 4-1BB costimulatory domain.

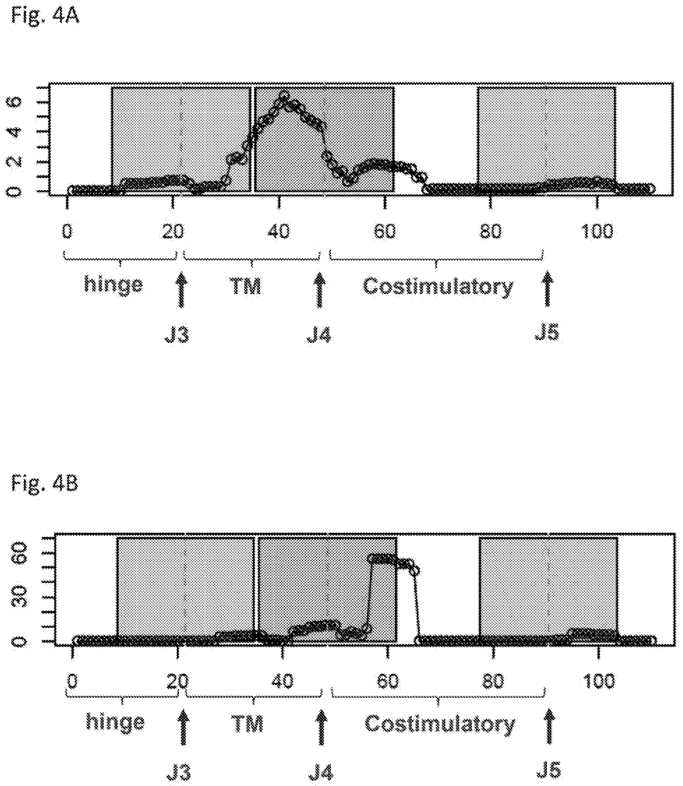

**Fig. 4A** and **Fig. 4B** depict algorithm-based T cell epitope predictions for HLA class I and HLA class II alleles, respectively, showing the total number of sequences in the dataset including each position along the length of the sequence with a predicted IC50 of less than 50nm weighted according to the frequency of the individual HLA alleles in the population.

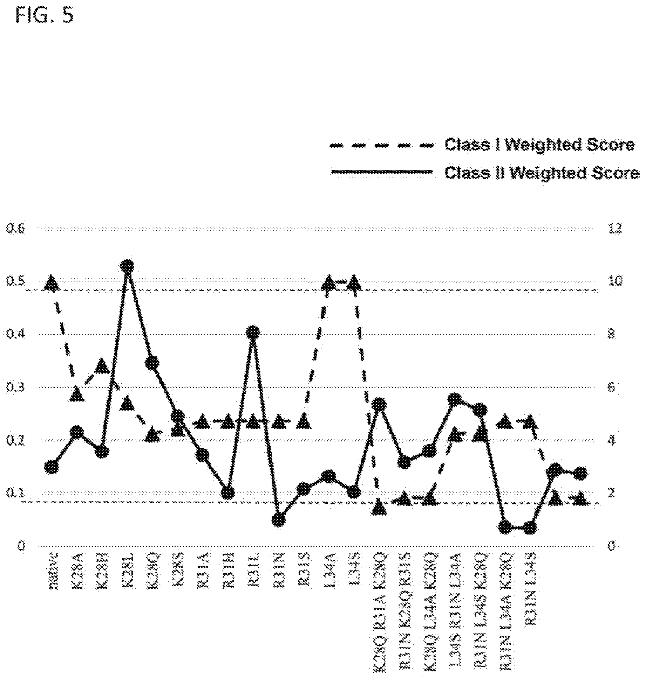

**Fig. 5** depicts algorithm-based T cell epitope predictions for HLA class I and HLA class II alleles of a series of variant peptides. Scores were determined and weighted as described in Example 2. Triangles and a dotted line indicated class I weighted scores. Circles and a solid line indicate class II weighted scores.

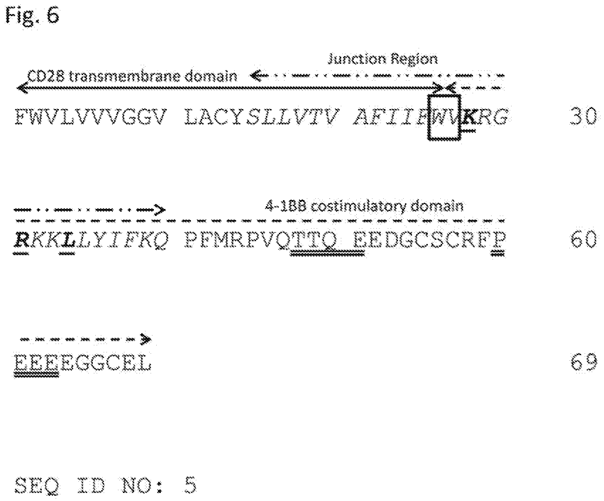

**Fig. 6** depicts the amino acid sequence of an exemplary CD28-4-1BB sequence of SEQ ID NO: 5. Amino acids corresponding to the CD28 transmembrane domain are indicated by a solid line with arrows indicating the beginning and end positions; amino acids of the exemplary 4-1BB costimulatory domain are indicated by a dashed line with arrows indicating the beginning and end positions; amino acids of the exemplary junction region are indicated by a dashed and dotted line with arrows indicating the beginning and end positions and by italics. The two amino acids immediately flanking the junction site are indicated by a box. Exemplary amino acids that in some embodiments are targeted for modification, including K28, R31, and L34, are bolded and underlined. Regions of acidic residues that may be involved in 4-1BB-mediated TRAF-binding and signaling are indicated by a double underline.

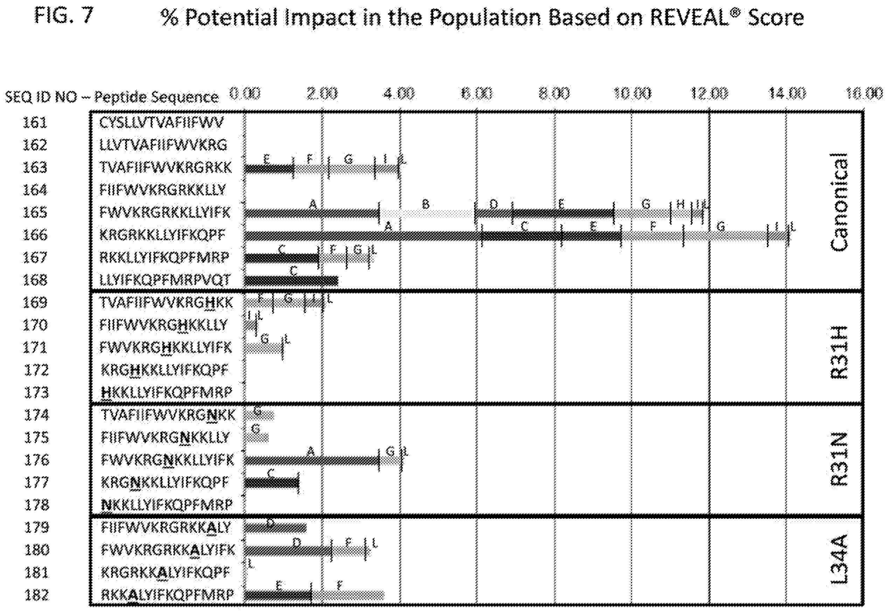

**Fig. 7** shows the relative binding to various MHC Class II molecules of overlapping 15-mer peptides within a portion of the region spanning the junction between the CD28 and 4-1BB-derived sequence (set forth in SEQ ID NO: 160; the canonical or "native" sequence) or within a corresponding portion containing either an R31H, R31N, or L34A substitution (with reference to numbering set forth in SEQ ID NO: 5). The REVEAL^{®} score for binding is shown. The score of individual HLA class II alleles are individually labeled with a corresponding letter as follows: A: DPA1^{∗}01:03; DPB1^{∗}04:01; B: DRA^{∗}01:01; DRB1^{∗}03:01; C: DRA^{∗}01:01; DRB1^{∗}15:01; D: DRA^{∗}01:01; DRB1^{∗}11:01; E: DPA1^{∗}01:03; DPB1^{∗}04:02; F: DPA1^{∗}01:03; DPB1^{∗}03:01; G: DPA1^{∗}01:03; DPB1^{∗}01:01; H: DPA1^{∗}02:01; DPB1^{∗}01:01; I: DPA1^{∗}02:01; DPB1^{∗}04:02; J: DRA^{∗}01:01; DRB1^{∗}11:04; K: DRA^{∗}01:01; DRB1^{∗}01:02; and L: DPA1^{∗}02:01; DPB1^{∗}15:01.

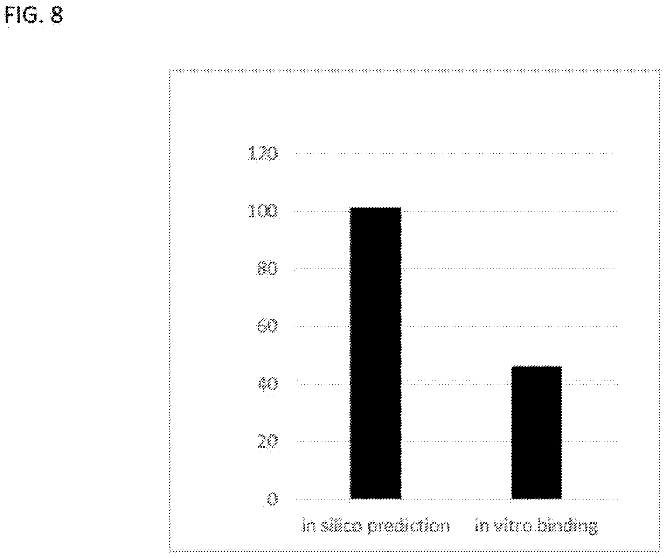

**Fig. 8** shows a comparison of the *in silico*-predicted versus in *vitro*-determined binding of a 15-mer peptide from the CD28-4-1BB junction to an MHC Class II molecule.

### Detailed Description

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

All publications, including patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### I. Methods Employing Peptides in Conjunction with Adoptive Cell Therapy

Provided herein are methods, compositions and articles of manufacture for use in conjunction with adoptive cell therapy, for example, for the treatment of various diseases and conditions. In some embodiments, the methods involve administering to the subject a peptide(s), such as an isolated peptide or combination of peptides, capable of preventing, reducing or otherwise down-regulating an immune response to a chimeric receptor, such as a chimeric antigen receptor (CAR), expressed on engineered cells. In some embodiments, cells, such as T cells, expressing a recombinant molecule, such as a receptor such as a genetically engineered chimeric receptor and/or antigen receptor, such as a CAR, are administered in connection with adoptive cell therapy methods. In some embodiments, the co-administered peptide(s) induces tolerance in a subject to cells expressing a genetically engineered chimeric receptor. In some embodiments, the peptide or peptides is administered prior to or simultaneously with administering the agent, e.g, the cells, e.g., expressing the molecule such as the genetically engineered chimeric receptor chimeric receptor, such as a CAR, to the subject.

In some aspects, the provided embodiments are based on observations that the efficacy of adoptive cell therapy may be limited by the development of an immune response in the subject to the cells and/or construct administered. For example, in some cases, exposure to engineered cells, such as those expressing a recombinant protein such as a chimeric receptor may be limited by host immune responses against the recombinant proteins, e.g., receptors expressed by the administered cells, which may prematurely eliminate the cells. It is observed that even in certain subjects having B cell malignancies, who often are immunocompromised, immune responses can be detected that are specific for regions of receptors expressed by cells administered in adoptive cell therapy. For example, as shown herein, subjects administered cells genetically engineered with a CAR developed a specific immune response to an immunogenic region of the chimeric region containing the junction between the transmembrane and costimulatory domain of the CAR.

In some aspects, the development of an immune response can reduce the exposure or persistence of engineered cells such as T cells expressing the molecule, e.g., the chimeric receptor, for example, over the course of adoptive therapy methods, which can in turn reduce their long-term efficacy. Observations indicate that, in some cases, increased exposure of the subject to administered cells expressing the recombinant receptors (e.g., increased number of cells or duration over time) may improve efficacy and therapeutic outcomes in adoptive cell therapy. Preliminary analysis conducted following the administration of different CD19-targeting CAR-expressing T cells to subjects with various CD19-expressing cancers in multiple clinical trials revealed a correlation between greater and/or longer degree of exposure to the CAR-expressing cells and treatment outcomes. Such outcomes included patient survival and remission, even in individuals with severe or significant tumor burden.

Further, in some cases, once such a host immune response develops, either acquired or innate, it may not be feasible or effective to attempt to increase exposure or provide retreatment of subjects by administering a subsequent dose of cells expressing the same recombinant receptor. Once such an immune response has developed against the receptor, administration of such a second or subsequent dose of cells expressing the same receptor or one with similar immunogenic epitopes may result in rapid elimination of the cells before they have had a chance to expand and/or persist to an effective or substantial degree. Thus, it would be advantageous to minimize unwanted immune responses that may destroy or interfere with the activity of engineered cells used in adoptive cell therapy methods, thereby diminishing the effectiveness of the treatment.

In some embodiments, the provided methods offer improved efficacy in one or more of such contexts. In some embodiments, the methods and compositions include features that result in a decreased immune response to administered engineered cells expressing a recombinant protein such as a recombinant receptor such as a chimeric receptor and/or increased exposure or persistence of such cells, such as in connection with adoptive immunotherapy. In some embodiments, by co-administering (which can include administering prior to, simultaneously with, or subsequent to, and/or intermittently with) to a subject a peptide(s) comprising an epitope or epitopes within the recombinant protein such as the of a chimeric receptor, such as a CAR, the provided methods can result in the generation or production of a population of genetically engineered T cells that have the capability to exhibit increased or longer exposure and/or persistence when administered to a subject, for example, as compared with a method that does not involve administration of the peptide(s) antigen.

In particular, the provided methods relate to inducing immunological tolerance towards an antigenic chimeric receptor (e.g. CAR) expressed on engineered cells by administering to a subject a peptide antigen or peptide antigens of the chimeric receptor (e.g. CAR). Immunological tolerance (or "immune tolerance") is a process that is part of the normal function of the immune system. Antigen-specific immune tolerance is characterized by a decrease in responsiveness to an antigen, which is induced by previous exposure to that antigen. When specific lymphocytes encounter antigens, the lymphocytes may be activated, leading to an antigen-specific immune response, or the cells may be inactivated or eliminated, leading instead to antigen-specific immune tolerance. The same antigen may induce an antigen-specific immune response or tolerance, depending on the circumstances of the antigen's presentation to the immune system.

In some aspects, tolerance can be caused by clonal anergy, peripheral clonal deletion, suppression of T cells and/or other forms of antigen-specific tolerance. In some embodiments, tolerance may result from or be characterized by the induction of anergy. In some aspects, anergy can result by exposure of CD4+ T cells to an antigen in the absence of costimulation and/or innate immune factors may make the cells incapable of responding to that antigen. Since full activation of T cells generally requires the recognition of antigen by the TCR (signal one) as well as recognition of costimulators (signal two), prolonged antigen recognition alone, in the absence of the second signal, may lead to anergy (i.e., functional unresponsiveness). Anergic T cells may be refractory to subsequent antigenic challenge, and may be capable of suppressing other immune responses. In some embodiments, by administering peptide(s) and inducing tolerance in the absence of adjuvant is advantageous in that it promotes antigen presentation by certain inhibitory cells such as inhibitory antigen presenting cells (such as immature or suppressive DCs or myeloid cells, e.g., IDO+ DCs, pDCs, iDCs, or Treg-inducing DCs (tDCs). The absence of adjuvant may favor presentation by an immune suppressive APC instead of a stimulatory DC or equivalent APC.

Generally, in the natural setting tolerance is involved in non-reactivity or non-productive reactivity to self-antigens. In some cases, however, tolerance to a non-self antigen can be induced. Thus, in some aspects, the same mechanisms by which mature T cells that recognize self-antigens in peripheral tissues become incapable of subsequently responding to these antigens also may regulate unresponsiveness to foreign antigens. In some aspects, the peptide(s) can induce tolerance by inducing clonal anergy, clonal deletion, e.g., peripheral clonal deletion, suppression of T cells and/or other forms of antigen-specific tolerance. In some embodiments, tolerance can be induced by administering an antigenic component into a subject, which, under certain conditions, can be capable of reducing or minimizing an immune response to that component. Thus, while a peptide(s) that is immunogenic can provoke an immune response, it can be administered to induce tolerance when administered under particular conditions, such as at particular tolerogenic doses or under defined routes of administration.

In some aspects, the peptide(s) is one that is or includes an immunogenic region of the recombinant protein such as the chimeric receptor, such as a CAR, which generally is a genetically engineered protein, such as chimeric receptor, expressed on or in or encoded by cells to be administered to the subject to which the peptide is administered. The peptide(s) can be a combination or plurality of peptides. In some embodiments, the peptide(s) is derived from or contains a contiguous sequence of amino acids contained in the recombinant protein, e.g., chimeric receptor, such as CAR. In some embodiments, the peptide(s) includes all or part of non-self or non-native (with reference to the subject or species to which the peptide is administered) sequence present in the recombinant protein such as the chimeric receptor (e.g. CAR). In some embodiments, the non-self or non-native sequence is a sequence that contains amino acid residues that span a junction region between two domains of the molecule. In some embodiments, the non-self or non-native sequence is a sequence that contains amino acid residues with origins in different species than the subject to be treated. In some embodiments, the domain(s) of the chimeric receptor (such as antigen-binding domains, e.g., sFvs, and/or or other domains of chimeric receptors, e.g., other CAR domains) have sequences with origins in the same species as the subject to be treated and/or have sequences with origins in different species than the subject to be treated. For example, in some embodiments, the chimeric receptor can contain a binding domain that is derived from a murine sequence. In other embodiments, the chimeric receptor can contain a binding domain that is derived from a human sequence or that is a humanized sequence. In some embodiments, the subject to be treated is a human and the non-self or non-native sequence is or includes a non-human sequence. In some embodiments, even if all domains of the chimeric receptor are human or otherwise derived from the same species to be treated, the junction region that includes amino acid residues that span the two domains may nonetheless be recognized by the immune system as non-self.

In some embodiments, the peptide(s) includes a sequence of amino acids of a molecule in or on a therapeutic agent, such as an engineered cell such as a chimeric receptor (e.g. a CAR), that is or contains at least one immune cell epitope such as a T cell epitope (or peptide epitope) or at least one B cell epitope that is recognized by the immune system. In some embodiments, the peptide(s) is or contains at least one T cell epitope. In some embodiments, a T cell epitope is a peptide antigen that is capable of inducing an immune response in an animal by its binding characteristics to major histocompatibility complex (MHC) molecules. In some embodiments, the peptide is capable of binding to an MHC molecule (HLA molecule in humans). In some embodiments, the MHC molecule is an MHC class I (e.g. HLA class I) or MHC class II (e.g. HLA class II) molecule. In some embodiments, the peptide(s) binds to an allele of an MHC molecule that is expressed or present in the subject to which the peptide is administered.

In some embodiments, the provided methods and compositions are advantageous in that the peptides may be used regardless of the HLA type or subtype of the subject. For example, it generally is not required to know or pre-determine the subject's HLA type or subtype in order to select the correct peptide or pool (combination or plurality) thereof. In some embodiments, the pool of peptides representing immunogenic or neo-antigens or epitopes in the agent to be administered, only those that bind to the subject's HLA molecules will induce tolerance, thereby self selecting and specifically tolerizing the subject to the peptides that naturally bind with some affinity to their HLA molecules, and thus specifically tolerizing the subject against the immune epitopes most likely to be presented in a potential immune response upon administration of the agent.

In some embodiments, the peptide(s) has a binding affinity for binding to a human leukocyte antigen (HLA) with a binding affinity of less than 1000 nM. less than 500 nM or less than 50 nM. In some cases, the binding affinity can be an IC50 for binding, for example, the concentration able to lead to 50% inhibition of a standard peptide. In some embodiments, the HLA molecule is an HLA class I molecule or an HLA class II molecule, such as any set forth in Table 1A or Table 1B. In some embodiments, the HLA is HLA-A^{∗}02:01, HLA-A^{∗}03:01, HLA-A^{∗}11:01 and HLA-B^{∗}08:01.

In some embodiments, the peptide(s) binds to an HLA-A2 allele, which in some populations is expressed by approximately 50% of the population. In some embodiments, the HLA-A2 allele can be an HLA-A^{∗}0201, ^{∗}0202, ^{∗}0203, ^{∗}0206, or ^{∗}0207 gene product. In some cases, there can be differences in the frequency of subtypes between different populations. For example, in some embodiments, more than 95% of the HLA-A2 positive Caucasian population is HLA-A0201, whereas in the Chinese population the frequency has been reported to be approximately 23% HLA-A^{∗}0201, 45% HLA-A^{∗}0207, 8% HLA-A^{∗}0206 and 23% HLA-A^{∗}0203. In some embodiments, the MHC-restricted epitope is HLA-A^{∗}0201, which is expressed in approximately 39-46% of all caucasians.

In some embodiments, the peptide(s) typically is about 8 to about 24 amino acids in length, such as about 8 to about 15 amino acids in length. In some embodiments, a peptide or plurality of peptides individually has a length of from or from about 9 to 22 amino acids for recognition in the MHC Class II complex, such as for example about 15 amino acids. In some embodiments, a peptide or a plurality of peptides individually has a length of from or from about 7 to 15 amino acids, such as 8 to 13 amino acids, for recognition in the MHC Class I complex. In some embodiments, the peptide(s) is an isolated peptide.

In some embodiments of the methods provided herein, a peptide(s) that is a peptide antigen or epitope or epitopes or portion or portions thereof, is administered in a way that inhibits, reduces, or prevents the potential for an immune response (such as upon the later administration of the engineered cells or recombinant protein, such as CAR, containing the epitope). In some aspects, this inhibition or reduction or prevention is carried out by the induction of antigen (or epitope)-specific tolerance to proteins or cells containing an epitope present in the protein, e.g., in lymphocytes. Induction of tolerance can depend on factors, such as the physical form or nature of the antigen, the route of administration and/or the dose of peptide (see e.g. Hochweller et al. (2006) Curr. Mol. Med., 6:631-43; Parija (2014) Immune Response In Textbook of Microbiology and Immunology (pp. 134-142)). For example, peptides administered in soluble form, e.g. non-aggregated, can be more tolerogenic than aggregated antigens. In some embodiments, repeated low doses can, in some aspects, induce tolerance. In some cases, administration of a high dose of a peptide antigen can induce tolerance. Studies have shown that, in some cases, peptide, when given in soluble form intraperitoneally (i.p.), intravenously (i.v.), intranasally (i.n.), or orally can induce T cell tolerance (Anderton and Wraith, Eur. J. Immunol. 28:1251-1261 (1998); Liu and Wraith, Int. Immunol. 7:1255-1263 (1995); Metzler and Wraith (1999) Immunology 97:257-263; Aichele et al. (1995) J. Exp. Med., 182:261-266). In some embodiments, one or more of the above conditions is employed when administering a peptide or combination of peptides in the context of the provided methods.

In some embodiments, the peptide(s) is administered in an aqueous soluble form but in the absence of a pro-inflammatory signal, such as in the absence of adjuvant. For example, in some cases, adjuvants are known to be able to facilitate lymphocyte activation and promotion of an antigen-specific immune response.

In some embodiments, the peptide(s) is administered in the presence of an adjuvant, but along with a tolerogenic agent to reduce or dampen an immune response. In some aspects, a tolerogenic agent can interfere with T cell activation, such as interfere with costimulation of a T cell required for T cell activation. Generally, T lymphocytes that recognize foreign antigens without costimulation, but in the presence of an adjuvant, may cause the T cells to die (deletion) by activation-induced cell death (AICD).

In some embodiments, the peptide(s) can be administered by repeat administration of the peptide a plurality of times, which can promote tolerance. In some embodiments, repeated administration is with a low dose of peptide. In some embodiments, the repeat administration of the peptide(s) occurs over an extended time period, such as for up to three days, four days, five days, six days, one week, two weeks, three weeks or a month. In some embodiments, the peptide(s) can be administered in a high dose amount. In some embodiments, one or more of the above administration modes, schedules or frequency of administration are employed.

In some embodiments, the peptide(s) is co-administered with cells genetically engineered with a recombinant protein or molecule such as a recombinant receptor such as a chimeric receptor (e.g. CAR), and generally a corresponding chimeric receptor (e.g. CAR) that contains an immunogenic region that is the same or related to the sequence as present in the peptide, e.g. a region containing a peptide epitope that exhibits a binding affinity for an HLA molecule of less than 1000 nM. less than 500 nM or less than 50 nM. In some embodiments, administration of the chimeric receptor (e.g. CAR) is by adoptive cell therapy methods using cells, such as T cells, engineered with a CAR. In some aspects, the cells are isolated from a subject, engineered, and administered to the same subject. In other aspects, they are isolated from one subject, engineered, and administered to another subject.

In some embodiments, the chimeric receptor is a CAR that generally specifically binds to one or more antigens expressed by, associated with, and /or specific for a disease or condition in the subject and/or cell(s) or tissue(s) thereof. In some embodiments, the chimeric receptor is a receptor other than an antigen receptor, such as one of a pair of binding partners and/or variant thereof, the other partner of which is specifically expressed in the context of a disease or condition or cells or tissues thereof, and/or expression of which is associated with the disease or condition. In some embodiments, such chimeric receptors contain extracellular binding portions that specifically interact with such a binding partner, and contain, for example, transmembrane and/or intracellular signaling domain(s) capable of potentiating an immunostimulatory signal or signals, such as an activating and/or costimulatory domains such as those present in certain chimeric antigen receptors. Similarly, in some forms of chimeric receptors the receptor may mediate a suppressive function, such as those demonstrated in regulatory T cells. To maintain the desired suppressive environment, such cells also need to persist and resist immunological rejection due to immunogenicity of their neo-epitopes formed by gene fusion. In these cases, tolerance to immune rejection is also of value and can be mediated through appropriate "neo-epitope" derived peptides. In some embodiments, the antigen or binding partner is associated with and/or specific for a disease or condition that may include tumors, cancers, other proliferative diseases, autoimmune disease or disorders, and/or infectious agents or disease.

In some embodiments, the peptide(s) can be administered prior to, simultaneously, intermittently or subsequent to the administration of the therapy or agent, e.g., engineered cells, expressing in or including the protein or other molecule containing the epitope or epitopes present in the peptide, e.g., a recombinant or genetically engineered receptor such as a chimeric receptor (e.g. CAR). Generally, the peptide(s) is administered prior to administration of the cells expressing the chimeric receptor, such as prior to administration of the agent such as the cells.

In some embodiments, the methods also include treatment with an immunosuppressive drug. In some embodiments, the immunosuppressive drug is administered prior to, simultaneously, subsequently or intermittently with administration of the peptide. In some embodiments, the immunosuppressive drug is administered prior to, simultaneously, subsequently or intermittently with administration of the agent, e.g., cells and/or chimeric receptor, such as CAR.

In some embodiments, the provided methods reduce or lessen or prevent an immune response in a subject administered with cells expressing the chimeric receptor, such as CAR, compared to the immune response generated in the subject administered with cells expressing the chimeric receptor, such as CAR, in the absence of peptide(s). In some embodiments, the subject does not exhibit an immune response or a particular type or degree of immune response, against the chimeric receptor, such as following the administration of the cells expressing the receptor. The type of immune response may be a detectable immune response, a humoral immune response, and/or a cell-mediated immune response.

In some embodiments, the provided methods achieve increased persistence of genetically engineered T cells expressing the chimeric receptor, such as CAR, when administered to a subject. In some embodiments, a genetically engineered cell with increased persistence exhibits better potency in a subject to which it is administered. In some embodiments, the persistence of genetically engineered cells, such as CAR-expressing T cells, in the subject upon administration is greater as compared to that which would be achieved by alternative methods, such as those involving administration of genetically engineered cells in which a peptide(s) is not co-administered with the genetically engineered cells. In some aspects, the persistence of administered cells is increased at least or about at least 1.5-fold, 2-fold, 3-fold, 4-fold, 5-fold, 6-fold, 7-fold, 8-fold, 9-fold, 10-fold, 20-fold, 30-fold, 50-fold, 60-fold, 70-fold, 80-fold, 90-fold, 100-fold or more.

In some embodiments, the degree or extent of persistence of administered cells can be detected or quantified after administration to a subject. For example, in some aspects, quantitative PCR (qPCR) is used to assess the quantity of cells expressing the chimeric receptor (e.g., CAR-expressing cells) in the blood or serum or organ or tissue (e.g., disease site) of the subject. In some aspects, persistence is quantified as copies of DNA or plasmid encoding the receptor, e.g., CAR, per microgram of DNA, or as the number of receptor-expressing, e.g., CAR-expressing, cells per microliter of the sample, e.g., of blood or serum, or per total number of peripheral blood mononuclear cells (PBMCs) or white blood cells or T cells per microliter of the sample. In some embodiments, flow cytometric assays detecting cells expressing the receptor generally using antibodies specific for the receptors also can be performed. Cell-based assays may also be used to detect the number or percentage of functional cells, such as cells capable of binding to and/or neutralizing and/or inducing responses, e.g., cytotoxic responses, against cells of the disease or condition or expressing the antigen recognized by the receptor. In any of such embodiments, the extent or level of expression of another marker associated with the recombinant receptor (e.g. CAR-expressing cells) can be used to distinguish the administered cells from endogenous cells in a subject.

Also provided are compositions containing the peptide(s) and/or engineered cells. Also provided are articles of manufacture, such as kits and devices, for administering the peptides, cells and compositions to subjects, such as for adoptive cell therapy.

### II. Peptides, Compositions And Methods of Administering Peptides

### A. Peptides

Provided herein are peptides that correspond to, such as are based on or derived from, a sequence of amino acids present in a therapy or agent, such as an engineered cell, such as a recombinant protein, e.g., a recombinant receptor, e.g., a chimeric receptor, such as a CAR. Also provided are methods of administering such peptides to a subject, e.g., to reduce or prevent an immune response to the agent or therapy, e.g., to induce tolerance in the subject to an antigen that is or is within the therapy or agent, e.g., that is expressed in or by the cell or on the cell or is included within the recombinant protein such as the chimeric receptor, for example a CAR. In some embodiments, chimeric receptors and other proteins administered, and cells expressing the same, such as CARs, can be antigenic. For example, they can contain one or more T cell epitopes and/or B cell epitopes recognized by the immune system in a manner that elicits a productive and specific immune response against such agent, which for example may result in clearance or reduction of the cells. In some embodiments, such immunogenicity may develop because the peptides contain sequences that are not naturally produced in the body of the subject, and which are recognized as foreign to the subject. In some embodiments, the peptide or peptides administered in the provided methods and/or the provided peptide or peptides is or contains an epitope, such as a peptide epitope (or T cell epitope), present within the agent to be administered, such as the recombinant protein, such as the chimeric receptor.

In some embodiments, the peptide and/or the epitope is recognized by the immune response to initiate an immune response or activation or primary signal of an immune cell thereof, such as initiating humoral or cell-mediated activation, against such peptide sequence. In some embodiments, the peptide epitope can associate or bind with an MHC molecule expressed on a cell for recognition by a T cell receptor (TCR). In some embodiments, the peptide(s), under certain conditions, can elicit an immune response in an animal, such as a T cell response. For example, in some cases, T cells expressing a TCR that recognizes a T cell epitope in the context of an MHC molecule can become stimulated, e.g., with a primary and secondary signal, thereby leading to T cell responses, such as T cell proliferation, lymphokine secretion, cytotoxic responses, local inflammatory reactions, recruitment of additional immune cells and /or activation of B cells leading to production of antibodies against the protein containing the peptide epitope, such as a chimeric receptor. It is within the level of a skilled artisan to identify a peptide epitope of any chimeric receptor.

In some embodiments, the peptide(s), under certain conditions, is capable of inducing tolerance, e.g., tolerizing a subject to an antigen containing the epitope, e.g., the peptide epitope, such as by preventing, reducing or otherwise down-regulating a specific immune response to a chimeric receptor containing the antigen. In some cases, the peptide(s) is or includes or is related to an immunogenic region of the agent to be administered such as the engineered cell and/or protein or molecule expressed by the same such as a recombinant receptor, e.g., a chimeric receptor, such as a CAR, containing the peptide epitope. In some embodiments, this ability is due to the ability of the peptide(s) to induce activation of immune cells in certain contexts and/or to induce partial or transient or short-lived immune cell activation, e.g., without an adjuvant. In some embodiments, the peptide(s) is or includes or has a sequence related to a non-native amino acid sequence of the agent or recombinant molecule such as the receptor, e.g., chimeric receptor, such as a non-human sequence, present in the chimeric receptor (e.g. CAR). In some aspects, tolerance can be induced by the peptide(s) when it is administered in soluble form, at a low dose (including a plurality of times) or high dose, in the absence of adjuvant and/or in the presence of adjuvant with coadministration of a tolerogenic agent. In some embodiments, tolerance can be induced by administering the peptide(s) intraperitoneally (i.p.), intravenously (i.v.), intranasally (i.n.), or orally or subcutaneously (s.c.).

A peptide is generally a portion of a polypeptide less than the full length but that is greater than or equal to 2 amino acids in length, such as one that is greater than or equal to 2 and less than or equal to 50 or 40 amino acids in length. In some embodiments, the peptide is between 7 and 40 amino acids, 8 and 20 amino acids, 10 and 17 amino acids, 7 and 13 amino acids or 8 and 10 amino acids. In some embodiments, the peptide has a length of between 7 and 20 amino acids. In some embodiments, the peptide has a length of 7, 8, 9, 10, 11, 12, 13, 14, 15, 16, 17, 18, 19, or 20 amino acids.

In some embodiments, the peptide(s) is a peptide comprising an epitope within an immunogenic region of the agent, e.g., the chimeric receptor. In some embodiments, the immunogenic region is or include or is contained in an scFv portion, a linker portion, an amino acid sequence of the chimeric receptor not endogenous to the subject, a sequence of the chimeric receptor derived from a different species than that of the subject, and/or a junction between two domains of a chimeric receptor.

In some embodiments, the chimeric receptor is a chimeric antigen receptor (CAR) containing an extracellular ligand binding domain. In some cases, the extracellular ligand binding domain is an antibody or antibody fragment. In some embodiments, the antibody or antibody fragment is or includes a sequence derived from a species other than the subject to which the chimeric receptor is administered. In some embodiments, the species is a mouse or other rodent. In some embodiments, the chimeric receptor, such as a CAR, includes an extracellular antigen binding domain, such as a portion of an antibody molecule, generally a variable heavy (V_{H}) chain region and/or variable light (V_{L}) chain region of the antibody, e.g., an scFv antibody fragment. In some embodiments, the peptide epitope is a sequence present in an immunogenic region that is or include or is contained in a framework region (FR) within the scFv portion, a heavy chain FR sequence a heavy chain CDR sequence, a light chain FR sequence, and/or a light chain CDR sequence.

In some embodiments, the chimeric receptor contains at least a first domain and a second domain, and the immunogenic region of the chimeric receptor is a junction region comprising amino acids on each side of a junction between said two domains. In some embodiments, the peptide has a sequence of amino acids that is, contains or is related to a sequence of amino acids that spans a junction region of a chimeric receptor, such as a CAR, between two domains. Generally, chimeric receptors include a plurality of different domains, such as a plurality of endogenous or naturally-occuring domains. For example, in some embodiments, a CARs includes an extracellular antigen-recognition domain that specifically binds to a target antigen and an intracellular signaling domain comprising an ITAM (e.g. CD3-zeta intracellular signaling domain). In some embodiments, the chimeric receptor includes (instead or in addition to an ITAM-containing domain) other signaling domains which may be costimulatory and/or inhibitory. In some embodiments, the extracellular antigen-recognition domain comprises an antibody or antigen-binding fragment (e.g. scFv). A CAR in some embodiments also can contain a transmembrane domain and/or endodomain between the extracellular recognition domain and signaling domain, which can include transmembrane and/or intracellular signaling domains of a co-stimulatory molecule, such as a CD28 or 4-1BB co-stimulatory molecule. In some cases, such domains can be connected by linkers.

In some cases, the plurality of domains are not normally present adjacent to each other in sequence in an endogenous molecule in a subject. For example, in some embodiments, a first domain and a second domain of a chimeric receptor that are joined at a junction can create a contiguous sequence of amino acids that may not be present in a subject, particularly where such two domains do not naturally occur adjacent together in the same protein and/or are separated by a synthetic linker. In some cases, this can result in the presence of a non-native sequence that is not identical to a sequence present in an endogenous molecule of the host in the portion of the sequence of a chimeric receptor, such as a CAR, containing the junction between two domains.

In some cases, a junction region that contains potential peptide epitopes spanning the junction of the two domains can be immunogenic and result in the generation of an immune response upon administration to a subject of a chimeric receptor containing the junction region. In some embodiments, the junction region can include, such as upon antigen processing for display on an HLA, a plurality of individual overlapping peptide fragments of contiguous sequence of about 8 to 24 amino (e.g. 8 to 15 amino acids or 8 to 13 amino acids, such as about or 8, 9, 10, 11, 12, 13, 14, 15 or more amino acids) directly C-terminal of the junction that joins a first domain and a second domain of the chimeric receptor and/or of about 8 to 24 amino acids (e.g. 8 to 15 amino acids or 8 to 13 amino acids, such as about or 8, 9, 10, 11, 12, 13, 14, 15 or more amino acids) directly N-terminal of the junction, which peptide fragments each can include or span the junction of the two domains. Thus, in some cases, the junction region can contain a plurality of potential peptide epitopes that may exhibit a binding affinity for an HLA molecule and/or be capable of inducing an immune response.

In some embodiments, the peptide(s) contains a contiguous sequence of amino acids of the junction region. In some cases, the first domain and second domain are, respectively, an extracellular ligand binding domain and a hinge domain, an extracellular ligand binding domain and a transmembrane domain, a transmembrane domain and an intracellular costimulatory signaling domain, and an intracellular costimulatory signaling domain and an ITAM signaling domain. In some embodiments, the first domain is a transmembrane domain or a functional portion thereof and the second domain is a costimulatory signaling domain or a functional portion thereof. In some embodiments, the chimeric receptor is a CAR and the peptide(s) has a sequence of amino acids that is, contains or is related to a sequence of amino acids containing a junction region between any of the following domains: an scFv, a CD28 transmembrane domain, a 4-1BB intracellular signaling domain, a CD3-zeta intracellular signaling domain, a T2A domain, a truncated EGFR (EGFRt) portion, a CD8α signaling domain, an IgG4 hinge domain and/or a linker, including, for example, a (GGGGS)ₓ linker.

In some embodiments, a junction region of a chimeric receptor (e.g. CAR) contains a contiguous portion of a transmembrane domain and a contiguous portion of a costimulatory domain joined at a junction. In some embodiments, the transmembrane domain includes a transmembrane portion of CD28. In some embodiments, the costimulatory signaling domain includes the intracellular costimulatory signaling domain of a T cell costimulatory molecule. In some embodiments, the costimulatory molecule is CD28. In some embodiments, the costimulatory domain is 41BB. In some embodiments, the CD28 transmembrane domain comprises the sequence of amino acids set forth in SEQ ID NO:2, 103 or 104 or a functional portion or variant thereof comprising a sequence that exhibits at least 95%, 96%, 97%, 98%, 99% or more sequence identity to SEQ ID NO:2, 103 or 104. In some embodiments, the 4-1BB costimulatory signaling domain comprises the sequence of amino acids set forth in SEQ ID NO:3 or a functional portion or variant thereof comprising a sequence that exhibits at least 95%, 96%, 97%, 98%, 99% or more sequence identity to SEQ ID NO:3. In some embodiments, the CD28 transmembrane domain and 4-1BB costimulatory domain together have the sequence of amino acids set forth in SEQ ID NO:5 or a functional portion or functional variant thereof having a sequence of amino acids that exhibits at least 85%, 86%, 87%, 88%, 89%, 90%, 91%, 92%, 93%, 94%, 95%, 96%, 97%, 98% or 99% sequence identity to SEQ ID NO:5.

In some embodiments, the junction region contains a portion of SEQ ID NO:5 that is or includes the junction of the two domains. In some embodiments, the junction region is or includes the amino acid sequence CYSLLVTVAFIIFWVKRGRKKLLYIFKQPF (SEQ ID NO: 6). In some embodiments, the junction region is or includes the amino acid sequence SLLVTVAFIIFWVKRGRKKLLYIFKQ (SEQ ID NO:137). In some embodiments, the junction region is or includes the amino acid sequence
CYSLLVTVAFIIFWVKRGRKKLLYIFKQPFMRPVQT (SEQ ID NO:160).

In some embodiments, the peptide(s) has a sequence of amino acids that is or is contained within a junction region portion of SEQ ID NO:5. In some embodiments, the peptide(s) has a sequence of amino acids that is or is contained within a junction region set forth in SEQ ID NO:6. In some embodiments, the peptide(s) has a sequence of amino acids that is or is contained within a junction region set forth in SEQ ID NO:137. In some embodiments, the peptide(s) has a sequence of amino acids that is contained within a junction region set forth in SEQ ID NO: 160. In some embodiments, the peptide(s) has a contiguous sequence of about 8 to 24 amino (e.g. 8 to 15 amino acids or 8 to 13 amino acids, such as about or 8, 9, 10, 11, 12, 13, 14, 15 or more amino acids) contained within a junction region of SEQ ID NO:5 or contained within SEQ ID NO:6 or contained within SEQ ID NO:137 or contained within SEQ ID NO:160.

In some embodiments, a plurality of peptides can be administered to the subject, in which the peptides represent two or more peptides present in a junction region or immunogenic region of a chimeric receptor. In some cases, the plurality can represent a series of overlapping peptide fragments of the same junction region or immunogenic region. In some case, the plurality of peptides represents peptide fragments present in two or more different junction regions or immunogenic regions of the chimeric receptor. In some embodiments, the plurality of peptides can be overlapping peptides of, for example 8 to 24 amino acids (e.g. 8 to 15 amino acids or 8 to 13 amino acids, such as about or 8, 9, 10, 11, 12, 13, 14, 15 or more amino acids), of the sequence set forth in SEQ ID NO:5, 6, 137 or 160. The plurality of peptides can include 2, 3, 4, 5, 6 ,7, 8, 9, 10, 11, 12, 13, 14, 15, 16, 17, 18, 19, 20 or more peptides.

In some embodiments, the peptide or the plurality of peptides individually is or contains the amino acid sequence AFIIFWVKRGRKKLL (SEQ ID NO: 8), FWVKRGRKKLLYIFK (SEQ ID NO: 9, also set forth in SEQ ID NO:165), FIIFWVKRGRKKLL (SEQ ID NO: 10), FIIFWVKRGRKKL (SEQ ID NO: 11), IIFWVKRGRKKLL (SEQ ID NO:12), VAFIIFWVK (SEQ ID NO:16), AFIIFWVKR (SEQ ID NO:17), FIIFWVKRG (SEQ ID NO:18), IIFWVKRGR (SEQ ID NO:19), IFWVKRGRK (SEQ ID NO:20), FWVKRGRKK (SEQ ID NO:21), or WVKRGRKKL (SEQ ID NO:22), TVAFIIFWVKRGRKK (SEQ ID NO:163), KRGRKKLLYIFKQPF (SEQ ID NO:166), RKKLLYIFKQPFMRP (SEQ ID NO:167), LLYIFKQPFMRPVQT (SEQ ID NO:168), a portion thereof, or is a peptidomimetic, analog or a variant thereof. In some embodiments, the portion is a peptide that comprises at least 7 amino acids, specifically binds to an MHC molecule, and is capable of inducing tolerance, i.e. preventing, reducing or otherwise down-regulating an immune response to a co-administered chimeric receptor, such as a CAR.

In some embodiments, the peptide(s) is capable of binding to a major histocompatibility complex (MHC) molecule, such as a class I or class II protein, which are molecules that contain a polymorphic peptide binding site or binding groove that can, in some cases, complex with peptide fragments of polypeptides, including peptides processed by the cell machinery. In some cases, MHC molecules can be displayed or expressed on the cell surface, including as a complex with peptide, i.e. MHC-peptide complex, for presentation of an antigen in a conformation recognizable by TCRs on T cells. Generally, MHC class I molecules are heterodimers having a membrane spanning α chain, in some cases with three α domains, and a non-covalently associated β2 microglobulin. Generally, MHC class II molecules are composed of two transmembrane glycoproteins, α and β, both of which typically span the membrane. An MHC molecule can include an effective portion of an MHC that contains an antigen binding site or sites for binding a peptide and the sequences necessary for recognition by the appropriate binding molecule, such as TCR. In some embodiments, MHC class I molecules deliver peptides originating in the cytosol to the cell surface, where a peptide:MHC complex is recognized by T cells, such as generally CD8⁺ T cells, but in some cases CD4+ T cells. In some embodiments, MHC class II molecules deliver peptides originating in the vesicular system to the cell surface, where they are typically recognized by CD4⁺ T cells. Generally, MHC molecules are encoded by a group of linked loci, which are collectively termed H-2 in the mouse and human leukocyte antigen (HLA) in humans. Hence, typically human MHC can also be referred to as human leukocyte antigen (HLA). The sequences of MHC alleles are known and can be found, for example, at the IMGT/HLA database available at www.ebi.ac.uk/ipd/imgt/hla.

In some embodiments, the peptide(s) is capable of binding to an MHC molecule that is a human MHC molecule. In some embodiments, the MHC molecule is a human leukocyte antigen (HLA) molecule.

In some embodiments, the peptide(s) requires further processing. For example, in some embodiments, some epitopes bind MHC in a way that is dependent on MHC-loading in endosomes, and hence require processing (Viner et al (1995) Proc. Natl. Acad. Sci. 92:2214-2218). In some embodiments, the peptide(s) can be presented on MHC molecules without processing.

In some embodiments, the peptide(s) (or a processed form thereof) exhibits binding affinity for an MHC allele of the subject to which it will be administered. In some embodiments, binding affinity can range from 0.1 nM to 25,000 nM, from 0.1 nM to 5,000 nM, from 0.1 nM to 1,000 nM. from 0.1 nM to 500 nM. from 0.1 nM to 250 nM. from 0.1 nM to 100 nM, from 0.1 nM to 50 nM, from 0.1 nM to 25 nM, or from 0.1 nM to 10 nM. It is within the level of the skilled artisan to determine the peptide's binding affinity. In some embodiments, the peptide(s) exhibits a binding affinity (e.g. IC50) that is less than 1000 nM, less than 500 nM or less than 50 nM.

In some embodiments, the binding affinity can be determined experimentally or algorithmically. In some embodiments, a peptide binding affinity for an MHC can be determined computationally, such as by using algorithms based on quantitative binding affinity models (Lafuente and Reche (2009) Current Pharmaceutical Design, 15:3209-3220).

In some embodiments, determining a peptide's binding affinity to an MHC molecule involves radioactivity or fluorescence competition binding assays. See, e.g. Ettinger et al., J. Immunol. 160:2365 (1998). In some embodiments, the competition assay yield a comparison of binding affinities of different peptides. Some MHC binding studies utilize detergent solubilized class I molecules from EBV transformed cell lines (see, e.g. Sette, A., et al., Mol Immunol, 31(11):813-22 (1994). In some embodiments, the competitive assay involves naturally loaded MHC, and the MHC molecule of interest can be purified away from other MHC molecules in the detergent lysate or be used in a mixture with other MHC molecules. In some embodiments, radiolabeled peptides can be identified that have a high affinity for the MHC molecule in question. In some embodiments, the affinity of additional "test" peptides for the MHC molecule in question is then determined by their ability to compete with the high affinity radiolabeled peptide.

In some embodiments, determining peptide affinity can involve a reconstitution assay, e.g. using "T2" cells, in which cells expressing an appropriate MHC allele are "stripped" of a native binding peptide by incubating at pH 2-3 for a short period of time. In some embodiments, to determine the binding affinity of a putative MHC-binding peptide for the same MHC allele, the stripped MHC monomer can be combined in solution with the putative MHC-binding peptide, beta2-microglobulin and a conformation-dependent monoclonal antibody. In some embodiments, the difference in fluorescence intensity determined between cells incubated with and without the test binding peptide after labeling, for example, either directly with the labeled monoclonal antibody or a fluorescence-labeled secondary antibody, can be used to determine binding of the test peptide.

In some embodiments, the binding affinity can be predicted using in silico methods. Exemplary in silico methods for predicting binding affinity are described below.

In some embodiments, the binding affinity of a peptide for an MHC (e.g. HLA) molecule is represented by an IC50, which is the concentration of peptide in a binding assay at which 50% inhibition of binding of a reference peptide is observed. In some cases, such assays can be run under conditions in which IC50 values approximate K_{D} values (i. e., limiting HLA proteins and labeled peptide concentrations). In some embodiments, binding can be expressed relative to a reference peptide.

In some embodiments, the peptide(s) is an MHC class II epitope. In some embodiments, peptides that bind to MHC class II molecules can be between 8 and 20 amino acids in length, including between 10 and 17 amino acids in length, such as or about at least or at least or about 10, 11, 12, 13, 14, 15, 16 or 17 amino acids in length, for example about 15 amino acids in length.. In some embodiments, the peptides that bind to MHC class II molecules can be longer than 20 amino acids. In some embodiments, the peptide(s) lies in an extended conformation along the MHC II peptide-binding groove. In some embodiments, the MHC II peptide-binding groove is open at both ends. In some embodiments, the peptide is held in place at least in part by main-chain atom contacts with conserved residues that line the peptide-binding groove. In some embodiments, the MHC class II is an αβ dimer that can be or include any MHC class II allelic protein known to be present in a subject, such as a human subject. In some embodiments, the MHC allele can be, but is not limited to, DR1, DR3, DR4, DR7, DR52, DQ1, DQ2, DQ4, DQ8 and DP1. In some embodiments, the MHC class II allele can be any set forth in Tables 1B, including isotype matched alpha and beta associations or mixed-isotype heterodimers combining alpha and beta chains of different alleles. In some embodiments, the MHC class II can be a dimer that is or includes an alpha and/or beta allele from among HLA-DPA1^{∗}0103, HLA-DPA1^{∗}0201, HLA-DPB 1^{∗}0101, HLA-DPB 1^{∗}0301, HLA-DPB1^{∗}0401, HLA-DPB^{∗}0402, HLA-DPB 1^{∗}1501, HLA-DRA^{∗}0101, HLA-DRB1^{∗}0101, HLA-DRB1^{∗}0102, HLA-DRB^{∗}0301, HLA-DRB^{∗}0701, HLA-DRB^{∗}0401, HLA-DRB1^{∗}1101, HLA-DRB1^{∗}1104, HLA-DRB^{∗}1501, and/or HLA-DQB1^{∗}0201.

In some embodiments, the peptide(s) is or contains an MHC class I epitope. In some embodiments, peptides that bind to MHC class I molecules can be between 7 to 15 amino acids in length. In some embodiments, peptides that bind to MHC class I molecule can be between 8 to 13 amino acids in length. In some embodiments, the binding of the peptide is stabilized at its two ends by contacts between atoms in the main chain of the peptide and invariant sites in the peptide-binding groove of all MHC class I molecules. In some embodiments, there are invariant sites at both ends of the groove which bind the amino and carboxy termini of the peptide. In some embodiments, variations in peptide length can be accommodated by a kink in the peptide backbone. In some embodiments, the kink includes proline or glycine residues, which may allow flexibility. In some embodiments, the MHC class I allele can be any known to be present in a subject, such as a human subject. In some embodiments, the MHC class I allele is an HLA-A2, HLA-A1, HLA- A3, HLA-A24, HLA-A28, HLA-A31, HLA-A33, HLA-A34, HLA-B7, HLA-B45 or HLA-Cw8 allele. In some embodiments, the MHC class I allele can be any set forth in Tables 1A, which are among the most frequent MHC class I alleles (Solberg et al., (2008) Hum Immunol. 2008 Jul; 69(7):443-6).

In some embodiments, the MHC class I allele is an HLA-A2 allele, which in some populations is expressed by approximately 50% of the population. In some embodiments, the HLA-A2 allele can be an HLA-A^{∗}0201, ^{∗}0202, ^{∗}0203, ^{∗}0206, or ^{∗}0207 gene product. In some cases, there can be differences in the frequency of subtypes between different populations. For example, in some embodiments, more than 95% of the HLA-A2 positive Caucasian population is HLA-A^{∗}0201, whereas in the Chinese population the frequency has been reported to be approximately 23% HLA-A^{∗}0201, 45% HLA-A^{∗}0207, 8% HLA-A^{∗}0206 and 23% HLA-A^{∗}0203. In some embodiments, the MHC molecule is HLA-A^{∗}0201.

In some embodiments, the peptide(s) may be produced synthetically by chemical synthesis using standard techniques (Peptide Chemistry, A practical Textbook. Mikos Bodansky, Springer-Verlag, Berlin.). For example, peptides can be synthesized by solid phase techniques (Roberge J Y et al (1995) Science 269: 202-204). In some such embodiments, synthesized peptides can be cleaved from the resin and purified, such as by preparative high performance liquid chromatography (e.g., Creighton (1983) Proteins Structures And Molecular Principles, WH Freeman and Co, New York N.Y.). In some embodiments, peptides can be generated by automated synthesis methods, for example, using the ABI 431A Peptide Synthesizer (Perkin Elmer).

In some embodiments, the peptide(s) may be made by recombinant methods. In some embodiments, peptides can be produced in a host cell transformed with a nucleic acid sequence coding for such peptide. In some cases, such host cells can be cultured in medium suitable for the cells and isolated peptides can be purified using techniques known in the art for purifying peptides. Exemplary of such methods include, for example, ion-exchange chromatography, ultrafiltration, electrophoresis or immunopurification with antibodies specific for a desired peptide. In some embodiments, peptides can be generated by cleavage from a longer polypeptide, such as by chemical or enzymatic cleavage. For example, the peptide may be obtained by cleavage from the target antigen, such as a particular chimeric receptor (e.g. CAR).

In some embodiments, the peptides are purified and isolated substantially free of any other polypeptides or contaminants, such as purified from cellular materials, other polypeptides or peptides, culture medium, chemicals or other contaminants. In some embodiments, the peptides are purified to at least 90%, 95%, 97% or greater purity. In some embodiments, the peptides are purified to homogeneity.

In some embodiments, the composition of a peptide may be confirmed by amino acid analysis or sequencing (e.g., the Edman degradation procedure).

### B. Modifications to Peptides

In some embodiments, the provided peptides include a sequence of amino acids that is related to an immunogenic region of a chimeric receptor, such as a CAR, i.e. the peptide is a modified peptide, such as a peptidomimetic, or peptide analog, or other peptide variant, that has a chemical structure that is altered compared to the immunogenic region of the chimeric receptor, such as a CAR, but nevertheless is capable of preventing, reducing or otherwise down-regulating an antigen specific immune response to the chimeric receptor. In some embodiments, a modified peptide has an altered amino acid sequence compared to the native protein sequence from which it is derived (e.g. a chimeric receptor, such as a CAR), such as by amino acid substitution, deletion or addition or by substitution of chemical bonds or linkages.

In some embodiments, the peptide(s) can be a peptidomimetic that mimics the immunogenic activity of a peptide derived from a chimeric receptor, such as a CAR. In some embodiments, a peptidomimetic can be based on any immunogenic region of a chimeric receptor (e.g. CAR), such as any of the peptides described herein. In some embodiments, the peptidomimetic can include a compound not joined entirely of subunits joined by peptide bonds, but joined by other linkages, such as thiolester bonds, reduced bond analogs or aminide bond isosterases. In some embodiments, the normal amide bond can be replaced by ester or alkyl backbone bonds, N- or C-alkyl substituents, side chain modifications, and constraints such as disulphide bridges and side chain amide or ester linkages can be included.

In some embodiments, the peptide(s) can be modified to increase solubility or stability, which may result in greater efficacy, *in vivo* stability of the peptide, or a longer biological lifetime. In some embodiments, the peptide is modified with a polymer, such as polyethylene glycol (PEG), monomethoxypolyethylene glycol (mPEG) or polyvinyl alcohol (PVA).

In some embodiments, the peptide(s) is modified to enhance its *in vivo* efficacy, for example by improving the ability to down-regulate the immune response to a chimeric receptor (e.g. CAR). In some embodiments, residues involved in TCR recognition can be determined using known techniques, for example, substitution of each residue and determination of the presence or absence of MHC binding and/or T cell reactivity. Those residues shown to be required or involved in interacting with the TCR can be modified by replacing such an amino acid or amino acids with another amino acid, such as a similar amino acid residue, for example a conservative substitution, and the modified peptide can be assessed for MHC binding, T cell reactivity and/or ability to down-regulate the immune response to a chimeric receptor (e.g. CAR). In some embodiments, modification of epitopes may be performed based on predictions for more efficient T-cell induction derived using the program "Peptide Binding Predictions" (see Parker, K. C et al. 1994. J. Immunol. 152:163).

In some embodiments, peptides can be modified to incorporate one or more polymorphisms based on the amino acid sequence of the protein antigen resulting from natural allelic variation. In some embodiments, D-amino acids, non-natural amino acids, or non-amino acid analogs can be substituted or added to produce a modified peptide. In some embodiments, modifications of peptides can include reduction/alkylation, acylation, chemical coupling to an appropriate carrier, or mild formalin treatment.

In some embodiments, a peptide(s) can be modified by adding a reporter group(s) to the peptide backbone. For example, in some cases, addition of a reporter group can facilitate purification or increase solubility of peptides. In some embodiments, poly-histidine can be added to a peptide, for example, to purify the peptide on immobilized metal ion affinity chromatography. In some embodiments, a specific endoprotease cleavage site can be introduced between a reporter group and amino acid sequence to facilitate isolation of peptides free of such reporter groups. In some embodiments, to ensure proper antigen processing of T cell epitopes within a peptide, canonical protease sensitive sites can be recombinantly or synthetically engineered before or after a T cell epitope so that it is not disrupted. In an exemplary embodiment, charged amino acid pairs, such as KK or RR, can be introduced between regions within a peptide during recombination construction or synthesis of the peptide. In such an example, the resulting peptide can be rendered sensitive to cleavage by cathepsin and/or other trypsin-like enzymes to generate portions of the peptide containing a T cell epitope.

In some embodiments, a peptide(s) can be modified by substitution of cysteine residues, such as to serine, threonine, leucine or glutamic acid, which can minimize dimerization via disulfide linkages. In some embodiments, peptides can be modified by adding a functional group or groups to the peptide, for example, to increase solubility of the peptide in a buffered aqueous solution such as a pharmaceutically acceptable carrier or diluent. For example, in some aspects, charged amino acids or charged amino acid pairs or triplets can be added to the carboxy terminus or amino terminus or both, of the peptide. Exemplary charged amino acids include, for example, arginine (R), lysine (K), histidine (H), glutamic acid (E) and aspartic acid. In some embodiments, an amino acid or amino acids can be deleted of substituted from the N-or C-terminus, for example, to increase the efficiency of peptide synthesis. For example, in some aspects, the N-terminal or C-terminal amino acid of the peptide may be capable of cyclization, or may be subject to degradation either during or after peptide synthesis. In such an example, one or more additional amino acids may be added to "block" the less desirable amino or carboxy-terminal amino acid. Such added amino acids may either be derived from the native protein sequence or may be a non-native amino acid residue.

In some embodiments, a modified peptide has a sequence that exhibits at least 90%, 95%, 96%, 97%, 98%, 99%, or 99.5% sequence identity to any of SEQ ID NOS: 6, 8-12, 16-22, 137, 160, 163, 165, 166, 167 or 168, so long as the modified peptide retains activity to bind to an MHC and/or to prevent, reduce or otherwise down-regulate an immune response to a corresponding chimeric receptor. In some embodiments, a modified peptide is a peptide analog or a peptidomimetic of a peptide set forth in any of SEQ ID NOS: 6, 8-12, 16-22, 137, 160, 163, 165, 166, 167 or 168, so long as the modified peptide binds to an MHC and/or retains activity to prevent, reduce or otherwise down-regulate an immune response to a corresponding chimeric receptor. Methods of assessing activity of a peptide are known in the art, and exemplary assays are described below. In some embodiments, the modified peptide retains its ability to bind to an MHC molecule or its ability to elicit an immune response under certain conditions compared to the corresponding unmodified peptide. In some embodiments, the modified peptide retains activity to downregulate the immune response to a chimeric receptor (e.g. CAR), such as determined in a humanized mouse model. Generally, a peptide retains a recited activity if it exhibits at least 75%, 80%, 85%, 90%, 95% or more of the recited activity of the unmodified peptide.

### C. Methods of Identifying Peptides

In some embodiments, peptides can be identified by determining immunogenic regions of a protein, including, for example, immunogenic regions of a chimeric receptor. In some embodiments, the peptide is or includes the identified immunogenic region. The chimeric receptor may include any as described herein or known in the art, including antigen receptors, such as chimeric antigen receptors (CARs) or non-antigen binding chimeric receptors.

In some embodiments, the immunogenic region can be identified by its ability to bind to an MHC molecule or by its ability to elicit an immune response under certain conditions.

In some embodiments, predicting if a peptide of interest binds to a given MHC molecule includes analyzing the peptide for binding motifs likely to bind to a particular MHC molecule. In some embodiments, overlapping peptides of a chimeric receptor, such as overlapping 8mer to 20 mer peptides, such as 9mers, 10mers, 11mers, 12mers, 13mers, 14mers or 15mers can be assessed for MHC binding using algorithmic or other computational methods. See, for example, Marsh, et al., The HLA Factsbook (Academic Press, 2000). In some embodiments, an algorithm can be used to predict if a peptide of interest should bind to a given MHC molecule. See, e.g., Southwood, et al., J. Immunol. 160:3363 (1998); Honeyman, et al., Nat. Biotechnol. 16:966-969 (1998); Breisie, et al., Bioinformatics 14:121-131 (1998), as well as the "SYFPEITHI" algorithm (Hans-Georg Rammensee, et al., Immunogenetics (1999) 50: 213-219), Zhang et al., PLoS ONE 7(2): e30483. doi: 10.1371/journal.pone.0030483, the Immune Epitope and Analysis Resource (IEDB) (Peters B, et al. PLoS Biology 3: 379 (2005)), and the "BIMAS" algorithm (Parker, K. C., M. A. Bednarek, and J. E. Coligan. J. Immunol. 152:163 (1994).

In some embodiments, algorithm prediction tools, including those available from IEDB, use one or more predictions using ANN (Nielsen et al. (2003) Protein Sci., 12:1007-1017 and Lundegaard et al. (2008) NAR, 36:W509-512), SMM (Peters and Sette (2005) BMC Bioinformatics, 6:132) and comblib (Sidney et al. (2008) Immunome Res. 4:2), or the Consensus tool (see Kim, et al. (2012) Immune epitope database analysis resource, NAR, combining predictions from any of the foregoing).

In some embodiments, prediction of antigen processing can be accomplished using an algorithm for proteosomal cleavage (PaProC). See Kuttler et al., J. Mol. Biol. 298 (2000), 417-429 and Nussbaum et al., Immunogenetics 53 (2001), 87-94.

In some embodiments, a chimeric receptor, such as a CAR, can be assessed to determine if it is immunogenic by assessing an immune response to the molecule. In some embodiments, a subject can be treated with a chimeric receptor, such as a CAR, for example, by administering cells genetically engineered with the chimeric receptor or CAR. The presence of a humoral or cell-mediated human response can be assessed. In some embodiments, the presence of antibodies that specifically bind to and/or neutralize binding epitopes of the chimeric receptor (e.g. CAR) can be identified by methods such as ELISpot, intracellular cytokine staining, ELISAs (e.g. for cytokines), or cell-based antibody detection methods, for example, by flow cytometry, on serum from the subject. In some embodiments, a cell-mediated immune response to the chimeric receptor, such as a chimeric receptor expressed on engineered cells, can be assessed using a cytotoxic T-lymphocyte (CTL) assay for detection of CD8+ T cells that specifically bind to and induce cytotoxicity and/or a mixed lymphocyte reaction, using cells, e.g., irradiated cells, expressing the chimeric receptor, as stimulator cells.

In some embodiments, the particular immunogenic region can be identified in an in vitro assay by stimulating isolated immune cells (e.g. PBMCs) from a subject administered with a chimeric receptor (e.g. CAR) with a synthetic library of peptides which overlap and span the length of the particular antigen. The immune response in stimulated cells can be assessed using methods that include, but are not limited to, ELISpot, intracellular cytokine staining, cytotoxic T-lymphocyte assays and/or mixed lymphocyte reactions.

In some embodiments, overlapping peptide studies can indicate the area of the antigen in which an epitope is located. The minimal epitope for a particular T cell can then be assessed by measuring the response to truncated peptides. For example, if a response is obtained to the peptide comprising residues 1-15 in the overlapping library, sets which are truncated at both ends (i.e. 1-14, 1-13, 1-12 etc. and 2-15, 3-15, 4-15 etc.) can be used to identify the minimal epitope.

In some embodiments, an immunogenic region may be identified by mass spectrometric analysis of peptides eluted from antigen-loaded APC. In some embodiments, these APC have either been encouraged to take up antigen (e.g. a chimeric receptor), or have been forced to produce the protein intracellularly by transformation with the appropriate gene. In some embodiments, APC can be incubated with protein either in solution or suitably targeted to the APC cell surface. After incubation at 37° C the cells can be lysed in detergent and the MHC protein purified by, for example affinity chromatography. Treatment of the purified MHC with a suitable chemical medium (for example, acid conditions) can result in the elution of peptides from the MHC. This pool of peptides can be separated and the profile compared with peptide from control APC treated in the same way. The peaks unique to the protein expressing/fed cells can be analyzed (for example by mass spectrometry, including tandem mass spectrometry (MS/MS), or high-performance liquid chromatography electrospray ionization mass spectrometry (HPLC ESI MS)) and the peptide fragments identified. This procedure can generate information about the range of peptides (sometimes found in "nested sets") generated from a particular antigen by antigen processing.

In some embodiments, the immunogenic region is identified by screening a synthetic library of peptides which overlap and span the length of the antigen in an in vitro assay. For example, peptides which are 15 amino acids in length and which overlap by 5 or 10 amino acids may be used. The peptides can be tested in an antigen presentation system which comprises antigen presenting cells and T cells. For example, the antigen presentation system may be a murine splenocyte preparation, or a preparation of human cells from tonsil or PBMC. Alternatively, the antigen presentation system may comprise a particular T cell line/clone and/or a particular antigen presenting cell type.

In some embodiments, T cell activation is measured via T cell proliferation (for example using 3H-thymidine incorporation) or cytokine production. Activation of TH1-type CD4+ T cells can, for example, may be detected via IFNγ production which may be detected by standard techniques, such as an ELISPOT assay.

In some embodiments, a peptide that is or includes an immunogenic region of a chimeric receptor antigen can be assessed for its ability to induce tolerance in a subject *in vivo.* In some embodiments, a humanized mouse model is employed. Exemplary of such mouse models include HLA-A^{∗}0201 transgenic mouse model (see, e.g. Tey, et al. Immunology and Cell Biology. 84:281 (2006)). In some embodiments, the mouse model expresses both HLA-A^{∗}0201 class I and HLA-DR1 class II molecules (see e.g. BenMohamed L, et al. Immunol. 2000 August;61 (8):764-79). Other transgenic mouse models are well-known to a skilled artisan.

In some embodiments, the transgenic mouse is co-administered (e.g. prior to, subsequently or intermittently) a potential or candidate peptide and a chimeric receptor (e.g. CAR), such as cells genetically engineered with the chimeric receptor. Generally, the peptide is administered under one or more conditions that may or is likely to induce tolerance. For example, the peptide is administered in the absence of adjuvant. Immune cells from the mice, such as splenocytes, can be isolated and assayed for a humoral or cell-mediated immune response as described, for example, to determine if co-administration with the peptide prevents, reduces or down-regulates the immune response compared to mice administered the chimeric receptor but without co-administration with the peptide. For example, in some embodiments, splenocytes from primed mice are isolated and rechallenged in culture with the same candidate peptide or peptides, or optionally with gamma-irradiated cells transduced with a CAR containing the peptide sequence. Rechallenge can occur at any point after the initial challenge, including, for example 7 days, 10 days, 14 days, 21 days, 28 days, 35 days, or 42 days post-administration. In some embodiments, cells can be derived from challenged mice and assessed for an immune response to the rechallenge by chromium release assay, intracellular cytokine staining, ELISpot assay, and/or clonal TCR sequencing. In some embodiments, a control subset of mice can be administered peptides in the presence of adjuvant and/or are administered control non-MHC binding peptides.

For example, an enzyme-linked immunospot (ELISpot) assay can be employed to assess an immune response to the chimeric receptor, such as in the presence or absence of co-administration with the peptide. In some aspects, an ELISpot can be adapted for the detection of individual cells secreting specific cytokines or other effector molecules by attachment of a monoclonal antibody specific for a cytokine or effector molecule on a microplate. Cells stimulated by an antigen can be contacted with the immobilized antibody. After washing away cells and any unbound substances, a tagged polyclonal antibody or more often, a monoclonal antibody, specific for the same cytokine or other effector molecule can be added to the wells. Following a wash, a colorant that binds to the tagged antibody can be added such that a blue-black colored precipitate (or spot) forms at the sites of cytokine localization. The spots can be counted manually or with automated ELISpot reader system to quantitated the response.

In some embodiments, CD8+ T cell-mediated cytotoxic activity can be assessed to assess the presence or absence or level or degree of an immune response to the recombinant protein or cell, e.g., to the chimeric receptor, such as in the presence or absence of co-administration with the peptide. In some embodiments, cells stimulated with an antigen (e.g. splenocytes) can be analyzed by chromium release assay for cytotoxicity against ⁵¹Cr-labeled cells. This assay can measure the release of (biological) radioactive chromium from cells as a result of killer cell activity. The cells can be re-stimulated with an antigen in vitro, for example, cells genetically engineered with a chimeric receptor (e.g. CAR-expressing cells), including T cells, peptide-loaded T cells and/or non-transduced ("Mock") T cells (targets) at various effector-to-target (E/T) ratios. Following co-incubation, release of chromium can be quantified and the percentage of maximum achievable lysis in each sample determined. Conditions in which no cytolytic activity specific is detected indicates that a specific immune responses to the agent, e.g., the chimeric receptor did not develop following co-administration with the peptide. This result may indicate a potential hit for administration of a specific peptide under conditions capable of inducing immunogenic tolerance to the chimeric receptor.

In some embodiments, intracellular cytokine staining is employed to assess an immune response to the chimeric receptor, such as in the presence or absence of co-administration with the peptide. Immune cells, such as splenocytes, can be isolated from the mice and stained with antibodies to detect CD8 and CD4 surface expression and intracellular expression of cytokine, including, for example, IFNγ. Conditions in which intracellular cytokine staining indicates T cells did not generate a specific immune response to the chimeric receptor may indicate a potential hit for administration of a specific peptide under conditions capable of inducing immunogenic tolerance to the chimeric receptor.

In some embodiments, clonal sequencing is employed to assess an immune response to the agent, e.g., the chimeric receptor, such as in the presence or absence of co-administration with the peptide. T cell receptors from T cells isolated from challenged mice or patients can be sequenced (see Arstila et al. (1999) Science 286, 958-961; WO 2012/083069). The absence of clonal TCR expansion indicates T cells did not generate a specific immune response to the chimeric receptor. This may indicate a potential hit for administration of a specific peptide under conditions capable of inducing immunogenic tolerance to the chimeric receptor.

### D. Compositions

Also provided are compositions containing a peptides or peptides, such as those provided and/or used in the provided methods, including pharmaceutical compositions and formulations. The pharmaceutical compositions and formulations generally include one or more optional pharmaceutically acceptable carrier or excipient.

In some aspects, the choice of carrier is determined in part by the particular peptide and/or by the method of administration. Accordingly, there are a variety of suitable formulations. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

In some embodiments, a pharmaceutical carrier can include at least one excipient, such as sterile water, sodium phosphate, mannitol, sorbitol, sodium chloride or any combination thereof. In some embodiments, other pharmaceutically acceptable carriers may include solvents or dispersion medium containing, for example, water, ethanol, polyol (e.g. glycerol, propylene glycol and liquid polyethylene glycol) and vegetable oils. In some embodiments, prevention of the action of microorganisms can be achieved by various antibacterial and antifungal angents, for example, parabens, chlorobutanol, phenol, ascorbic acid and thimerosol.

In some embodiments, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition.

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

Compositions in some embodiments are provided as sterile liquid preparations, e.g., isotonic aqueous solutions, suspensions, emulsions, dispersions, or viscous compositions, which may in some aspects be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the cells in a solvent, such as in admixture with a suitable carrier, diluent, or excipient such as sterile water, physiological saline, glucose, dextrose, or the like. The compositions can contain auxiliary substances such as wetting, dispersing, or emulsifying agents (e.g., methylcellulose), pH buffering agents, gelling or viscosity enhancing additives, preservatives, flavoring agents, and/or colors, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, and sorbic acid. Prolonged absorption of the injectable pharmaceutical form can be brought about by the use of agents delaying absorption, for example, aluminum monostearate and gelatin.

The formulations to be used for in vivo administration are generally sterile. Sterility may be readily accomplished, e.g., by filtration through sterile filtration membranes.

The formulations can include aqueous solutions. In some embodiments, the peptide is formulated as a lyophilized composition, which can be reconstituted as an aqueous or liquid composition in a pharmaceutically acceptable carrier, such as sterile water, prior to use. In some embodiments, a sterile powder can be prepared by vacuum drying, freeze-drying or spin drying.

The formulation or composition may also contain more than one active ingredient useful for the particular indication, disease, or condition being treated with the peptide, preferably those with activities complementary to the peptide, where the respective activities do not adversely affect one another. Such active ingredients are suitably present in combination in amounts that are effective for the purpose intended.

The pharmaceutical composition in some embodiments is formulated to contain a tolerizing amount of peptide for administration. The composition can be formulated to contain a single dosage or multiple dosages of the peptide. In some embodiments, the peptide is formulated to be soluble at a concentration which permits its use in vivo. For example, in some embodiments the peptide is soluble at concentrations of up to 0.5 mg/ml, concentrations of up to 1 mg/ml, and/or at concentrations of up to 5 mg/ml.

### E. Methods of Peptide Administration

Also provided are methods that generally involve administering to a subject a peptide(s), including any as described above. For example, in some embodiments, the methods include administering a peptide or peptides set forth in any of SEQ ID NOS: 6, 8-12, 16-22, 137, 160, 163, 165, 166, 167 or 168 or a peptidomimetic, analog or variant thereof that retains activity, i.e. activity to prevent, reduce or otherwise downregulate an immune response to co-administered cells engineered with a chimeric receptor. Generally, the peptide(s) is co-administered with cells expressing a chimeric receptor (e.g. CAR) in connection with adoptive cell therapy, for example for treating a disease or condition, such as a cancer. The peptide(s) can be co-administered to a subject prior to, simultaneous to, intermittently with, or subsequent to administration of the cells expressing the chimeric receptor (e.g. CAR). Typically, the peptide(s) is administered prior to administering cells expressing the chimeric receptor (e.g. CAR). Thus, in some embodiments, the methods improve efficacy of treatment with the chimeric receptor (e.g. CAR) by increasing exposure of the subject to the administered cells by preventing or reducing host immune responses that would otherwise clear or prevent expansion of the administered cells.

As used herein, a "subject" is a mammal, such as a human or other animal, and typically is human. In some embodiments, the subject is one who, at the time of treatment, is naive to the antigen, for example, the subject has not been previously administered cells expressing the chimeric receptor, such as a CAR. In some embodiments, the subject is receiving or will receive therapy with a chimeric antigen, such as a CAR, that may be immunogenic, such as in the context of cells engineered with the chimeric receptor (e.g. CAR) in connection with adoptive cell therapy.

In some embodiments, the peptide(s) that is administered to the subject corresponds to an immunogenic region present in the chimeric receptor (e.g. CAR) that is to be co-administered to the subject, for example, in the context of cells genetically engineered to express the chimeric receptor. The term "corresponds to" means that the peptide contains an immunogenic region that is derived from or based on, i.e. that is or includes or is related to, an immunogenic sequence of amino acids present in the chimeric receptor to be administered to the subject. In some cases, the peptide need not contain a chemical structure, including a sequence of amino acids, that is identical to those present in an immunogenic region of the chimeric receptor, such as a CAR, so long as the peptide is sufficiently related or similar to such a region to be able to prevent, reduce or down-regulate an immune response to such a chimeric receptor when co-administered. For example, a peptide that is modified compared to an immunogenic region present in a chimeric receptor, e.g. the peptide is a peptidomimetic, analog or amino acid variant containing one or more amino acid mutations (i.e. deletions, substitutions/replacements or insertions), can nonetheless correspond to a particular chimeric receptor if the peptide can prevent, reduce or down-regulate an immune response to such a chimeric receptor when co-administered.

Reference to "a corresponding" chimeric receptor, such as a CAR, refers to a chimeric receptor that contains an immunogenic sequence of amino acids from which the peptide is based on or derived and/or for which the peptide is capable of preventing, reducing or otherwise down-regulating an immune response to the chimeric receptor, such as a CAR, when it is co-administered to a subject.

In some embodiments, the subject is administered a peptide(s) with known efficacy specific to the subject's MHC allele or allege group, such as where the peptide is known to bind or be presented particularly well to or by-or to induce a particularly good signal when bound to-the MHC allele or group, e.g., as compared to others. In some embodiments, prior to administering a peptide to a subject, a subject is selected for expression of an MHC allele based on the known efficacy for a particular MHC by the peptide. In some embodiments, the subject's MHC alleles can be determined using methods known in the art, for example by using molecular haplotype assays (BioTest ABC SSPtray, BioTest Diagnostics Corp., Denville, NJ; SeCore Kits, Life Technologies, Grand Island, NY). For example, in the case where the peptide is specific to or advantageous in the context of an HLA-A^{∗}0201 allele (e.g. a peptide set forth in any of SEQ ID NOS: 8, 9, 10 or 11), a subject is selected that expresses the HLA-A^{∗}0201 allele.

In some embodiments, the subject is administered the peptide(s) under conditions that are capable of inducing tolerance to the agent, e.g., cell or protein such as chimeric receptor (e.g. CAR), e.g. any condition of peptide administration, peptide formulation and/or peptide form that prevents, reduces or otherwise effects down-regulation of an antigen-specific immune response to a co-administered chimeric receptor (e.g. CAR) compared to a host immune response to the chimeric receptor (e.g. CAR) in the absence of the peptide. Conditions known to induce tolerance include, but are not limited to, administration of the peptide in soluble form, administration in the absence of adjuvant, administration in the presence of adjuvant with a tolerogenic agent, administration in a low dose amount (which, in some cases, can be repeated), administration of a high dose amount, or administration intraperitoneally (i.p.), intravenously (i.v.), intranasally (i.n.), or orally, and combinations of any of the foregoing. Other routes of administration also are contemplated depending on the one or more other conditions employed to induce tolerance. In some embodiments, the peptide is not administered subcutaneously or intradermally.

In some embodiments, preventing, reducing or down-regulating a host immune specific response to a specific antigen, for example, a chimeric receptor, can be determined clinically, or may be determined subjectively, such as based on the efficacy or response to the CAR, i.e. the patient feels as if some or all of the symptoms related to the disease or condition being treated have been alleviated without any immune-mediated side effects. In some embodiments, signs or symptoms of an immune response can include any detectable indicator of an immune response, e.g. a humoral or cell-mediated immune response, such as generation of neutralizing or non-neutralizing antibodies to the chimeric receptor (e.g. CAR) or presence of CD8+ T cells that induce cytotoxicity. Other indicators of an immune response can include infusion reactions or acute reactions, such as anaphylaxis or cytokine release syndrome. For example, in some cases, no host immune response to the agent, e.g., chimeric receptor (e.g. CAR) is observed in embodiments in which a peptide is co-administered.

In some embodiments, the peptide(s) is administered to the subject in soluble form in an aqueous solution.

In some embodiments, the peptide(s) is administered in the absence of adjuvant. In general, protein antigens administered with adjuvants, such as pro-inflammatory adjuvants, favor a robust immune response to the antigen. For example, in some cases, adjuvants can stimulate release of cytokines and expression of costimulators on antigen presenting cells (APCs), thereby accounting for their immunostimulatory activity. In some cases, administering a peptide antigen in the absence of adjuvant has been shown to induce T cell tolerance. For example, it has been demonstrated that mice exposed to an antigenic peptide in an immunogenic form (with adjuvant) showed great expansion of T cells, but not mice exposed to the same peptide administered in a tolerogenic form (large dose of aqueous peptide without adjuvant) (Pape KA et al., Immunological Reviews 156:67-78, 1997).

In some embodiments, the peptide(s) is administered in the presence of adjuvant and a tolerogenic agent (or a toleragenic agent and no antigen) (see e.g. published international application WO2010/056143). The term "tolerogenic agent" refers to any agent that interferes with adaptive immune response, e.g., with T cell activation, proliferation, expansion, survival, or differentiation to a memory or long-lived state, such as by targeting molecules that are involved in T cell activation and/or interfering with the interaction between a T cell and an antigen presenting cell (APC). In some embodiments, the tolerogenic agent is an agent that blocks a co-stimulation signal. In some embodiments, the tolerogenic agent is an agent that binds to a cell surface molecule expressed on a T cell or an antigen presenting cell. In some embodiments, the tolerizing agent targets one or more molecules that are expressed on T cells and involved in T cell activation or other stimulatory signal, or inhibition thereof, which can include, but are not limited to, CD4, CD40, CD40L, OX40, OX40L, CD26, CD44, CD28, PD1, BTLA, B7-1, ICOS, CTLA-4, B7-2 family, CD99, CD137 (4-1BBL), CD2, LFA3, CD27, CD70, CD3, CD8, ICAM1, LFA1, MHC class II or MHC class I molecules. In some embodiments, the tolerogenic agent can be an antibody, such as a monoclonal antibody, that binds to and blocks the action of one or more molecules in involved in T cell activation, such as listed above. In some embodiments, the tolerogenic agent can be a soluble fusion protein derived from a protein that specifically binds to molecule involved in T cell activation, such as listed above. Exemplary tolerogenic agents include, but are not limited to, antagonistic or blocking anti-CD4, antagonistic or blocking anti-CD3, antagonistic or blocking anti-CD25, antagonistic or blocking anti-CD28, agonistic or signal-inducing anti-PDl, anti-BTLA, blocking anti-B7 (anti-B7-1 or anti-B7-2), blocking or antagonistic anti-ICOS, anti-CTLA, anti-CD40, anti- CD40L, anti-CD99, anti-CD2, anti-LFA3, anti-CD27, anti-CD70, anti-DC8, anti-OX40, anti- OX40L, anti-LFA-1, anti-CD11a, anti-ICAM1, anti-CD26, anti-CD44, anti-CD137 (anti-4- IBBL), CTLA4-Ig, anti-MHC class II and anti-MHC class I, or antigen-binding fragments thereof. In some embodiments, the tolerogenic agent is administered in an amount that is from or from about 1 mg to 1000 mg, such as 25 mg to 600 mg. The tolerogenic agent can be administered by injection, such as intravenous or subcutaneous injection.

In some embodiments, the tolerogenic agent is an anti-CD3 antibody, such as Muromonab-CD3 (Orthoclone OKT3^{®}), which is an anti-CD3 monoclonal antibody that is commonly used to suppress or prevent transplant rejection. In some embodiments, the tolerogenic agent targets and/or specifically binds to, and optionally induces signaling by or mediated by, one or more molecules that is expressed on a non-T cell, such as an antigen presenting cell (APC), e.g., a macrophage or a dendritic cell, e.g., a cell that serve as the ligand/receptor pair for any of the aforementioned molecules identified above expressed on the T cells, such as PD-L1, PD-L2 and B7 family members.

In general, an adjuvant is not co-administered. In some cases, the adjuvant can be administered with a peptide(s), such as in the presence of a tolerogenic agent. The adjuvant in some embodiments, can be or include a toxin derived from microbiological organisms, such as bacterial products, including those derived from *Corynebacterium diphtheriae, Clostridium tetani, Bordetella pertussis,* and *Mycobacterium bovis.* In some cases, adjuvants can include inorganic compounds, organic compounds, plant saponins, and cytokines. Inorganic compounds used in adjuvants can include alum, aluminum hydroxide, aluminum phosphate, calcium phosphate hydroxide. Organic compounds used in adjuvants can include squalene and thimerosal. Plant saponins include those from Quillaja, Soybean, and *Polygala senega.* Cytokines used in adjuvants can include IL-1, IL-2, and IL-12. Additional examples of adjuvants include Freund's complete adjuvant and Freund's incomplete adjuvant. In some embodiments, the adjuvant is alum (aluminum hydroxide, aluminum phosphate); mineral oil, non-mineral oil, water-in-oil emulsions, oil-in-water emulsions, Seppic ISA series of Montanide adjuvants (e.g., Montanide ISA 720, AirLiquide, Paris, France); MF-59 (a squalene-in-water emulsion stabilized with Span 85 and Tween 80; Chiron Corporation, Emeryville, Calif); PROVAX (an oil-in-water emulsion containing a stabilizing detergent and a micelle-forming agent; IDEC, Pharmaceuticals Corporation, San Diego, Calif.); saponins, including saponins purified from the bark of the Q. saponaria tree, such as QS-21 (Aquila Biopharmaceuticals, Inc., Worcester, Mass.); poly[di(carboxylatophenoxy)phosphazene (PCPP; Virus Research Institute, USA); derivatives of lipopolysaccharides such as monophosphorlyl lipid (MPL; Ribi ImmunoChem Research, Inc., Hamilton, Mont.), muramyl dipeptide (MDP; Ribi) and threonyl muramyl dipeptide (tMDP; Ribi); OM- 174 (a glucosamine disaccharide related to lipid A; OM Pharma SA, Meyrin, Switzerland); and Leishmania elongation factor (a purified Leishmania protein; Corixa Corporation, Seattle, Wash.); ISCOMS (Immunostimulating complexes which contain mixed saponins, lipids and form virus-sized particles with pores that can hold antigen; CSL, Melbourne, Australia); SB-AS2 (SmithKline Beecham adjuvant system #2 which is an oil-in- water emulsion containing MPL and QS21 : SmithKline Beecham Biologicals [SBB]. Rixensart, Belgium); SB-AS4 (SmithKline Beecham adjuvant system #4 which contains alum and MPL; SBB, Belgium); non-ionic block copolymers that form micelles such as CRL 1005 (these contain a linear chain of hydrophobic polyoxpropylene flanked by chains of polyoxyethylene; Vaxcel, Inc., Norcross, Ga.); Syntex Adjuvant Formulation (SAF, an oil- in-water emulsion containing Tween 80 and a nonionic block copolymer; Syntex Chemicals, Inc., Boulder, Colo.) or an immunostimulatory nucleic acid molecule. In some embodiments, the adjuvant is a pro-inflammatory adjuvant. In some cases, an adjuvant may be mixed together with an antigen.

If an adjuvant is used, the adjuvant is generally administered at the same time as the peptide(s), and at the same location such that the effects of the adjuvant are present when the immune system sees the peptide. Generally, the adjuvant and peptide are administered together as a single formulation. In such aspects, a tolerogenic agent is generally administered in conjunction with the peptide and adjuvant. The tolerogenic agent can be administered prior to, simultaneously, intermittently or subsequent to administration of the peptide and adjuvant. In some embodiments, the tolerogenic agent may be formulated in the same preparation with the peptide/adjuvant or in a separate formulation.

In some embodiments, the peptide(s) is administered orally, sublingualis buccally, intranasally, intravenously, intramuscularly, intrathecally, intraperitoneally, or subcutaneously. In some embodiments, the peptide(s) is administered orally, intraperitoneally, intranassaly or intravenously. The peptide(s) and compositions may be administered using standard administration techniques, formulations, and/or devices.

The peptide(s) is administered in a therapeutically effective amount, which is an amount that, under certain conditions of administration, is suitable and capable of inducing inducing tolerance, i.e. preventing, reducing or otherwise down-regulating an immune response, to a co-administered chimeric receptor (e.g. CAR). The particular dosage amount can be empirically determined, and may depend on the particular chimeric receptor (e.g. CAR), the particular peptide, the age and physical condition of the subject being treated, the immune status of the subject, the duration of treatment, the nature of any concurrent treatment (if any), the specific route of administration, the presence or absence of adjuvant and, optionally, a tolerogenic agent and other factors within the level of a skilled artisan.

In some embodiments, the peptide(s) is administered at a high dose (*e.g.* 100 mg to 1000 mg, such as 200 mg to 600 mg, for example about 500 mg). In some cases, administration of high doses can induce T cell unresponsiveness, such as by anergy and/or deletion. In some embodiments, the peptide(s) is administered at a low dose (e.g. 0.1 mg to 5 mg, such as 0.2 mg to 2 mg). In some cases, administration of low doses can result in bystander suppression due to activation of regulatory T cells that can secrete suppressive cytokines, such as TGF-β, IL-4 or IL-10.

In some embodiments, administering the peptide(s) includes administering a dose of between 0.1 mg and 1000 mg of peptide to a subject, such as at least 0.1 mg, 0.25 mg, 0.5 mg, 0.75 mg, 1 mg, 2 mg, 1.5 mg, 2 mg, 2.5 mg, 3 mg, and 4 mg, 5 mg, 10 mg, 50 mg, 100 mg, 200 mg, 300 mg, 400 mg, 500 mg or more per subject. In some embodiments, the peptide(s) is administered in an amount from 0.005 mg/kg of the subject to about 500 mg/kg of the subject, such as from about 0.01 mg/kg to about 100 mg/kg.

In some embodiments, the peptide(s) is administered as a single dose. In some embodiments, the dose is administered in multiple doses, such as repeated doses. Studies in mice have demonstrated that, in some cases, repeated doses of peptide may be required to induce tolerance (Burkhart et al. (1999)). The exact dose and number of doses of peptide(s) may depend on the individual or the route of administration. In some embodiments, the peptide(s) is administered a plurality of times, including, for example, 2, 3, 4, 5, 6, 7, 8, 9, or 10 times. In some embodiments, the peptide(s) is administered at least once daily for two days, three days, four days, five days, six days or seven days. In some embodiments, repeated low dose amounts of the peptide are administered.

In some embodiments, a plurality of peptides, such as each including an epitope of a cell or recombinant protein therein such as a chimeric receptor (e.g. CAR) is administered. In some embodiments each of the peptides contains all or a portion of an immunogenic region of the chimeric receptor (e.g. CAR). In some embodiments, each of the plurality of peptides binds to the same MHC allele. In some embodiments, the plurality of peptides binds to one or more different MHC alleles. In some embodiments, such combinations of peptides can be administered simultaneously or sequentially. In some embodiments, when administered simultaneously, the peptides can be administered as a mixture in a single composition. In some embodiments, when administered simultaneously, one or more of the peptides are administered in separate compositions.

In some embodiments, the peptide(s) is administered prior to, simultaneously, subsequently or intermittently from administration of cells genetically engineered to express a corresponding chimeric receptor (e.g. a CAR). In general, the peptide(s) is administered separately from cells expressing the chimeric receptor, such as CAR-expressing cells, but in some embodiments, it is administered together with such cells. Generally, the peptide(s) is administered prior to administering chimeric receptor-expressing cells, such as prior to administering the agents to be administerd, such as the recombinant molecule-expressing, e.g., CAR-expressing, cells.

In some embodiments, the peptide(s) is administered prior to administering cells genetically engineered with the molecule. In some embodiments, the peptide(s) is administered a sufficient of time prior to effect the tolerization of immune cells or response against the agent to be administered. In some embodiments, the time between administration of the peptide(s) and administration of the molecule, e.g., chimeric receptor, such cells genetically engineered with the chimeric receptor, is 1 day to 60, such as 7 to 28 days, for example 14 to 21 days. In some embodiments, the time is at 5, 6, 7, 8, 9, 10, 11, 12, 13, 14, 15, 16, 17, 18, 19, 20, 21, 22, 23, 24, 25, 26, or 27 days.

In some cases, the peptide(s) is administered separately and is administered from or from about 1 hour to 96 hours before or after administering cells genetically engineered to express the corresponding chimeric receptor, such as from or from about 2 hours to 48 hours, 6 hours to 36 hours or 12 hours to 24 hours, each inclusive, before or after administering cells genetically engineered to express the corresponding chimeric receptor.

In some embodiments, the subject can be further administered an immunosuppressive agent. Non-limiting examples of immunosuppressive agents include, but are not limited to, antilymphocytic serum or cyclosporine, or chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, and/or vincristine. The immunosuppressive agent can be administered prior to, simultaneously, intermittently or subsequently to administration of the peptide and/or cells, e.g., genetically engineered with the chimeric receptor, such as CAR-expressing cells.

### III. Cells Genetically Engineered to Express Chimeric Receptors, Compositions, Dosages, and Methods of Administration

Provided are cells for adoptive cell therapy, e.g., adoptive immunotherapy, and method for producing or generating the cells. The cells include immune cells such as T cells. The cells generally are engineered to express a recombinant receptor, such as a chimeric receptor. The receptors may include antigen receptors, such as functional non-TCR antigen receptors, including chimeric antigen receptors (CARs), and other chimeric antigen-binding receptors such as, in some cases, transgenic T cell receptors (TCRs). The receptors may also include other chimeric receptors, such as receptors binding to particular ligands and having transmembrane and/or intracellular signaling domains similar to those present in a CAR.

In practicing the provided methods, the cells expressing a chimeric receptor can be co-administered with a peptide(s), such as administered prior to, simultaneously, subsequently or intermittently with the peptide. Generally, cells are administered after or subsequent to administration of the agent, e.g., cell or receptor. In some embodiments, the methods prevent, reduce, or down-regulate an immune response in the subject to the agent, e.g., cells or molecule expressed thereon, e.g., chimeric receptor expressed on the administered cells. In some embodiments, this improves exposure to the agent, such as by improving persistence or expansion of the cells.

### A. Genetically Engineered Chimeric Receptors

In some embodiments, the cells comprise one or more nucleic acids introduced via genetic engineering, and genetically engineered products of such nucleic acids. In some embodiments, the nucleic acids are heterologous, i.e., normally not present in a cell or sample obtained from the cell, such as one obtained from another organism or cell, which for example, is not ordinarily found in the cell being engineered and/or an organism from which such cell is derived. In some embodiments, the nucleic acids are not naturally occurring, such as a nucleic acid not found in nature, including one comprising chimeric combinations of nucleic acids encoding various domains from multiple different cell types. For example, the cells generally express recombinant receptors, such as antigen receptors including functional non-TCR antigen receptors, e.g., chimeric antigen receptors (CARs), and other chimeric receptors.

### 1. Cells

In some embodiments, the cells, e.g., engineered cells, are eukaryotic cells, such as mammalian cells, e.g., human cells. In some embodiments, the cells are derived from the blood, bone marrow, lymph, or lymphoid organs, are cells of the immune system, such as cells of the innate or adaptive immunity, e.g., myeloid or lymphoid cells, including lymphocytes, typically T cells and/or NK cells. Other exemplary cells include stem cells, such as multipotent and pluripotent stem cells, including induced pluripotent stem cells (iPSCs). In some aspects, the cells are human cells. The cells typically are primary cells, such as those isolated directly from a subject and/or isolated from a subject and frozen. In some embodiments, the cells include one or more subsets of T cells or other cell types, such as whole T cell populations, CD4+ cells, CD8+ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. Among the methods include off-the-shelf methods. In some aspects, such as for off-the-shelf technologies, the cells are pluripotent and/or multipotent, such as stem cells, such as induced pluripotent stem cells (iPSCs). In some embodiments, the methods include isolating cells from the subject, preparing, processing, culturing, and/or engineering them, as described herein, and re-introducing them into the same patient, before or after cryopreservation.

Among the sub-types and subpopulations of T cells and/or of CD4+ and/or of CD8+ T cells are naïve T (T_{N}) cells, effector T cells (T_{EFF}), memory T cells and sub-types thereof, such as stem cell memory T (T_{SCM}), central memory T (T_{CM}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells. In some embodiments, Tregs are used in the context of allogeneic transplant.

In some embodiments, one or more of the T cell populations is enriched for or depleted of cells that are positive for (marker⁺) or express high levels (marker^{high}) of one or more particular markers, such as surface markers, or that are negative for (marker ⁻) or express relatively low levels (marker^{low}) of one or more markers. In some cases, such markers are those that are absent or expressed at relatively low levels on certain populations of T cells (such as non-memory cells) but are present or expressed at relatively higher levels on certain other populations of T cells (such as memory cells). In one embodiment, the cells (such as the CD8⁺ cells or the T cells, e.g., CD3⁺ cells) are enriched for (i.e., positively selected for) cells that are positive or expressing high surface levels of CD45RO, CCR7, CD28, CD27, CD44, CD127, and/or CD62L and/or depleted of (e.g., negatively selected for) cells that are positive for or express high surface levels of CD45RA. In some embodiments, cells are enriched for or depleted of cells positive or expressing high surface levels of CD122, CD95, CD25, CD27, and/or IL7-Rα (CD127). In some examples, CD8+ T cells are enriched for cells positive for CD45RO (or negative for CD45RA) and for CD62L.

In some embodiments, a CD4+ T cell population and a CD8+ T cell sub-population, e.g., a sub-population enriched for central memory (T_{CM}) cells.

In some embodiments, the cells are natural killer (NK) cells. In some embodiments, the cells are monocytes or granulocytes, e.g., myeloid cells, macrophages, neutrophils, dendritic cells, mast cells, eosinophils, and/or basophils.

### 2. Chimeric Antigen Receptors (CARs)

In some embodiments, cells express a chimeric antigen receptor (CAR). The CAR is generally a genetically engineered receptor with an extracellular ligand binding domain, such as an extracelluar portion containing an antibody or fragment thereof, linked to one or more intracellular signaling components. In some embodiments, the chimeric antigen receptor includes a transmembrane domain and/or intracellular domain linking the extracellular domain and the intracellular signaling domain. Such molecules typically mimic or approximate a signal through a natural antigen receptor and/or signal through such a receptor in combination with a costimulatory receptor.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, , 8,339,645, 8,398,282, 7,446,179, 6,410,319, 7,070,995, 7,265,209, 7,354,762, 7,446,191, 8,324,353, and 8,479,118, and European patent application number EP2537416,and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some aspects, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, Kochenderfer et al, 2013, Nature Reviews Clinical Oncology, 10, 267-276 (2013); Wang et al. (2012) J. Immunother. 35(9): 689-701; and Brentjens et al., Sci Transl Med. 2013 5(177). *See also* WO2014031687, US 8,339,645, US 7,446,179, US 2013/0149337, U.S. Patent No.: 7,446,190, and US Patent No.: 8,389,282 and U.S. patent application Publication No. US 2013/0149337. Among the chimeric receptors are chimeric antigen receptors (CARs). The chimeric receptors, such as CARs, generally include an extracellular antigen binding domain, such as a portion of an antibody molecule, generally a variable heavy (V_{H}) chain region and/or variable light (V_{L}) chain region of the antibody, e.g., an scFv antibody fragment.

In some embodiments, CARs are constructed with a specificity for a particular antigen or marker, such as an antigen or marker expressed in a particular cell type to be targeted by adoptive therapy, e.g., a cancer marker and/or any of the antigens described. Thus, the CAR typically includes one or more ligand-binding domain. In some embodiments, the ligand binding domain is a TCR or a functional non-TCR. In some embodiments, the ligand-binding domain is an antigen-binding fragment, domain, or portion of an antibody, or one or more antibody variable domains, and/or antibody molecules. In some embodiments, the CAR includes an antigen-binding portion or portions of an antibody molecule, such as a variable heavy chain (VH) or antigen-binding portion thereof, or a single-chain antibody fragment (scFv) derived from the variable heavy (VH) and variable light (VL) chains of a monoclonal antibody (mAb). Exemplary of such ligand-binding domains are described below.

In some embodiments, the extracellular portion of the CAR, such as an antibody portion thereof, further includes a spacer, such as a spacer region between the antigen-recognition component, e.g. scFv, and a transmembrane domain. The spacer may be or include at least a portion of an immunoglobulin constant region or variant or modified version thereof, such as a hinge region, e.g., an IgG4 hinge region, and/or a CH1/CL and/or Fc region. In some embodiments, the constant region or portion is of a human IgG, such as IgG4 or IgG1. The spacer can be of a length that provides for increased responsiveness of the cell following antigen binding, as compared to in the absence of the spacer. In some examples, the spacer is at or about 12 amino acids in length or is no more than 12 amino acids in length. Exemplary spacers include those having at least about 10 to 229 amino acids, about 10 to 200 amino acids, about 10 to 175 amino acids, about 10 to 150 amino acids, about 10 to 125 amino acids, about 10 to 100 amino acids, about 10 to 75 amino acids, about 10 to 50 amino acids, about 10 to 40 amino acids, about 10 to 30 amino acids, about 10 to 20 amino acids, or about 10 to 15 amino acids, and including any integer between the endpoints of any of the listed ranges. In some embodiments, a spacer region has about 12 amino acids or less, about 119 amino acids or less, or about 229 amino acids or less. Exemplary spacers include IgG4 hinge alone, IgG4 hinge linked to CH2 and CH3 domains, or IgG4 hinge linked to the CH3 domain. Exemplary spacers include, but are not limited to, those described in Hudecek et al. (2013) Clin. Cancer Res., 19:3153. international patent application publication number WO2014031687, or U.S. Patent No. 8,822,647 or published app. No. US2014/0271635.

In some embodiments, the constant region or portion is of a human IgG, such as IgG4 or IgG1. In some embodiments, the spacer has the sequence ESKYGPPCPPCP (set forth in SEQ ID NO: 1), and is encoded by the sequence set forth in SEQ ID NO: 158. In some embodiments, the spacer has the sequence set forth in SEQ ID NO: 107. In some embodiments, the spacer has the sequence set forth in SEQ ID NO: 108. In some embodiments, the constant region or portion is of IgD. In some embodiments, the spacer has the sequence set forth in SEQ ID NO: 109. In some embodiments, the spacer has a sequence of amino acids that exhibits at least 85%, 86%, 87%. 88%, 89%, 90%, 91%, 92%, 93%, 94%, 95%, 96%, 97%, 98%, 99% or more sequence identity to any of SEQ ID NOS: 1, 107, 108 or 109.

The extracellular ligand binding, such as antigen recognition domain, generally is linked to one or more intracellular signaling components, such as signaling components that mimic activation through an antigen receptor complex, such as a TCR complex, in the case of a CAR, and/or signal via another cell surface receptor. In some embodiments, a transmembrane domain links the extracellular ligand binding and intracellular signaling domains. In some embodiments, the CAR includes a transmembrane domain fused to the extracellular domain. In one embodiment, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some embodiments is derived either from a natural or from a synthetic source. Where the source is natural, the domain in some aspects is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD 16, CD22, CD33, CD37, CD64, CD80, CD86, CD 134, CD137, CD 154 and/or transmembrane regions containing functional variants thereof such as those retaining a substantial portion of the structural, e.g., transmembrane, properties thereof. In some embodiments, the transmembrane domain is a transmembrane domain derived from CD4, CD28, or CD8, e.g., CD8alpha, or functional variant thereof. Alternatively the transmembrane domain in some embodiments is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain. In some embodiments, the linkage is by linkers, spacers, and/or transmembrane domain(s).

Among the intracellular signaling domains are those that mimic or approximate a signal through a natural antigen receptor, a signal through such a receptor in combination with a costimulatory receptor, and/or a signal through a costimulatory receptor alone. In some embodiments, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

The receptor, e.g., the CAR, generally includes at least one intracellular signaling component or components. In some embodiments, the receptor includes an intracellular component of a TCR complex, such as a TCR CD3 chain that mediates T-cell activation and cytotoxicity, e.g., CD3 zeta chain. Thus, in some aspects, the antigen-binding portion is linked to one or more cell signaling modules. In some embodiments, cell signaling modules include CD3 transmembrane domain, CD3 intracellular signaling domains, and/or other CD transmembrane domains. In some embodiments, the receptor, e.g., CAR, further includes a portion of one or more additional molecules such as Fc receptor γ, CD8, CD4, CD25, or CD16. For example, in some aspects, the CAR or other chimeric receptor includes a chimeric molecule between CD3-zeta (CD3-ζ) or Fc receptor γ and CD8, CD4, CD25 or CD16.

In some embodiments, upon ligation of the CAR or other chimeric receptor, the cytoplasmic domain or intracellular signaling domain of the receptor activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some embodiments, a truncated portion of an intracellular signaling domain of an antigen receptor component or costimulatory molecule is used in place of an intact immunostimulatory chain, for example, if it transduces the effector function signal. In some embodiments, the intracellular signaling domain or domains include the cytoplasmic sequences of the T cell receptor (TCR), and in some aspects also those of co-receptors that in the natural context act in concert with such receptors to initiate signal transduction following antigen receptor engagement.

In the context of a natural TCR, full activation generally requires not only signaling through the TCR, but also a costimulatory signal. Thus, in some embodiments, to promote full activation, a component for generating secondary or co-stimulatory signal is also included in the CAR. In other embodiments, the CAR does not include a component for generating a costimulatory signal. In some aspects, an additional CAR is expressed in the same cell and provides the component for generating the secondary or costimulatory signal.

T cell activation is in some aspects described as being mediated by two classes of cytoplasmic signaling sequences: those that initiate antigen-dependent primary activation through the TCR (primary cytoplasmic signaling sequences), and those that act in an antigen-independent manner to provide a secondary or co-stimulatory signal (secondary cytoplasmic signaling sequences). In some aspects, the CAR includes one or both of such signaling components.

In some aspects, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from CD3 zeta chain, FcR gamma, CD3 gamma, CD3 delta andCD3 epsilon. In some embodiments, cytoplasmic signaling molecule(s) in the CAR contain(s) a cytoplasmic signaling domain, portion thereof, or sequence derived from CD3 zeta.

In some embodiments, the CAR includes a signaling domain and/or transmembrane portion of a costimulatory receptor, such as CD28, 4-1BB, OX40, DAP10, and ICOS. In some aspects, the same CAR includes both the activating and costimulatory components.

In some embodiments, the activating domain is included within one CAR, whereas the costimulatory component is provided by another CAR recognizing another antigen. In some embodiments, the CARs include activating or stimulatory CARs, costimulatory CARs, both expressed on the same cell (see WO2014/055668). In some aspects, the cells include one or more stimulatory or activating CAR and/or a costimulatory CAR. In some embodiments, the cells further include inhibitory CARs (iCARs, see Fedorov et al., Sci. Transl. Medicine, 5(215) (December, 2013), such as a CAR recognizing an antigen other than the one associated with and/or specific for the disease or condition whereby an activating signal delivered through the disease-targeting CAR is diminished or inhibited by binding of the inhibitory CAR to its ligand, e.g., to reduce off-target effects.

In some embodiments, the intracellular signaling component of the recombinant receptor, such as CAR, comprises a CD3 zeta intracellular domain and a costimulatory signaling region. In certain embodiments, the intracellular signaling domain comprises a CD28 transmembrane and signaling domain linked to a CD3 (e.g., CD3-zeta) intracellular domain. In some embodiments, the intracellular signaling domain comprises a chimeric CD28 and CD137 (4-1BB, TNFRSF9) co-stimulatory domains, linked to a CD3 zeta intracellular domain.

In some embodiments, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some embodiments, the CAR or other antigen receptor further includes a marker, such as a cell surface marker, which may be used to confirm transduction or engineering of the cell to express the receptor, such as a truncated version of a cell surface receptor, such as truncated EGFR (tEGFR). In some aspects, the marker includes all or part (e.g., truncated form) of CD34, a NGFR, or epidermal growth factor receptor (e.g., tEGFR). In some embodiments, the nucleic acid encoding the marker is operably linked to a polynucleotide encoding for a linker sequence, such as a cleavable linker sequence, e.g., T2A. For example, a marker, and optionally a linker sequence, can be any as disclosed in published patent application No. WO2014031687. For example, the marker can be a truncated EGFR (tEGFR) that is, optionally, linked to a linker sequence, such as a T2A cleavable linker sequence. An exemplary polypeptide for a truncated EGFR (e.g. tEGFR) comprises the sequence of amino acids set forth in SEQ ID NO: 111 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 111. An exemplary T2A linker sequence comprises the sequence of amino acids set forth in SEQ ID NO: 110 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 110.

In some embodiments, the marker is a molecule, e.g., cell surface protein, not naturally found on T cells or not naturally found on the surface of T cells, or a portion thereof.

In some embodiments, the molecule is a non-self molecule, e.g., non-self protein, i.e., one that is not recognized as "self" by the immune system of the host into which the cells will be adoptively transferred.

In some embodiments, the marker serves no therapeutic function and/or produces no effect other than to be used as a marker for genetic engineering, e.g., for selecting cells successfully engineered. In other embodiments, the marker may be a therapeutic molecule or molecule otherwise exerting some desired effect, such as a ligand for a cell to be encountered in vivo, such as a costimulatory or immune checkpoint molecule to enhance and/or dampen responses of the cells upon adoptive transfer and encounter with ligand.

In some cases, CARs are referred to as first, second, and/or third generation CARs. In some aspects, a first generation CAR is one that solely provides a CD3-chain induced signal upon antigen binding; in some aspects, a second-generation CARs is one that provides such a signal and costimulatory signal, such as one including an intracellular signaling domain from a costimulatory receptor such as CD28 or CD137; in some aspects, a third generation CAR is one that includes multiple costimulatory domains of different costimulatory receptors.

### Ligand Binding Domain

In some embodiments, the chimeric receptor is constructed with a specificity for a particular antigen (or marker or ligand), such as an antigen expressed in a particular cell type to be targeted by adoptive therapy, *e.g.,* a cancer marker, and/or an antigen intended to induce a dampening response, such as an antigen expressed on a normal or non-diseased cell type. Thus, the chimeric receptor typically includes in its extracellular potion on or more ligand binding domains.

In some embodiments, the chimeric receptor is a CAR. In some embodiments, the CAR typically includes in its extracellular portion one or more antigen binding molecules, such as one or more antigen-binding fragment, domain, or portion, or one or more antibody variable domains, and/or antibody molecules. In some embodiments, the CAR includes an antigen-binding portion or portions of an antibody molecule, such as a single-chain antibody fragment (scFv) derived from the variable heavy (VH) and variable light (VL) chains of a monoclonal antibody (mAb).

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, variable heavy chain (V_{H}) regions capable of specifically binding the antigen, single chain antibody fragments, including single chain variable fragments (scFv), and single domain antibodies (*e.g*., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, e.g., bispecific, antibodies, diabodies, triabodies, and tetrabodies, tandem di-scFv, tandem tri-scFv. Unless otherwise stated, the term "antibody" should be understood to encompass functional antibody fragments thereof. The term also encompasses intact or full-length antibodies, including antibodies of any class or sub-class, including IgG and sub-classes thereof, IgM, IgE, IgA, and IgD.

In some embodiments, the antigen-binding proteins, antibodies and antigen binding fragments thereof specifically recognize an antigen of a full-length antibody. In some embodiments, the heavy and light chains of an antibody can be full-length or can be an antigen-binding portion (a Fab, F(ab')2, Fv or a single chain Fv fragment (scFv)). In other embodiments, the antibody heavy chain constant region is chosen from, *e.g.,* IgG1, IgG2, IgG3, IgG4, IgM. IgA1, IgA2, IgD, and IgE, particularly chosen from, *e.g..* IgG1, IgG2, IgG3, and IgG4, more particularly, IgG1 (*e.g*., human IgG1). In another embodiment, the antibody light chain constant region is chosen from, *e.g.,* kappa or lambda, particularly kappa.

Among the provided antibodies are antibody fragments. An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; variable heavy chain (V_{H}) regions, single-chain antibody molecules such as scFvs and single-domain V_{H} single antibodies; and multispecific antibodies formed from antibody fragments. In particular embodiments, the antibodies are single-chain antibody fragments comprising a variable heavy chain region and/or a variable light chain region, such as scFvs.

The term "variable region" or "variable domain" refers to the domain of an antibody heavy or light chain that is involved in binding the antibody to antigen. The variable domains of the heavy chain and light chain (V_{H} and V_{L}, respectively) of a native antibody generally have similar structures, with each domain comprising four conserved framework regions (FRs) and three CDRs. (See, *e.g.,* Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, *e.g.,* Portolano et al., J. Immunol. 150:880-887 (1993); Clarkson et al. Nature 352:624-628 (1991).

Single-domain antibodies are antibody fragments comprising all or a portion of the heavy chain variable domain or all or a portion of the light chain variable domain of an antibody. In certain embodiments, a single-domain antibody is a human single-domain antibody. In some embodiments, the CAR comprises an antibody heavy chain domain that specifically binds the antigen, such as a cancer marker or cell surface antigen of a cell or disease to be targeted, such as a tumor cell or a cancer cell, such as any of the target antigens described herein or known in the art.

Antibody fragments can be made by various techniques, including but not limited to proteolytic digestion of an intact antibody as well as production by recombinant host cells. In some embodiments, the antibodies are recombinantly-produced fragments, such as fragments comprising arrangements that do not occur naturally, such as those with two or more antibody regions or chains joined by synthetic linkers, e.g., peptide linkers, and/or that are may not be produced by enzyme digestion of a naturally-occurring intact antibody. In some embodiments, the antibody fragments are scFvs.

A "humanized" antibody is an antibody in which all or substantially all CDR amino acid residues are derived from non-human CDRs and all or substantially all FR amino acid residues are derived from human FRs. A humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some embodiments, some FR residues in a humanized antibody are substituted with corresponding residues from a non-human antibody (*e.g*., the antibody from which the CDR residues are derived), *e.g.,* to restore or improve antibody specificity or affinity.

In some embodiments, the CAR contains an antibody or an antigen-binding fragment (*e.g*. scFv) that specifically recognizes an antigen, such as an intact antigen, expressed on the surface of a cell.

In some embodiments, the CAR contains a TCR-like antibody, such as an antibody or an antigen-binding fragment (*e.g*. scFv) that specifically recognizes an intracellular antigen, such as a tumor-associated antigen, presented on the cell surface as a MHC-peptide complex. In some embodiments, an antibody or antigen-binding portion thereof that recognizes an MHC-peptide complex can be expressed on cells as part of a recombinant receptor, such as an antigen receptor. Among the antigen receptors are functional non-TCR antigen receptors, such as chimeric antigen receptors (CARs). Generally, a CAR containing an antibody or antigen-binding fragment that exhibits TCR-like specificity directed against peptide-MHC complexes also may be referred to as a TCR-like CAR.

In some embodiments, an antibody or antigen-binding portion thereof that specifically binds to a MHC-peptide complex, can be produced by immunizing a host with an effective amount of an immunogen containing a specific MHC-peptide complex. In some cases, the peptide of the MHC-peptide complex is an epitope of antigen capable of binding to the MHC, such as a tumor antigen, for example a universal tumor antigen, myeloma antigen or other antigen as described below. In some embodiments, an effective amount of the immunogen is then administered to a host for eliciting an immune response, wherein the immunogen retains a three-dimensional form thereof for a period of time sufficient to elicit an immune response against the three-dimensional presentation of the peptide in the binding groove of the MHC molecule. Serum collected from the host is then assayed to determine if desired antibodies that recognize a three-dimensional presentation of the peptide in the binding groove of the MHC molecule is being produced. In some embodiments, the produced antibodies can be assessed to confirm that the antibody can differentiate the MHC-peptide complex from the MHC molecule alone, the peptide of interest alone, and a complex of MHC and irrelevant peptide. The desired antibodies can then be isolated.

In some embodiments, an antibody or antigen-binding portion thereof that specifically binds to an MHC-peptide complex can be produced by employing antibody library display methods, such as phage antibody libraries. In some embodiments, phage display libraries of mutant Fab, scFV or other antibody forms can be generated, for example, in which members of the library are mutated at one or more residues of a CDR or CDRs. Exemplary of such methods are known in the art (see *e.g.* US published application No. US20020150914, US2014/0294841; and Cohen CJ. et al. (2003) J Mol. Recogn. 16:324-332).

### Exemplary Chimeric Receptors

In some embodiments, the chimeric antigen receptor includes an extracellular portion containing an antibody or antibody fragment. In some aspects, the chimeric antigen receptor includes an extracellular portion containing the antibody or fragment and an intracellular signaling domain. In some embodiments, the antibody or fragment includes an scFv and the intracellular domain contains an ITAM. In some aspects, the intracellular signaling domain includes a signaling domain of a zeta chain of a CD3-zeta (CD3ζ) chain. In some embodiments, the chimeric antigen receptor includes a transmembrane domain linking the extracellular domain and the intracellular signaling domain. In some aspects, the transmembrane domain contains a transmembrane portion of CD28. In some embodiments, the transmembrane domain of the receptor, e.g., the CAR is a transmembrane domain of human CD28 or variant thereof, e.g., a 27-amino acid transmembrane domain of a human CD28 (Accession No.: P10747.1). In some embodiments, the chimeric antigen receptor contains an intracellular domain of a T cell costimulatory molecule. The extracellular domain and transmembrane domain can be linked directly or indirectly. In some embodiments, the extracellular domain and transmembrane are linked by a spacer, such as any described herein. In some embodiments, the receptor contains extracellular portion of the molecule from which the transmembrane domain is derived, such as a CD28 extracellular portion. In some embodiments, the chimeric antigen receptor contains an intracellular domain derived from a T cell costimulatory molecule or a functional variant thereof, such as between the transmembrane domain and intracellular signaling domain. In some aspects, the T cell costimulatory molecule is CD28 or 41BB.

For example, in some embodiments, the CAR contains an antibody, e.g., an antibody fragment, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some embodiments, the CAR contains an antibody, e.g., antibody fragment, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such embodiments, the receptor further includes a spacer containing a portion of an Ig molecule, such as a human Ig molecule, such as an Ig hinge, e.g. an IgG4 hinge, such as a hinge-only spacer.

In some embodiments, the transmembrane domain of the recombinant receptor, e.g., the CAR, is or includes a transmembrane domain of human CD28 (e.g. Accession No. P01747.1) or variant thereof, such as a transmembrane domain that comprises the sequence of amino acids set forth in SEQ ID NO: 2 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 2; in some embodiments, the transmembrane-domain containing portion of the recombinant receptor comprises the sequence of amino acids set forth in SEQ ID NO: 104 or a sequence of amino acids having at least at or about 85%, 86%, 87%, 88%, 89%, 90%, 91%, 92%, 93%, 94%, 95%, 96%, 97%, 98%, 99% or more sequence identity thereto.

In some embodiments, the chimeric antigen receptor contains an intracellular domain of a T cell costimulatory molecule. In some aspects, the T cell costimulatory molecule is CD28 or 41BB.

In some embodiments, the intracellular signaling component(s) of the recombinant receptor, e.g. the CAR, contains an intracellular costimulatory signaling domain of human CD28 or a functional variant or portion thereof, such as a domain with an LL to GG substitution at positions 186-187 of a native CD28 protein. For example, the intracellular signaling domain can comprise the sequence of amino acids set forth in SEQ ID NO: 112 or 113 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 112 or 113. In some embodiments, the intracellular domain comprises an intracellular costimulatory signaling domain of 4-1BB (e.g. (Accession No. Q07011.1) or functional variant or portion thereof, such as the sequence of amino acids set forth in SEQ ID NO: 3 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 3.

In some embodiments, the intracellular signaling domain of the recombinant receptor, e.g. the CAR, comprises a human CD3 zeta stimulatory signaling domain or functional variant thereof, such as an 112 AA cytoplasmic domain of isoform 3 of human CD3ζ (Accession No.: P20963.2) or a CD3 zeta signaling domain as described in U.S. Patent No.: 7,446,190 or U.S. Patent No. 8,911,993. For example, in some embodiments, the intracellular signaling domain comprises the sequence of amino acids as set forth in SEQ ID NO: 4, 105 or 159 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 4, 105 or 159.

In some aspects, the spacer contains only a hinge region of an IgG, such as only a hinge of IgG4 or IgG1, such as the hinge only spacer set forth in SEQ ID NO: 1. In other embodiments, the spacer is or contains an Ig hinge, e.g., an IgG4-derived hinge, optionally linked to a CH2 and/or CH3 domains. In some embodiments, the spacer is an Ig hinge, e.g., an IgG4 hinge, linked to CH2 and CH3 domains, such as set forth in SEQ ID NO: 108. In some embodiments, the spacer is an Ig hinge, e.g., an IgG4 hinge, linked to a CH3 domain only, such as set forth in SEQ ID NO: 107. In some embodiments, the spacer is or comprises a glycine-serine rich sequence or other flexible linker such as known flexible linkers.

For example, in some embodiments, the CAR includes an antibody such as an antibody fragment, including scFvs, a spacer, such as a spacer containing a portion of an immunoglobulin molecule, such as a hinge region and/or one or more constant regions of a heavy chain molecule, such as an Ig-hinge containing spacer, a transmembrane domain containing all or a portion of a CD28-derived transmembrane domain, a CD28-derived intracellular signaling domain, and a CD3 zeta signaling domain. In some embodiments, the CAR includes: an extracellular ligand-binding portion, such as an antigen-binding portion, such as an antibody or fragment thereof, including sdAbs and scFvs, that specifically binds an antigen, e.g. an antigen described herein; a spacer such as any of the Ig-hinge containing spacers; a transmembrane domain that is a portion of CD28 or a variant thereof; an intracellular signaling domain containing a signaling portion of 4-1BB or functional variant thereof; and a signaling portion of CD3 zeta signaling domain or functional variant thereof.

In some embodiments, nucleic acid molecules encoding such CAR constructs further includes a sequence encoding a T2A ribosomal skip element and/or a tEGFR sequence, e.g., downstream of the sequence encoding the CAR. In some embodiments, the sequence encodes a T2A ribosomal skip element and/or a tEGFR sequence set forth in SEQ ID NO: 110 and/or 111, respectively, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 110 and/or 111. In some embodiments, T cells expressing an antigen receptor (e.g. CAR) can also be generated to express a truncated EGFR (EGFRt) as a non-immunogenic selection epitope (e.g. by introduction of a construct encoding the CAR and EGFRt separated by a T2A ribosome switch to express two proteins from the same construct), which then can be used as a marker to detect such cells (see e.g. U.S. Patent No. 8,802,374).

In some embodiments, the CAR includes a junction region that is formed by the joining of a transmembrane portion of CD28 and a second costimulatory portion of 41BB. In some embodiments, the CAR comprises the amino acid sequence CYSLLVTVAFIIFWVKRGRKKLLYIFKQPF (SEQ ID NO: 6), the amino acid sequence SLLVTVAFIIFWVKRGRKKLLYIFKQ (SEQ ID NO:137) or the amino acid sequence CYSLLVTVAFIIFWVKRGRKKLLYIFKQPFMRPVQT (SEQ ID NO: 160).

### 3. Vectors and methods for genetic engineering

Also provided are methods, nucleic acids, compositions, and kits, for producing the genetically engineered cells. In some aspects, the genetic engineering involves introduction of a nucleic acid encoding the genetically engineered component or other component for introduction into the cell, such as a component encoding a gene-disruption protein or nucleic acid.

In some embodiments, gene transfer is accomplished by first stimulating cell growth, e.g., T cell growth, proliferation, and/or activation, followed by transduction of the activated cells, and expansion in culture to numbers sufficient for clinical applications.

In some contexts, overexpression of a stimulatory factor (for example, a lymphokine or a cytokine) may be toxic to a subject. Thus, in some contexts, the engineered cells include gene segments that cause the cells to be susceptible to negative selection in vivo, such as upon administration in adoptive immunotherapy. For example in some aspects, the cells are engineered so that they can be eliminated as a result of a change in the in vivo condition of the patient to which they are administered. The negative selectable phenotype may result from the insertion of a gene that confers sensitivity to an administered agent, for example, a compound. Negative selectable genes include the Herpes simplex virus type I thymidine kinase (HSV-ITK) gene (Wigler et al., Cell II :223, 1977) which confers ganciclovir sensitivity; the cellular hypoxanthine phosphribosyltransferase (HPRT)gene, the cellular adenine phosphoribosyltransferase (APRT) gene, bacterial cytosine deaminase, (Mullen et al., Proc. Natl. Acad. Sci. USA. 89:33 (1992)).

In some aspects, the cells further are engineered to promote expression of cytokines or other factors. Various methods for the introduction of genetically engineered components, e.g., antigen receptors, e.g., CARs, are well known and may be used with the provided methods and compositions. Exemplary methods include those for transfer of nucleic acids encoding the receptors, including via viral, e.g., retroviral or lentiviral, transduction, transposons, and electroporation.

In some embodiments, recombinant nucleic acids are transferred into cells using recombinant infectious virus particles, such as, e.g., vectors derived from simian virus 40 (SV40), adenoviruses, adeno-associated virus (AAV). In some embodiments, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November; 29(11): 550-557.

In some embodiments, the retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV), spleen focus forming virus (SFFV), or adeno-associated virus (AAV). Most retroviral vectors are derived from murine retroviruses. In some embodiments, the retroviruses include those derived from any avian or mammalian cell source. The retroviruses typically are amphotropic, meaning that they are capable of infecting host cells of several species, including humans. In one embodiment, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

In some embodiments, recombinant nucleic acids are transferred into T cells via electroporation (see, e.g., Chicaybam et al, (2013) PLoS ONE 8(3): e60298 and Van Tedeloo et al. (2000) Gene Therapy 7(16): 1431-1437). In some embodiments, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA coprecipitation (Brash et al., Mol. Cell Biol., 7: 2031-2034 (1987)).

Other approaches and vectors for transfer of the genetically engineered nucleic acids encoding the genetically engineered products are those described, e.g., in international patent application, Publication No.: WO2014055668, and U.S. Patent No. 7,446,190.

Among additional nucleic acids, e.g., genes for introduction are those to improve the efficacy of therapy, such as by promoting viability and/or function of transferred cells; genes to provide a genetic marker for selection and/or evaluation of the cells, such as to assess in vivo survival or localization; genes to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also the publications of PCT/US91/08442 and PCT/US94/05601 by Lupton et al. describing the use of bifunctional selectable fusion genes derived from fusing a dominant positive selectable marker with a negative selectable marker. See, e.g., Riddell et al., US Patent No. 6,040,177, at columns 14-17.

### 4. Preparation of cells for engineering

In some embodiments, preparation of the engineered cells includes one or more culture and/or preparation steps. The cells for introduction of the nucleic acid encoding the chimeric receptor such as the CAR, may be isolated from a sample, such as a biological sample, e.g., one obtained from or derived from a subject. In some embodiments, the subject from which the cell is isolated is one having the disease or condition or in need of a cell therapy or to which cell therapy will be administered. The subject in some embodiments is a human in need of a particular therapeutic intervention, such as the adoptive cell therapy for which cells are being isolated, processed, and/or engineered.

Accordingly, the cells in some embodiments are primary cells, e.g., primary human cells. The samples include tissue, fluid, and other samples taken directly from the subject, as well as samples resulting from one or more processing steps, such as separation, centrifugation, genetic engineering (e.g. transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some aspects, the sample from which the cells are derived or isolated is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some embodiments, the cells are derived from cell lines, e.g., T cell lines. The cells in some embodiments are obtained from a xenogeneic source, for example, from mouse, rat, non-human primate, and pig.

In some embodiments, isolation of the cells includes one or more preparation and/or non-affinity based cell separation steps. In some examples, cells are washed, centrifuged, and/or incubated in the presence of one or more reagents, for example, to remove unwanted components, enrich for desired components, lyse or remove cells sensitive to particular reagents. In some examples, cells are separated based on one or more property, such as density, adherent properties, size, sensitivity and/or resistance to particular components.

In some examples, cells from the circulating blood of a subject are obtained, e.g., by apheresis or leukapheresis. The samples, in some aspects, contain lymphocytes, including T cells, monocytes, granulocytes, B cells, other nucleated white blood cells, red blood cells, and/or platelets, and in some aspects contains cells other than red blood cells and platelets.

In some embodiments, the blood cells collected from the subject are washed, e.g., to remove the plasma fraction and to place the cells in an appropriate buffer or media for subsequent processing steps. In some embodiments, the cells are washed with phosphate buffered saline (PBS). In some embodiments, the wash solution lacks calcium and/or magnesium and/or many or all divalent cations. In some aspects, a washing step is accomplished a semi-automated "flow-through" centrifuge (for example, the Cobe 2991 cell processor, Baxter) according to the manufacturer's instructions. In some aspects, a washing step is accomplished by tangential flow filtration (TFF) according to the manufacturer's instructions. In some embodiments, the cells are resuspended in a variety of biocompatible buffers after washing, such as, for example, Ca++/Mg++ free PBS. In certain embodiments, components of a blood cell sample are removed and the cells directly resuspended in culture media.

In some embodiments, the methods include density-based cell separation methods, such as the preparation of white blood cells from peripheral blood by lysing the red blood cells and centrifugation through a Percoll or Ficoll gradient.

In some embodiments, the isolation methods include the separation of different cell types based on the expression or presence in the cell of one or more specific molecules, such as surface markers, e.g., surface proteins, intracellular markers, or nucleic acid. In some embodiments, any known method for separation based on such markers may be used. In some embodiments, the separation is affinity- or immunoaffinity-based separation. For example, the isolation in some aspects includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some examples, both fractions are retained for further use. In some aspects, negative selection can be particularly useful where no antibody is available that specifically identifies a cell type in a heterogeneous population, such that separation is best carried out based on markers expressed by cells other than the desired population.

The separation need not result in 100% enrichment or removal. of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some examples, multiple rounds of separation steps are carried out, where the positively or negatively selected fraction from one step is subjected to another separation step, such as a subsequent positive or negative selection. In some examples, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some aspects, specific subpopulations of T cells, such as cells positive or expressing high levels of one or more surface markers, e.g., CD28+, CD62L+, CCR7+, CD27+, CD127+, CD4+, CD8+, CD45RA+, and/or CD45RO+ T cells, are isolated by positive or negative selection techniques.

For example, CD3+, CD28+ T cells can be positively selected using CD3/CD28 conjugated magnetic beads (e.g., DYNABEADS^{®} M-450 CD3/CD28 T Cell Expander).

In some embodiments, isolation is carried out by enrichment for a particular cell population by positive selection, or depletion of a particular cell population, by negative selection. In some embodiments, positive or negative selection is accomplished by incubating cells with one or more antibodies or other binding agent that specifically bind to one or more surface markers expressed or expressed (marker+) at a relatively higher level (markerhigh) on the positively or negatively selected cells, respectively.

In some embodiments, T cells are separated from a PBMC sample by negative selection of markers expressed on non-T cells, such as B cells, monocytes, or other white blood cells, such as CD14. In some aspects, a CD4+ or CD8+ selection step is used to separate CD4+ helper and CD8+ cytotoxic T cells. Such CD4+ and CD8+ populations can be further sorted into sub-populations by positive or negative selection for markers expressed or expressed to a relatively higher degree on one or more naive, memory, and/or effector T cell subpopulations.

In some embodiments, CD8+ cells are further enriched for or depleted of naive, central memory, effector memory, and/or central memory stem cells, such as by positive or negative selection based on surface antigens associated with the respective subpopulation. In some embodiments, enrichment for central memory T (TCM) cells is carried out to increase efficacy, such as to improve long-term survival, expansion, and/or engraftment following administration, which in some aspects is particularly robust in such sub-populations. See Terakura et al. (2012) Blood.1:72-82; Wang et al. (2012) J Immunother. 35(9):689-701. In some embodiments, combining TCM-enriched CD8+ T cells and CD4+ T cells further enhances efficacy.

In embodiments, memory T cells are present in both CD62L+ and CD62L-subsets of CD8+ peripheral blood lymphocytes. PBMC can be enriched for or depleted of CD62L-CD8+ and/or CD62L+CD8+ fractions, such as using anti-CD8 and anti-CD62L antibodies.

In some embodiments, the enrichment for central memory T (TCM) cells is based on positive or high surface expression of CD45RO, CD62L, CCR7, CD28, CD3, and/or CD 127; in some aspects, it is based on negative selection for cells expressing or highly expressing CD45RA and/or granzyme B. In some aspects, isolation of a CD8+ population enriched for TCM cells is carried out by depletion of cells expressing CD4, CD14, CD45RA, and positive selection or enrichment for cells expressing CD62L. In one aspect, enrichment for central memory T (TCM) cells is carried out starting with a negative fraction of cells selected based on CD4 expression, which is subjected to a negative selection based on expression of CD14 and CD45RA, and a positive selection based on CD62L. Such selections in some aspects are carried out simultaneously and in other aspects are carried out sequentially, in either order. In some aspects, the same CD4 expression-based selection step used in preparing the CD8+ cell population or subpopulation, also is used to generate the CD4+ cell population or sub-population, such that both the positive and negative fractions from the CD4-based separation are retained and used in subsequent steps of the methods, optionally following one or more further positive or negative selection steps.

In a particular example, a sample of PBMCs or other white blood cell sample is subjected to selection of CD4+ cells, where both the negative and positive fractions are retained. The negative fraction then is subjected to negative selection based on expression of CD14 and CD45RA or CD19, and positive selection based on a marker characteristic of central memory T cells, such as CD62L or CCR7, where the positive and negative selections are carried out in either order.

CD4+ T helper cells are sorted into naïve, central memory, and effector cells by identifying cell populations that have cell surface antigens. CD4+ lymphocytes can be obtained by standard methods. In some embodiments, naive CD4+ T lymphocytes are CD45RO-, CD45RA+, CD62L+, CD4+ T cells. In some embodiments, central memory CD4+ cells are CD62L+ and CD45RO+. In some embodiments, effector CD4+ cells are CD62L- and CD45RO.

In one example, to enrich for CD4+ cells by negative selection, a monoclonal antibody cocktail typically includes antibodies to CD14, CD20, CD11b, CD16, HLA-DR, and CD8. In some embodiments, the antibody or binding partner is bound to a solid support or matrix, such as a magnetic bead or paramagnetic bead, to allow for separation of cells for positive and/or negative selection. For example, in some embodiments, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In Vitro and In Vivo, p 17-25 Edited by: S. A. Brooks and U. Schumacher © Humana Press Inc., Totowa, NJ).

In some aspects, the sample or composition of cells to be separated is incubated with small, magnetizable or magnetically responsive material, such as magnetically responsive particles or microparticles, such as paramagnetic beads (e.g., such as Dynalbeads or MACS beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some embodiments, the magnetic particle or bead comprises a magnetically responsive material bound to a specific binding member, such as an antibody or other binding partner. There are many well-known magnetically responsive materials used in magnetic separation methods. Suitable magnetic particles include those described in Molday, U.S. Pat. No. 4,452,773, and in European Patent Specification EP 452342 B, which are hereby incorporated by reference. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some aspects, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some aspects, a combination of positive and negative selection is performed during the same selection step, where the positive and negative fractions are retained and further processed or subject to further separation steps.

In certain embodiments, the magnetically responsive particles are coated in primary antibodies or other binding partners, secondary antibodies, lectins, enzymes, or streptavidin. In certain embodiments, the magnetic particles are attached to cells via a coating of primary antibodies specific for one or more markers. In certain embodiments, the cells, rather than the beads, are labeled with a primary antibody or binding partner, and then cell-type specific secondary antibody- or other binding partner (e.g., streptavidin)-coated magnetic particles, are added. In certain embodiments, streptavidin-coated magnetic particles are used in conjunction with biotinylated primary or secondary antibodies.

In some embodiments, the magnetically responsive particles are left attached to the cells that are to be subsequently incubated, cultured and/or engineered; in some aspects, the particles are left attached to the cells for administration to a patient. In some embodiments, the magnetizable or magnetically responsive particles are removed from the cells. Methods for removing magnetizable particles from cells are known and include, e.g., the use of competing non-labeled antibodies, and magnetizable particles or antibodies conjugated to cleavable linkers. In some embodiments, the magnetizable particles are biodegradable.

In some embodiments, the affinity-based selection is via magnetic-activated cell sorting (MACS) (Miltenyi Biotec, Auburn, CA). Magnetic Activated Cell Sorting (MACS) systems are capable of high-purity selection of cells having magnetized particles attached thereto. In certain embodiments, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain embodiments, the non-target cells are labelled and depleted from the heterogeneous population of cells.

In certain embodiments, the isolation or separation is carried out using a system, device, or apparatus that carries out one or more of the isolation, cell preparation, separation, processing, incubation, culture, and/or formulation steps of the methods. In some aspects, the system is used to carry out each of these steps in a closed or sterile environment, for example, to minimize error, user handling and/or contamination. In one example, the system is a system as described in International Patent Application, Publication Number WO2009/072003, or US 20110003380 A1.

In some embodiments, the system or apparatus carries out one or more, e.g., all, of the isolation, processing, engineering, and formulation steps in an integrated or self-contained system, and/or in an automated or programmable fashion. In some aspects, the system or apparatus includes a computer and/or computer program in communication with the system or apparatus, which allows a user to program, control, assess the outcome of, and/or adjust various aspects of the processing, isolation, engineering, and formulation steps.

In some aspects, the separation and/or other steps is carried out using CliniMACS system (Miltenyi Biotec), for example, for automated separation of cells on a clinical-scale level in a closed and sterile system. Components can include an integrated microcomputer, magnetic separation unit, peristaltic pump, and various pinch valves. The integrated computer in some aspects controls all components of the instrument and directs the system to perform repeated procedures in a standardized sequence. The magnetic separation unit in some aspects includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some aspects uses antibody-coupled magnetizable particles that are supplied in a sterile, non-pyrogenic solution. In some embodiments, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some embodiments, the cell populations for use with the methods described herein are unlabeled and are not retained in the column. In some embodiments, the cell populations for use with the methods described herein are labeled and are retained in the column. In some embodiments, the cell populations for use with the methods described herein are eluted from the column after removal of the magnetic field, and are collected within the cell collection bag.

In certain embodiments, separation and/or other steps are carried out using the CliniMACS Prodigy system (Miltenyi Biotec). The CliniMACS Prodigy system in some aspects is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood is automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. (2012) J Immunother. 35(9): 651-660, Terakura et al. (2012) Blood. 1:72-82, and Wang et al. (2012) J Immunother. 35(9):689-701.

In some embodiments, a cell population described herein is collected and enriched (or depleted) via flow cytometry, in which cells stained for multiple cell surface markers are carried in a fluidic stream. In some embodiments, a cell population described herein is collected and enriched (or depleted) via preparative scale (FACS)-sorting. In certain embodiments, a cell population described herein is collected and enriched (or depleted) by use of microelectromechanical systems (MEMS) chips in combination with a FACS-based detection system (see, e.g., WO 2010/033140, Cho et al. (2010) Lab Chip 10, 1567-1573; and Godin et al. (2008) J Biophoton. 1(5):355-376. In both cases, cells can be labeled with multiple markers, allowing for the isolation of well-defined T cell subsets at high purity.

In some embodiments, the antibodies or binding partners are labeled with one or more detectable marker, to facilitate separation for positive and/or negative selection. For example, separation may be based on binding to fluorescently labeled antibodies. In some examples, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flow-cytometric detection system. Such methods allow for positive and negative selection based on multiple markers simultaneously.

In some embodiments, the preparation methods include steps for freezing, e.g., cryopreserving, the cells, either before or after isolation, incubation, and/or engineering. In some embodiments, the freeze and subsequent thaw step removes granulocytes and, to some extent, monocytes in the cell population. In some embodiments, the cells are suspended in a freezing solution, e.g., following a washing step to remove plasma and platelets. Any of a variety of known freezing solutions and parameters in some aspects may be used. One example involves using PBS containing 20% DMSO and 8% human serum albumin (HSA), or other suitable cell freezing media. This is then diluted 1:1 with media so that the final concentration of DMSO and HSA are 10% and 4%, respectively. The cells are generally then frozen to -80° C. at a rate of 1° per minute and stored in the vapor phase of a liquid nitrogen storage tank.

In some embodiments, the provided methods include cultivation, incubation, culture, and/or genetic engineering steps. The incubation and/or engineering may be carried out in a culture vessel, such as a unit, chamber, well, column, tube, tubing set, valve, vial, culture dish, bag, or other container for culture or cultivating cells. In some embodiments, the cells are incubated and/or cultured prior to or in connection with genetic engineering. The incubation steps can include culture, cultivation, stimulation, activation, and/or propagation. In some embodiments, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some embodiments, the stimulating conditions or agents include one or more agent, e.g., ligand, which is capable of activating an intracellular signaling domain of a TCR complex. In some aspects, the agent turns on or initiates TCR/CD3 intracellular signaling cascade in a T cell. Such agents can include antibodies, such as those specific for a TCR component and/or costimulatory receptor, e.g., anti-CD3, anti-CD28, for example, bound to solid support such as a bead, and/or one or more cytokines. Optionally, the expansion method may further comprise the step of adding anti-CD3 and/or anti CD28 antibody to the culture medium (e.g., at a concentration of at least about 0.5 ng/ml). In some embodiments, the stimulating agents include IL-2 and/or IL-15, for example, an IL-2 concentration of at least about 10 units/mL.

In some aspects, incubation is carried out in accordance with techniques such as those described in US Patent No. 6,040,1 77 to Riddell et al., Klebanoff et al.(2012) J Immunother. 35(9): 651-660, Terakura et al. (2012) Blood.1:72-82, and/or Wang et al. (2012) J Immunother. 35(9):689-701.

In some embodiments, the T cells are expanded by adding to the culture-initiating composition feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g. for a time sufficient to expand the numbers of T cells). In some aspects, the non-dividing feeder cells can comprise gamma-irradiated PBMC feeder cells. In some embodiments, the PBMC are irradiated with gamma rays in the range of about 3000 to 3600 rads to prevent cell division. In some aspects, the feeder cells are added to culture medium prior to the addition of the populations of T cells.

In some embodiments, the stimulating conditions include temperature suitable for the growth of human T lymphocytes, for example, at least about 25 degrees Celsius, generally at least about 30 degrees, and generally at or about 37 degrees Celsius. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some aspects is provided in any suitable amount, such as a ratio of LCL feeder cells to initial T lymphocytes of at least about 10:1.

### B. Pharmaceutical Compositions and Formulations

Also provided are compositions including the cells, including pharmaceutical compositions and formulations, such as unit dose form compositions including the number of cells for administration in a given dose or fraction thereof. The pharmaceutical compositions and formulations generally include one or more optional pharmaceutically acceptable carrier or excipient. In some embodiments, the composition includes at least one additional therapeutic agent.

In some aspects, the choice of carrier is determined in part by the particular cell and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulations can include aqueous solutions. The formulation or composition may also contain more than one active ingredient useful for the particular indication, disease, or condition being treated with the cells, preferably those with activities complementary to the cells, where the respective activities do not adversely affect one another. Such active ingredients are suitably present in combination in amounts that are effective for the purpose intended. Thus, in some embodiments, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, and/or vincristine.

Formulations include those for oral, intravenous, intraperitoneal, subcutaneous, pulmonary, transdermal, intramuscular, intranasal, buccal, sublingual, or suppository administration. In some embodiments, the cell populations are administered parenterally. Parenteral infusions may include intramuscular, intravenous, intraarterial, intraperitoneal, intrathoracic, intracranial, and/or subcutaneous administration. In some embodiments, a given dose is administered by a single bolus administration of the cells. In some embodiments, the cells are administered to the subject using peripheral systemic delivery by intravenous, intraperitoneal, or subcutaneous injection.

Compositions in some embodiments are provided as sterile liquid preparations, e.g., isotonic aqueous solutions, suspensions, emulsions, dispersions, or viscous compositions, which may in some aspects be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyoi (for example, glycerol, propylene glycol, liquid polyethylene glycol) and suitable mixtures thereof.

Sterile injectable solutions can be prepared by incorporating the cells in a solvent, such as in admixture with a suitable carrier, diluent, or excipient such as sterile water, physiological saline, glucose, dextrose, or the like. The compositions can contain auxiliary substances such as wetting, dispersing, or emulsifying agents (e.g., methylcellulose), pH buffering agents, gelling or viscosity enhancing additives, preservatives, flavoring agents, and/or colors, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, and sorbic acid. Prolonged absorption of the injectable pharmaceutical form can be brought about by the use of agents delaying absorption, for example, aluminum monostearate and gelatin.

The formulations to be used for in vivo administration are generally sterile. Sterility may be readily accomplished, e.g., by filtration through sterile filtration membranes.

The pharmaceutical composition in some embodiments contains the cells in amounts effective to treat or prevent the disease or condition, such as a therapeutically effective or prophylactically effective amount.

Administration of the cells can be autologous or allogeneic. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell), it will generally be formulated in a unit dosage injectable form (solution, suspension, emulsion).

### C. Methods of Administration and Uses of cells in adoptive cell therapy

Provided are methods, such as those encompassing administering peptides such as the provided peptides, for example to induce tolerance or reduce or prevent an immune response. In some embodiments, the methods further include administering cells, populations, and compositions, and uses of such cells, populations, and compositions to treat or prevent diseases, conditions, and disorders, including cancers, autoimmune diseases, and infectious diseases. In embodiments of the provided methods, the cells, populations and compositions of cells, including any containing cells expressing a recombinant molecule such as a receptor such as an antigen and/or chimeric receptor, such as a CAR, are co-administered with (generally, administered after) a peptide(s) that contains an epitope or sequence present in or encoded or expressed by the agent, such as the cell or molecule therein or thereon. In some embodiments, the epitope or sequence corresponds to the particular chimeric receptor, such as a CAR, expressed on the genetically engineered cells.

In some embodiments, the cells, populations, and compositions are administered to a subject or patient having the particular disease or condition to be treated, e.g., via adoptive cell therapy, such as adoptive T cell therapy. In some embodiments, cells and compositions prepared by the provided methods, such as engineered compositions and end-of-production compositions following incubation and/or other processing steps, are administered to a subject, such as a subject having or at risk for the disease or condition. In some aspects, the methods thereby treat, e.g., ameliorate one or more symptom of, the disease or condition, such as by lessening tumor burden in a cancer expressing an antigen recognized by an engineered T cell.

Methods for administration of cells for adoptive cell therapy are known and may be used in connection with the provided methods and compositions. For example, adoptive T cell therapy methods are described, e.g., in US Patent Application Publication No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

As used herein, "delaying development of a disease" means to defer, hinder, slow, retard, stabilize, suppress and/or postpone development of the disease (such as cancer). This delay can be of varying lengths of time, depending on the history of the disease and/or individual being treated. As is evident to one skilled in the art, a sufficient or significant delay can, in effect, encompass prevention, in that the individual does not develop the disease. For example, a late stage cancer, such as development of metastasis, may be delayed.

"Preventing," as used herein, includes providing prophylaxis with respect to the occurrence or recurrence of a disease in a subject that may be predisposed to the disease but has not yet been diagnosed with the disease. In some embodiments, the provided cells and compositions are used to delay development of a disease or to slow the progression of a disease.

As used herein, to "suppress" a function or activity is to reduce the function or activity when compared to otherwise same conditions except for a condition or parameter of interest, or alternatively, as compared to another condition. For example, cells that suppress tumor growth reduce the rate of growth of the tumor compared to the rate of growth of the tumor in the absence of the cells.

In some embodiments, the cell therapy, e.g., adoptive T cell therapy, is carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, following isolation and processing are administered to the same subject.

In some embodiments, the cell therapy, e.g., adoptive T cell therapy, is carried out by allogeneic transfer, in which the cells are isolated and/or otherwise prepared from a subject other than a subject who is to receive or who ultimately receives the cell therapy, e.g., a first subject. In such embodiments, the cells then are administered to a different subject, e.g., a second subject, of the same species. In some embodiments, the first and second subjects are genetically identical. In some embodiments, the first and second subjects are genetically similar. In some embodiments, the second subject expresses the same HLA class or supertype as the first subject.

In some embodiments, the subject has been treated with a therapeutic agent targeting the disease or condition, e.g. the tumor, prior to administration of the cells or composition containing the cells. In some aspects, the subject is refractory or non-responsive to the other therapeutic agent. In some embodiments, the subject has persistent or relapsed disease, e.g., following treatment with another therapeutic intervention, including chemotherapy, radiation, and/or hematopoietic stem cell transplantation (HSCT), e.g., allogenic HSCT. In some embodiments, the administration effectively treats the subject despite the subject having become resistant to another therapy.

In some embodiments, the subject is responsive to the other therapeutic agent, and treatment with the therapeutic agent reduces disease burden. In some aspects, the subject is initially responsive to the therapeutic agent, but exhibits a relapse of the disease or condition over time. In some embodiments, the subject has not relapsed. In some such embodiments, the subject is determined to be at risk for relapse, such as at a high risk of relapse, and thus the cells are administered prophylactically, e.g., to reduce the likelihood of or prevent relapse.

In some aspects, the subject has not received prior treatment with another therapeutic agent.

Among the diseases, conditions, and disorders for treatment with the provided compositions, cells, methods and uses are tumors, including solid tumors, hematologic malignancies, and melanomas, and infectious diseases, such as infection with a virus or other pathogen, e.g., HIV, HCV, HBV, CMV, and parasitic disease. In some embodiments, the disease or condition is a tumor, cancer, malignancy, neoplasm, or other proliferative disease or disorder. Such diseases include but are not limited to leukemia, lymphoma, e.g., chronic lymphocytic leukemia (CLL), acute-lymphoblastic leukemia (ALL), non-Hodgkin's lymphoma, acute myeloid leukemia, multiple myeloma, refractory follicular lymphoma, mantle cell lymphoma, indolent B cell lymphoma, B cell malignancies, cancers of the colon, lung, liver, breast, prostate, ovarian, skin, melanoma, bone, and brain cancer, ovarian cancer, epithelial cancers, renal cell carcinoma, pancreatic adenocarcinoma, Hodgkin lymphoma, cervical carcinoma, colorectal cancer, glioblastoma, neuroblastoma, Ewing sarcoma, medulloblastoma, osteosarcoma, synovial sarcoma, and/or mesothelioma.

In some embodiments, the disease or condition is an infectious disease or condition, such as, but not limited to, viral, retroviral, bacterial, and protozoal infections, immunodeficiency, Cytomegalovirus (CMV), Epstein-Barr virus (EBV), adenovirus, BK polyomavirus. In some embodiments, the disease or condition is an autoimmune or inflammatory disease or condition, such as arthritis, e.g., rheumatoid arthritis (RA), Type I diabetes, systemic lupus erythematosus (SLE), inflammatory bowel disease, psoriasis, scleroderma, autoimmune thyroid disease, Grave's disease, Crohn's disease multiple sclerosis, asthma, and/or a disease or condition associated with transplant.

In some embodiments, the antigen associated with the disease or disorder is selected from the group consisting of orphan tyrosine kinase receptor ROR1, tEGFR, Her2, L1-CAM, CD19, CD20, CD22, mesothelin, CEA, and hepatitis B surface antigen, anti-folate receptor, CD23, CD24, CD30, CD33, CD38, CD44, EGFR, EGP-2, EGP-4, EPHa2, ErbB2, 3, or 4, FBP, fetal acethycholine e receptor, GD2, GD3, HMW-MAA, IL-22R-alpha, IL-13R-alpha2, kdr, kappa light chain, Lewis Y, L1-cell adhesion molecule, MAGE-A1, mesothelin, MUC1, MUC16, PSCA, NKG2D Ligands, NY-ESO-1, MART-1, gp100, oncofetal antigen, ROR1, TAG72, VEGF-R2, carcinoembryonic antigen (CEA), prostate specific antigen, PSMA, Her2/neu, estrogen receptor, progesterone receptor, ephrinB2, CD123, CS-1, c-Met, GD-2, and MAGE A3 and/or biotinylated molecules, and/or molecules expressed by HIV, HCV, HBV or other pathogens.

In some embodiments, the cells are administered at a desired dosage, which in some aspects includes a desired dose or number of cells or cell type(s) and/or a desired ratio of cell types. Thus, the dosage of cells in some embodiments is based on a total number of cells (or number per kg body weight) and a desired ratio of the individual populations or sub-types, such as the CD4+ to CD8+ ratio. In some embodiments, the dosage of cells is based on a desired total number (or number per kg of body weight) of cells in the individual populations or of individual cell types. In some embodiments, the dosage is based on a combination of such features, such as a desired number of total cells, desired ratio, and desired total number of cells in the individual populations.

In some embodiments, the populations or sub-types of cells, such as CD8⁺ and CD4⁺ T cells, are administered at or within a tolerated difference of a desired dose of total cells, such as a desired dose of T cells. In some aspects, the desired dose is a desired number of cells or a desired number of cells per unit of body weight of the subject to whom the cells are administered, e.g., cells/kg. In some aspects, the desired dose is at or above a minimum number of cells or minimum number of cells per unit of body weight. In some aspects, among the total cells, administered at the desired dose, the individual populations or sub-types are present at or near a desired output ratio (such as CD4⁺ to CD8⁺ ratio), e.g., within a certain tolerated difference or error of such a ratio.

In some embodiments, the cells are administered at or within a tolerated difference of a desired dose of one or more of the individual populations or sub-types of cells, such as a desired dose of CD4+ cells and/or a desired dose of CD8+ cells. In some aspects, the desired dose is a desired number of cells of the sub-type or population, or a desired number of such cells per unit of body weight of the subject to whom the cells are administered, e.g., cells/kg. In some aspects, the desired dose is at or above a minimum number of cells of the population or sub-type, or minimum number of cells of the population or sub-type per unit of body weight.

Thus, in some embodiments, the dosage is based on a desired fixed dose of total cells and a desired ratio, and/or based on a desired fixed dose of one or more, e.g., each, of the individual sub-types or sub-populations. Thus, in some embodiments, the dosage is based on a desired fixed or minimum dose of T cells and a desired ratio of CD4⁺ to CD8⁺ cells, and/or is based on a desired fixed or minimum dose of CD4⁺ and/or CD8⁺ cells.

In certain embodiments, the cells, or individual populations of sub-types of cells, are administered to the subject at a range of about one million to about 100 billion cells, such as, e.g., 1 million to about 50 billion cells (e.g., about 5 million cells, about 25 million cells, about 500 million cells, about 1 billion cells, about 5 billion cells, about 20 billion cells, about 30 billion cells, about 40 billion cells, or a range defined by any two of the foregoing values), such as about 10 million to about 100 billion cells (e.g., about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or a range defined by any two of the foregoing values), and in some cases about 100 million cells to about 50 billion cells (e.g., about 120 million cells, about 250 million cells, about 350 million cells, about 450 million cells, about 650 million cells, about 800 million cells, about 900 million cells, about 3 billion cells, about 30 billion cells, about 45 billion cells) or any value in between these ranges.

In some embodiments, the dose of total cells and/or dose of individual sub-populations of cells is within a range of between at or about 10⁴ and at or about 10⁹ cells/kilograms (kg) body weight, such as between 10⁵ and 10⁶ cells / kg body weight, for example, at or about 1 × 10⁵ cells/kg, 1.5 × 10⁵ cells/kg, 2 × 10⁵ cells/kg, or 1 × 10⁶ cells/kg body weight. For example, in some embodiments, the cells are administered at, or within a certain range of error of, between at or about 10⁴ and at or about 10⁹ T cells/kilograms (kg) body weight, such as between 10⁵ and 10⁶ T cells / kg body weight, for example, at or about 1 × 10⁵ T cells/kg, 1.5 × 10⁵ T cells/kg, 2 × 10⁵ T cells/kg, or 1 × 10⁶ T cells/kg body weight.

In some embodiments, the cells are administered at or within a certain range of error of between at or about 10⁴ and at or about 10⁹ CD4⁺ and/or CD8⁺ cells/kilograms (kg) body weight, such as between 10⁵ and 10⁶ CD4⁺ and/or CD8⁺cells / kg body weight, for example, at or about 1 × 10⁵ CD4⁺ and/or CD8⁺ cells/kg, 1.5 × 10⁵ CD4⁺ and/or CD8⁺ cells/kg, 2 × 10⁵ CD4⁺ and/or CD8⁺ cells/kg, or 1 × 10⁶ CD4⁺ and/or CD8⁺ cells/kg body weight.

In some embodiments, the cells are administered at or within a certain range of error of, greater than, and/or at least about 1 × 10⁶, about 2.5 × 10⁶, about 5 × 10⁶, about 7.5 × 10⁶. or about 9 × 10⁶ CD4⁺ cells, and/or at least about 1 × 10⁶, about 2.5 × 10⁶, about 5 × 10⁶, about 7.5 × 10⁶, or about 9 × 10⁶ CD8+ cells, and/or at least about 1 × 10⁶, about 2.5 × 10⁶, about 5 × 10⁶, about 7.5 × 10⁶, or about 9 × 10⁶ T cells. In some embodiments, the cells are administered at or within a certain range of error of between about 10⁸ and 10¹² or between about 10¹⁰ and 10¹¹ T cells, between about 10⁸ and 10¹² or between about 10¹⁰ and 10¹¹ CD4⁺ cells, and/or between about 10⁸ and 10¹² or between about 10¹⁰ and 10¹¹ CD8⁺ cells.

For the prevention or treatment of disease, the appropriate dosage may depend on the type of disease to be treated, the type of cells or chimeric receptors, the severity and course of the disease, whether the cells are administered for preventive or therapeutic purposes, previous therapy, the subject's clinical history and response to the cells, and the discretion of the attending physician. The compositions and cells are in some embodiments suitably administered to the subject at one time or over a series of treatments.

The cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, they are administered by parenteral, intrapulmonary, and intranasal, and, if desired for local treatment, intralesional administration. Parenteral infusions include intramuscular, intravenous, intraarterial, intraperitoneal, or subcutaneous administration. In some embodiments, a given dose is administered by a single bolus administration of the cells. In some embodiments, it is administered by multiple bolus administrations of the cells, for example, over a period of no more than 3 days, or by continuous infusion administration of the cells.

In some embodiments, the cells are administered as part of a combination treatment, such as simultaneously with or sequentially with, in any order, another therapeutic intervention, such as an antibody or engineered cell or receptor or agent, such as a cytotoxic or therapeutic agent. The cells in some embodiments are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some embodiments, the cells are administered prior to the one or more additional therapeutic agents. In some embodiments, the cells are administered after the one or more additional therapeutic agents. In some embodiments, the one or more additional agents includes a cytokine, such as IL-2, for example, to enhance persistence. In some embodiments, the methods comprise administration of a chemotherapeutic agent.

Following administration of the cells, the biological activity of the engineered cell populations in some embodiments is measured, e.g., by any of a number of known methods. Parameters to assess include specific binding of an engineered or natural T cell or other immune cell to antigen, *in vivo,* e.g., by imaging, or *ex vivo,* e.g., by ELISA or flow cytometry. In certain embodiments, the ability of the engineered cells to destroy target cells can be measured using any suitable method known in the art, such as cytotoxicity assays described in, for example, Kochenderfer et al., J. Immunotherapy, 32(7): 689-702 (2009), and Herman et al. J. Immunological Methods, 285(1): 25-40 (2004). In certain embodiments, the biological activity of the cells is measured by assaying expression and/or secretion of one or more cytokines, such as CD 107a, IFNγ, IL-2, and TNF. In some aspects the biological activity is measured by assessing clinical outcome, such as reduction in tumor burden or load.

### IV. Monitoring a Host Immune Response

In some embodiments, the immune response of a subject to a chimeric receptor, such as a CAR, administered to a subject, including in the context of administering cells genetically engineered with the chimeric response, can be monitored. In some embodiments, immune response measures may be made in subjects naive to the antigen or previously sensitized to the antigen, i.e. subjects that have never been treated with genetically engineered cells expressing the chimeric receptor or subjects that have been previously treated with genetically engineered cells expressing the chimeric receptor, respectively. In some cases, such measures of an immune response also may be made in subjects prior to and/or after receiving thepeptide treatment, such as in accord with the provided methods. Any detectable immune response, such as a detectable humoral or cell-mediated immune response, can be measured. In some aspects, the presence or absence of such a host immune response and/or quantity, degree, or extent thereof, is detected or measured, for example, following the administration of cells expressing the chimeric receptor (e.g. CAR) in the presence or absence of co-administration, such as prior administration, of the peptide.

Antigen-specific immune responses may be periodically monitored following administration of a peptide(s). Tolerance induction can be confirmed when the immune response is prevented, reduced or otherwise downregulated in an antigen specific manner. For example, in some aspects, tolerance induction may be confirmed by showing a decrease in antigen specific antibody levels or antigen specific lymphocyte levels to administered antigen in a sensitized subject when co-administered with a peptide according to the provided methods, where the individual has pre-existing antibody or lymphocyte levels to the antigen. In some aspects, where a subject is naive to the antigen (i.e. has not previously been treated with cells expressing the chimeric receptor (e.g. CAR), tolerance induction may be confirmed by showing antigen specific antibody levels or antigen specific lymphocyte levels after treatment that are consistent with control levels of an immune response expected in a subject with immune tolerance. In other cases, where a subject is naive to the antigen (i.e. has not previously been treated with cells expressing the chimeric receptor (e.g. CAR), tolerance induction may be confirmed by showing antigen specific antibody levels or antigen specific lymphocyte levels after treatment that are consistent with control levels present in the subject prior to treatment with the cells expressing the chimeric receptor. Control levels can be established using methods well known to those of ordinary skill in the art. For example, T cells proliferation may be measured in response to the antigen in question. Immunoglobulin levels may be also measured to assess an immune response.

In some embodiments, the host immune response is or comprises a humoral immune response. The humoral immune response may be indicated by the presence of antibodies specific for the cells or receptors expressed thereby in the serum, other bodily fluid, and/or organ or tissue of the subject. In some embodiments, such antibodies of a particular isotype are present, such as IgM or IgG, e.g., IgG1, IgG2, IgG3, and/or IgG4; in some embodiments they include IgE.

In some embodiments, the immune response is or comprises a cell-mediated component. A cell-mediated response may be indicated by the presence of cells, e.g., T cells, e.g., helper or cytotoxic T cells, that specifically recognize one or more epitopes of the recombinant receptor or cells via a T cell receptor.

In some embodiments the immune response is a primary immune response; in some aspects, the immune response is a memory response.

In some of any of the above embodiments, a detectable immune response refers to an amount detectable by any of a number of known methods for assessing specific immune responses to particular antigens and cells. For example, in some embodiments, the immune response of the specified type is detectable by performing ELISpot, ELISAs, or cell-based antibody detection methods, for example, by flow cytometry, on serum from the subject to detect the presence of antibodies that specifically bind to and/or neutralize antigens present on the cells, e.g., binding to epitopes of the recombinant receptor, e.g., CAR. In some such assays, isotype of the detected antibody is determined and may indicate the type of response and/or whether the response is a memory response.

In some embodiments, the specified immune response is detectable by cytotoxic T-lymphocyte (CTL) assays for detection of CD8+ T cells that specifically bind to and induce cytotoxicity in response to epitopes in the recombinant receptor, and/or a mixed lymphocyte reaction, using cells, e.g., irradiated cells, expressing the recombinant receptor, as stimulator cells.

In some aspects, the detectable immune response is one that is detected by such a method above or significantly above the level of a control sample, such as a non-coated well or well coated with a control peptide or cells not expressing the chimeric receptor and/or levels detected based on pre-treatment serum or blood sample from the subject prior to treatment with the cells expressing the chimeric receptor.

Humoral immune responses may be detected by any of a number of well-known assays for detection of antibodies specific for particular antigens or cells, including binding assays, immunoassays, and including cell-based assays. The assays may include those designed to assess the presence or absence of particular functions of the antibodies, such as their ability to carry out a particular effector function upon binding to the antigen, such as neutralizing antibody assays. In some embodiments, outcomes of humoral immune responses, such as antigen-specific antibodies, e.g., neutralizing antibodies, are detected using cell-based assays, e.g., by incubating pre- and post-treatment cells from the subject with cells expressing the recombinant receptor (and control cells) and detecting antigen-specific binding and/or other outcomes, such as neutralizing outcomes, e.g., by flow cytometry or enzymatic assays. In some embodiments, ELISA, and/or ELISpot assays are used to detect and quantify antibodies specific for the recombinant receptors, such as CARs, and epitopes mapped using known techniques, such as those using individual peptides representing portions of the receptor. See, e.g., Berger et al. Blood. 2006 March; 107(6): 2294-2302, Berger et al. J Virol. 2001 January 75(2): 799-808, Riddell et al. Nature Medicine. 1996 February 2(2): 216-223, Berger et al. Blood. 2005 February 105(4): 1640-1647, Jensen et al. Biol Blood Marrow Transplant. 2010 September; 16(9): 1245-1256. In some embodiments isotype of the detected antibodies are assessed, for example by using detection antibodies specific for particular isotypes, e.g., human isotypes.

Cellular or cell-based immune response to the cells and/or receptors may be detected and/or measured using any of a number of well-known techniques. Such techniques may include cytotoxic T-lymphocyte (CTL) assays for detection of CD8+ T cells that specifically bind to and induce cytotoxicity in response to epitopes in the recombinant receptor, e.g., CAR, and/or cells administered. In some embodiments, the assay is a mixed lymphocyte reaction, such as those using PBMCs or other host-derived cells from blood or other organ or tissue as responder cells, and cells induced to express the recombinant receptor, e.g., irradiated T cells expressing the CAR, as stimulator cells. The stimulator cells generally are autologous and may be the same cells administered to the subject, and may be irradiated. Non-transduced cells or cells not expressing the transgene of interest may be used as negative controls in place of the stimulator cells in control samples. Likewise, responder cell samples from pre-treated time points or other subjects may be used in control samples. In some aspects, such assays assess the ability of host cells to carry out one or more effector functions, e.g., antigen-specific cell lysis, e.g., using a chromium release assay to detect cytotoxic T cells present in the subject which specifically recognize and antigens present on or in the administered cells and induce a cytotoxic response. In some embodiments, peripheral blood cells, e.g., PBMCs, are obtained from a subject before and after administration of the cells, and each used in an assay, such as a cell lysis assay, using autologous T cells modified to express the recombinant receptor, which generally are irradiated. Specific lysis indicates the presence of receptor-specific cell-mediated immune response. Epitope mapping may be carried out using panels of peptides representing portions of the recombinant receptor. See, e.g., Berger et al. Blood. 2006 March; 107(6): 2294-2302, Berger et al. J Virol. 2001 January 75(2): 799-808, Riddell et al. Nature Medicine. 1996 February 2(2): 216-223, Berger et al. Blood. 2005 February 105(4): 1640-1647, Lamers, Blood 2011 117: 72-82. HLA tetramer binding assays may be used for the enumeration of antigen-specific T cells. In some aspects, lymphoproliferative assays (LPAs) and/or assays to assess for secreted cytokines, such as ELISAs and/or intracellular staining and assessment by flow cytometry, are used for detection of transgene-specific CD4+ T cells.

In some embodiments, following administration of the chimeric receptor (e.g. CAR) to a subject also administered a peptide(s), the subject does not exhibit a detectable humoral and/or cell-mediated immune response specific for the chimeric receptor (e.g. CAR). The presence or degree of a specific immune response to the chimeric receptor can be related to the immunogenic properties of the receptor, e.g., the CAR, expressed by the cells, and/or the time during which the subject has been exposed thereto. For example, in some embodiments, an immune response, e.g., a specific humoral and/or cell-mediated immune response against the receptor, is detected at or about 28 days, at or about 35 days, or at or about 42 days following exposure of the subject to the cells expressing receptor. In some embodiments, the subject does not exhibit a detectable humoral or cell-mediated immune response against the chimeric receptor (e.g. CAR) within about 30 days, within about 60 days, or within about 90 days of the administration of cells expressing the chimeric receptor (e.g. CAR).

The methods may involve the detection of the presence or absence or level of such an immune response or indicator thereof, for example, following the administration of the chimeric receptor (e.g. CAR) in conjunction with administration of a peptide according to the methods provided herein.

In some embodiments, the provided methods ameliorate or prevent the induction of or reduce the level of antibodies against the chimeric receptor, such as a chimeric receptor expressed by the cells administered in the course of adoptive cell therapy. For example, antibody titers of anti-receptor, e.g. anti-CAR, antibodies, for example, as measured in the serum of the subject by ELISA, are decreased following administration of the peptide (e.g. such as by pre-administration of the peptide), as compared to methods in which the chimeric receptor (e.g. CAR), such as cells expressing the chimeric receptor, is administered in the absence of a peptide.

### V. Articles of Manufacture

Also provided are articles of manufacture, such as kits and devices, for use in connection with methods for inducing immune tolerance to a chimeric receptor, such as in conjunction with treatment methods employing the chimeric receptor, such as adoptive cell therapy methods.

The articles of manufacture include one or more containers, typically a plurality of containers, packaging material, and a label or package insert on or associated with the container or containers and/or packaging, generally including instructions for administration of the tolerorgenic peptide and/or administration of the cells to a subject.

In some embodiments, the article of manufacture, such as a kit, includes a container containing the peptide(s) to be used for inducing immune tolerance and, optionally, one or more containers containing agents for delivering the peptide(s). For example, in some aspects, the one or more additional containers contain an adjuvant and a agent. The instructions may include instructions for treating a subject with an effective amount of the peptide(s) and, optionally, one or more of each of the other components. For example, in some embodiments, the instructions include instructions for administering the peptide(s) by a particular route of administration and/or in a dosage amount for inducing tolerance. In some embodiments, the instruction can include instructions to administer the peptide(s) in the absence of adjuvant or other agent. In some embodiments, the instructions can include instructions to administer the peptide(s) in the presence of adjuvant and with a agent. In some cases, the instructions can provide instructions related to the dosage regime and frequency of administration of the peptide and, optionally, the one or more other agents.

In some aspects, the article of manufacture, such as a kit, can further include a pharmaceutical preparation container and a pharmaceutical preparation diluent and a concentrated or lyophilized preparation of the isolated peptide antigen. In some cases, the container containing the diluent contains a diluent, such as a pharmaceutically acceptable buffer, for diluting what could be a concentrated solution or lyophilized powder of the antigen, and in some aspects, optionally, the adjuvant and/or the tolerogenic agent. The instructions can include instructions for mixing a particular amount of the diluent with a particular amount of the concentrated pharmaceutical preparation, whereby a final formulation for injection or infusion is prepared.

In some embodiments, the articles of manufacture, such as kits, are for administration of the cells, such as compositions containing the cells, to subjects in accord with the provided methods for adoptive cell therapy, and for storage and administration of the cells and compositions. In some embodiments, the containers contain the cells to be administered for multiple or single dosage administration. For example, the containers can contain one or more unit doses thereof. Generally, the article of manufacture, such as a kit, includes cells engineered to express a chimeric receptor, for example a CAR, that corresponds to the peptide(s) also contained in a container of the article of manufacture, i.e. the chimeric receptor is one in which immune tolerance can be induced by co-administering the peptide.

In addition to the above one or more containers, the article of manufacture in some embodiments includes one or more additional containers with a composition contained therein which includes a further agent, such as an immunosuppressive agent, cytotoxic agent, or otherwise therapeutic agent, for example, which is to be administered in combination, e.g., simultaneously or sequentially in any order, with the cells. Alternatively, or additionally, the article of manufacture may further include another or the same container comprising a pharmaceutically-acceptable buffer. It may further include other materials such as other buffers, diluents, filters, tubing, needles, and/or syringes.

Suitable containers include, for example, bottles, vials, syringes, and flexible bags, such as infusion bags. In particular embodiments, the containers include bags, e.g., flexible bags, such as those suitable for infusion of cells to subjects, e.g., flexible plastic or PVC bags, and/or IV solution bags. The bags in some embodiments are sealable and/or able to be sterilized, so as to provide sterile solution and delivery of the cells and compositions. In some embodiments, the containers, e.g., bags, have a capacity of at or about or at least at or about 10, 20, 30, 40, 50, 60, 70, 80, 90, 100, 200, 300, 400, 500, or 1000 ml capacity, such as between at or about 10 and at or about 100 or between at or about 10 and at or about 500 mL capacity. In some embodiments, the containers, e.g., bags, are and/or are made from material which is stable and/or provide stable storage and/or maintenance of cells at one or more of various temperatures, such as in cold temperatures, e.g. below at or about or at or about - 20°C, -80°C, -120°C, 135°C and/or temperatures suitable for cryopreservation, and/or other temperatures, such as temperatures suitable for thawing the cells and body temperature such as at or about 37 °C, for example, to permit thawing, e.g., at the subject's location or location of treatment, e.g., at bedside, immediately prior to treatment.

The containers may be formed from a variety of materials such as glass or plastic. In some embodiments, the container has one or more port, e.g., sterile access ports, for example, for connection of tubing or cannulation to one or more tubes, e.g., for intravenous or other infusion and/or for connection for purposes of transfer to and from other containers, such as cell culture and/or storage bags or other containers. Exemplary containers include infusion bags, intravenous solution bags, vials, including those with stoppers pierceable by a needle for injection.

The article of manufacture may further include a package insert or label with one or more pieces of identifying information and/or instructions for use. In some embodiments, the information or instructions indicates that the contents can or should be used to administer the peptide and the cell population and/or providing instructions therefor.

In some embodiments, the information or instructions indicates that the contents can or should be used to treat a particular condition or disease, and/or providing instructions therefor. The label or package insert may indicate that the contents of the article of manufacture are to be used for treating the disease or condition. In some embodiments, the label or package insert provides instructions to treat a subject, e.g., the subject from which the cells have been derived, via a method involving the administration of an appropriate dosage for treating a disease or condition.

In some embodiments, the label or package insert or packaging comprises an identifier to indicate the specific identity of the subject from which the cells are derived and/or are to be administered. In the case of autologous transfer, the identity of the subject from which the cells are derived is the same as the identity of the subject to which the cells are to be administered. Thus, the identifying information may specify that the cells are to be administered to a particular patient, such as the one from which the cells were originally derived. Such information may be present in the packaging material and/or label in the form of a bar code or other coded identifier, or may indication the name and/or other identifying characteristics of the subject.

The term "package insert" is used to refer to instructions customarily included in commercial packages of therapeutic products, that contain information about the indications, usage, dosage, administration, combination therapy, contraindications and/or winnings concerning the use of such therapeutic products.

### VI. Definitions

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

As used herein, the term "recombinant" refers to a cell, microorganism, nucleic acid molecule, or vector that has been modified by introduction of an exogenous, such as heterologous, nucleic acid molecule, or refers to a cell or microorganism that has been altered such that expression of an endogenous nucleic acid molecule or gene is controlled, deregulated or constitutive, where such alterations or modifications may be introduced by genetic engineering. Genetic alterations may include, for example, modifications introducing nucleic acid molecules (which may include an expression control element, such as a promoter) encoding one or more proteins or enzymes, or other nucleic acid molecule additions, deletions, substitutions, or other functional disruption of or addition to a cell's genetic material. Exemplary modifications include those in coding regions or functional fragments thereof of heterologous or homologous polypeptides from a reference or parent molecule.

The term "about" as used herein refers to the usual error range for the respective value readily known to the skilled person in this technical field. Reference to "about" a value or parameter herein includes (and describes) embodiments that are directed to that value or parameter per se. For example, description referring to "about X" includes description of "X".

As used herein, "depleting" when referring to one or more particular cell type or cell population, refers to decreasing the number or percentage of the cell type or population, e.g., compared to the total number of cells in or volume of the composition, or relative to other cell types, such as by negative selection based on markers expressed by the population or cell, or by positive selection based on a marker not present on the cell population or cell to be depleted. The term does not require complete removal of the cell, cell type, or population from the composition.

As used herein, "enriching" when referring to one or more particular cell type or cell population, refers to increasing the number or percentage of the cell type or population, e.g., compared to the total number of cells in or volume of the composition, or relative to other cell types, such as by positive selection based on markers expressed by the population or cell, or by negative selection based on a marker not present on the cell population or cell to be depleted. The term does not require complete removal of other cells, cell type, or populations from the composition and does not require that the cells so enriched be present at or even near 100 % in the enriched composition.

As used herein, a statement that a cell or population of cells is "positive" or "+" for a particular marker refers to the detectable presence on or in the cell of a particular marker, typically a surface marker. When referring to a surface marker, the term refers to the presence of surface expression as detected, in some embodiments, by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected, in some embodiments, by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

As used herein, "isolated" or "purified with reference to a peptide refers to a peptide which is substantially free of all other polypeptides, contaminants, starting reagents or other materials, or substantially free from chemical precursors or other chemicals when chemically synthesized. Preparations can be determined to be substantially free if they appear free of readily detectable impurities as determined by standard methods of analysis, such as high-performance liquid chromatography (HPLC), thin-layer chromatography (TLC) or capillary electrophoresis (CE), used by those of skill in the art to assess such purity, or sufficiently pure such that further purification would not detectably alter the physical and chemical properties of the substance.

As used herein, "percent (%) amino acid sequence identity" and "percent identity" when used with respect to an amino acid sequence (reference polypeptide sequence) is defined as the percentage of amino acid residues in a candidate sequence (e.g., a Vpx or Vpr protein) that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

An amino acid substitution may include replacement of one amino acid in a polypeptide with another amino acid. Amino acids generally can be grouped according to the following common side-chain properties:
(1) hydrophobic: Norleucine. Met, Ala, Val, Leu, Ile:
(2) neutral hydrophilic: Cys, Ser, Thr. Asn. Gln:
(3) acidic: Asp. Glu;
(4) basic: His. Lys. Arg;
(5) residues that influence chain orientation: Gly. Pro;
(6) aromatic: Trp, Tyr, Phe.

Non-conservative amino acid substitutions will involve exchanging a member of one of these classes for another class. A conservative amino acid substitution refers to an amino acid substitution that does not alter the relative charge or size characteristics of the protein in which the amino acid substitution is made, and generally includes substitutions involving exchanging a member of one of these classes for a different member of the same class.

As used herein, a control refers to a sample that is substantially identical to the test sample, except that it is not treated with a test parameter, or, if it is a plasma sample, it can be from a normal volunteer not affected with the condition of interest. A control also can be an internal control.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors." Vectors include viral vectors, such as retroviral vectors, for example lentiviral or gammaretroviral vectors, having a genome carrying another nucleic acid and capable of inserting into a host genome for propagation thereof.

As used herein, a composition refers to any mixture of two or more products, substances, or compounds, including cells. It may be a solution, a suspension, liquid, powder, a paste, aqueous, non-aqueous or any combination thereof.

As used herein, "pharmaceutical formulation" refers to a preparation which is in such form as to permit the biological activity of an active ingredient contained therein to be effective, and which contains no additional components which are unacceptably toxic to a subject to which the formulation would be administered.

As used herein, a "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, a "subject" is a mammal, such as a human or other animal, and typically is human. In some embodiments, the subject, e.g., patient, to whom the cells, cell populations, or compositions are administered is a mammal, typically a primate, such as a human. In some embodiments, the primate is a monkey or an ape. The subject can be male or female and can be any suitable age, including infant, juvenile, adolescent, adult, and geriatric subjects. In some embodiments, the subject is a non-primate mammal, such as a rodent.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The terms do not imply complete curing of a disease or complete elimination of any symptom or effect(s) on all symptoms or outcomes. In certain embodiments, the effect is therapeutic, such that it partially or completely cures a disease or condition or adverse symptom attributable thereto.

An "effective amount" of an agent, e.g., a pharmaceutical formulation, cells, or composition, in the context of administration, refers to an amount effective, at dosages/amounts and for periods of time necessary, to achieve a desired result, such as a therapeutic or prophylactic result.

A "therapeutically effective amount" of an agent, e.g., a compound, composition, a pharmaceutical formulation or cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the populations of cells administered. In some embodiments, the provided methods involve administering the cells and/or compositions at effective amounts, e.g., therapeutically effective amounts.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount. In the context of lower tumor burden, the prophylactically effective amount in some aspects will be higher than the therapeutically effective amount.

### VII. Exemplary Embodiments

Among the provided embodiments are:
1. A method of inducing tolerance to a chimeric receptor, comprising administering to a subject at least one peptide comprising all or a portion of an immunogenic region of said chimeric receptor, said peptide being administered under conditions that induces tolerance in the subject to the chimeric receptor.
2. The method of embodiment 1, wherein the chimeric receptor is expressed by cells genetically engineered with nucleic acid encoding the chimeric receptor.
3. The method of embodiment 1, further comprising administering to the subject the chimeric receptor and/or administering cells expressing the chimeric receptor for treating a disease or condition in the subject.
4. The method of any of embodiments 1-3, wherein the chimeric receptor is a chimeric antigen receptor.
5. A method of treatment, comprising:
   a) administering to a subject a peptide comprising all or a portion of an immunogenic region of a chimeric antigen receptor, said peptide being administered under conditions that induce tolerance in the subject to the chimeric antigen receptor; and
   b) administering to the subject cells expressing the chimeric antigen receptor, wherein the chimeric antigen receptor specifically binds to an antigen associated with a disease or condition in the subject.
6. The method of any of embodiments 3-5, wherein the peptide is administered prior to, subsequently or intermittently from administration of the chimeric receptor.
7. The method of any of embodiments 3-6, wherein the peptide is administered prior to administration of the chimeric receptor.
8. The method of any of embodiments 3-7, wherein the peptide is administered from or from about 7 to 28 days before administration of the chimeric receptor.
9. The method of any of embodiments 1-8, wherein the peptide is administered in the absence of an adjuvant.
10. The method of any of embodiments 1-8, wherein the method further comprises administering a tolerogenic agent.
11. The method of embodiment 10, wherein the tolerogenic agent targets one or more molecules that is expressed on T cells and/or a molecule selected from among CD4, CD40, CD40L, OX40, OX40L, CD26, CD44, CD28, PD1, BTLA, B7-1, ICOS, CTLA-4, B7-2 family, CD99, CD137 (4-1BBL), CD2, LFA3, CD27, CD70, CD3, CD8, ICAM1, LFA1, MHC class II and MHC class I molecule, and/or a .
12. The method of embodiment 11, wherein the tolerogenic agent is an antibody or fragment thereof or is a soluble fusion protein.
13. The method of any of embodiments 10-12, wherein the peptide is administered in the presence of adjuvant.
14. The method of any of embodiments 1-13, wherein the peptide is administered by, intravenous, intraperitoneal, intranasal, oral or intrathymic administration.
15. The method of embodiment 14, wherein the peptide is administered by intraperitoneal injection.
16. The method of any of embodiments 1-15, wherein the peptide is administered in an amount that from or from about is 0.1 mg and 1000 mg.
17. The method of any of embodiments 1-16, wherein the peptide is administered in an amount that is from or from about 100 mg to 1000 mg.
18. The method of any of embodiments 1-16, wherein the peptide is administered in an amount that is from or from about 0.1 mg to 5 mg.
19. The method of any of embodiments 1-18, wherein the peptide is administered a plurality of times by repeated administration.
20. The method of embodiment 19, wherein the peptide is administered at least two times, at least three times, at least four times, at least five times, at least six times or at least seven times.
21. The method of any of embodiments 1-20, wherein the peptide is administered at least once daily for two days, three days, four days, five days, six days or seven days.
22. The method of any of embodiments 19-21, wherein each administration of the peptide occurs prior to administration of the chimeric receptor.
23. The method of any of embodiments 1-22, wherein the peptide comprises a plurality of peptides, each of said peptides comprising all or a portion of an immunogenic region of said chimeric receptor.
24. The method of embodiment 23, wherein the plurality of peptides are peptides of two or more different immunogenic regions of said chimeric receptor.
25. The method of embodiment 24, wherein the plurality of peptides are peptides of the same immunogenic region of said chimeric receptor, each of said peptides overlapping in sequence and comprising a portion of said immunogenic region.
26. The method of any of embodiments 23-25, wherein the plurality of peptides comprises at least two, three, four, five, six, seven, eight, nine or ten different peptides.
27. The method of any of embodiments 1-26, wherein said immunogenic region comprises a T cell epitope.
28. The method of embodiment 27, wherein the T cell epitope is an MHC class I or an MHC class II epitope.
29. The method of embodiment 27, wherein the T cell epitope is an MHC class I epitope.
30. The method of any of embodiments 1-29, wherein the subject is positive for an HLA allele that specifically binds to the administered peptide.
31. The method of any of embodiments 1-30, comprising prior to administering the peptide, selecting a subject that is positive for an HLA allele that specifically binds to the administered peptide.
32. The method of any of embodiments 29-31, wherein the HLA allele is selected from among HLA-A^{∗}02:01, HLA-A^{∗}03:01, HLA-A^{∗}11:01 and HLA-B^{∗}08:01.
33. The method of any of embodiments 1-32, wherein the immunogenic region comprises a region within one or more portions selected from the group consisting of an scFv portion, a linker portion, an amino acid sequence not endogenous to the subject, a sequence derived from a different species than that of the subject, and/or a junction between two CAR domains; and/or where the region of the chimeric receptor is a junction region comprising amino acids on each side of a junction between two domains.
34. The method of any of embodiments 1-33, wherein:
   the region comprises a framework region (FR) within the scFv portion,
   the region comprises a heavy chain FR sequence
   the region comprises a heavy chain CDR sequence,
   the region comprises a light chain FR sequence, and/or
   the region comprises a light chain CDR sequence.
35. The method of any of embodiments 1-33, wherein the immunogenic region comprises a contiguous sequence of amino acids of a junction region of the chimeric receptor, wherein the junction region comprises up to 15 contiguous amino acids directly C-terminal of a junction that joins a first domain and a second domain of the chimeric receptor and/or up to 15 contiguous amino acids directly N-terminal of the junction, and optionally further comprises the junction.
36. The method of embodiment 35, wherein the first domain and second domain are directly linked or are indirectly linked via a linker or linkers.
37. The method of embodiment 35 or embodiment 36, wherein each of the first and second domain comprise domain sequences of a protein derived from the same species as the subject and/or domain sequences of a protein endogenous to the subject.
38. The method of any of embodiments 1-37, wherein the subject is a human.
39. The method of any of embodiments 35-38, wherein the peptide comprises a contiguous sequence of amino acids of the junction region.
40. The method of any of embodiments 1-39, wherein the peptide is between 7 and 30 amino acids, 8 and 20 amino acids, 8 and 15 amino acids, 10 and 17 amino acids, 7 and 13 amino acids or 8 and 10 amino acids.
41. The method of any of embodiments 1-38, wherein the peptide is between 10 and 17 amino acids.
42. The method of any of embodiments 1-41, wherein the peptide is or is about 10 amino acids, 11 amino acids, 12 amino acids, 13 amino acids, 14 amino acids, 15 amino acids, 16 amino acids or 17 amino acids.
43. The method of any of embodiments 1-42, wherein the chimeric receptor is a CAR and the CAR comprises an extracellular antigen-recognition domain that specifically binds to a target antigen and an intracellular signaling domain comprising an activating cytoplasmic signaling domain, and/or comprises an inhibitory domain or intracellular domain that does not signal.
44. The method of embodiment 43, wherein the extracellular antigen-recognition domain comprises an antibody or antigen-binding fragment.
45. The method of embodiment 43 or embodiment 44, wherein the extracellular antigen-recognition domain comprises an scFv.
46. The method of any of embodiments 43-45, wherein:
   the activating cytoplasmic signaling domain comprises a T cell receptor (TCR) component and/or comprise an immunoreceptor tyrosine-based activation motif (ITAM); and/or
   the activating cytoplasmic signaling domain comprises an intracellular domain of a CD3-zeta (CD3ζ) chain.
47. The method of any of embodiments 43-46, further comprising a transmembrane domain linking the extracellular domain and the intracellular signaling domain.
48. The method of embodiment 47, wherein the transmembrane domain comprises a transmembrane portion of CD28.
49. The method of any of embodiments 43-48, wherein the intracellular signaling domain further comprises an intracellular costimulatory signaling domain of a T cell costimulatory molecule.
50. The method of embodiment 49, wherein the T cell costimulatory molecule is selected from the group consisting of CD28 and 41BB.
51. The method of any of embodiments 35-50, wherein the first domain and second domain are, respectively, an extracellular ligand binding domain and a hinge domain, an extracellular ligand binding domain and a transmembrane domain, a transmembrane domain and an intracellular costimulatory signaling domain, and an intracellular costimulatory signaling domain and an ITAM signaling domain.
52. The method of any of embodiments 35-51, wherein the first domain is a transmembrane domain or a functional portion thereof and the second domain is a costimulatory signaling domain or a functional portion thereof.
53. The method of embodiment 52, wherein the transmembrane domain is a CD28 transmembrane domain or a functional portion or variant thereof and the co stimulatory signaling domain is a 4-1BB signaling domain or a functional portion or variant thereof.
54. The method of embodiment 53, wherein
   the CD28 transmembrane domain comprises the sequence of amino acids set forth in SEQ ID NO:2, 103 or 104 or a functional portion or variant thereof comprising a sequence that exhibits at least 95% sequence identity to SEQ ID NO:2, 103 or 104; and
   the 4-1BB costimulatory signaling domain comprises the sequence of amino acids set forth in SEQ ID NO:3 or a functional portion or variant thereof comprising a sequence that exhibits at least 95% sequence identity to SEQ ID NO:3.
55. The method of embodiment 53 or embodiment 54, wherein the first domain and second domain together comprise the sequence of amino acids set forth in SEQ ID NO:5 or a functional portion or variant thereof comprising a sequence of amino acids that exhibits at least 85%, 86%, 87%, 88%, 89%, 90%, 91%, 92%, 93%, 94%, 95%, 96%, 97%, 98% or 99% sequence identity to SEQ ID NO:5.
56. The method of any of embodiments 53-54, wherein the first domain and second domain together comprise the sequence of amino acids set forth in SEQ ID NO:5.
57. The method of any of embodiments 1-56, wherein the peptide or one or more peptides of the plurality of peptides comprise:
   i) a sequence of amino acids set forth in any of SEQ ID NOS: 6, 7, 8-12, 16-22, 137, 160, 163, 165, 166, 167 or 168 or a variant thereof that retains activity to induce tolerance; or ii) a peptide epitope of a sequence of i) that binds to an HLA molecule.
58. The method of any of embodiments 1-57, wherein the peptide has a binding affinity for a human leukocyte antigen (HLA) molecule that is less than 1000 nM, less than 500 nM or less than 50 nM.
59. The method of embodiment 58, wherein the binding affinity is an IC50.
60. The method of any of embodiment 57-59, wherein:
   the peptide binds an MHC class I and the HLA allele is selected from among HLA-A^{∗}02:01, HLA-A^{∗}03:01, HLA-A^{∗}11:01 and HLA-B^{∗}08:01; or
   the peptide binds an MHC class II and the HLA allele comprises an alpha and/or beta chain selected from HLA-DPA1^{∗}0103, HLA-DPA1^{∗}0201, HLA-DPB1^{∗}0101, HLA-DPB1^{∗}0301, HLA-DPB1^{∗}0401, HLA-DPB^{∗}0402, HLA-DPB1^{∗}1501, HLA-DRA^{∗}0101, HLA-DRB1^{∗}1101.
61. The method of any of embodiments 1-60, wherein the peptide is administered no more than one week prior to administering the chimeric receptor.
62. The method of any of embodiments 1-61, further comprising administering an immunosuppressive compound.
63. The method of any of embodiments 3-62, wherein the chimeric antigen receptor specifically binds to an antigen associated with the disease or condition and/or an antigen that is not expressed or is not associated with the disease or condition, such as to improve safety.
64. The method of embodiment 63, wherein the disease or condition is a cancer, and autoimmune disease or disorder, or an infectious disease.
65. Use of a peptide for formulation of a medicament for reducing an immune response associated with treatment with a chimeric receptor, wherein said peptide is formulated for administration to induce tolerance in a subject to the chimeric receptor.
66. A pharmaceutical composition, comprising a peptide for use in reducing an immune response associated with treatment with a chimeric receptor, wherein said peptide is formulated for administration to induce tolerance in a subject to the chimeric receptor.
67. The use of embodiment 65 or pharmaceutical composition of embodiment 66, wherein the peptide is formulated for administration in the absence of an adjuvant.
68. Use of a chimeric receptor for formulation of a medicament for treating a disease or condition in a subject that has received a peptide to induce tolerance to the chimeric receptor.
69. A pharmaceutical composition, comprising a chimeric receptor for use in treating a disease or condition in a subject that has received a peptide to induce tolerance to the chimeric receptor.
70. The use or pharmaceutical composition of any of embodiments 65-69, wherein the chimeric receptor is a chimeric antigen receptor.
71. The use or pharmaceutical composition of any of embodiments 65-70, wherein the chimeric receptor is expressed by cells genetically engineered with nucleic acid encoding the chimeric receptor.
72. The use or pharmaceutical composition of any of embodiments 65-71, wherein the chimeric receptor binds to an antigen associated with the disease or condition.
73. The use or pharmaceutical composition of embodiment 72, wherein the disease or condition is a cancer, and autoimmune disease or disorder, or an infectious disease.
74. An isolated peptide, comprising a contiguous sequence of amino acids of a junction region of a chimeric receptor, wherein the junction region comprises up to 15 contiguous amino acids directly C-terminal of a junction that joins a first domain of the chimeric receptor and a second domain of the chimeric receptor and/or up to 15 contiguous amino acids directly N-terminal of the junction, and optionally further comprises the junction.
75. The isolated peptide of embodiment 74, wherein the first domain and second domain are directly linked or are indirectly linked via a linker or linkers.
76. The isolated peptide of embodiment 74 or embodiment 75, wherein each of the first and second domain comprise domain sequences of a protein derived from the same species as the subject and/or domain sequences of a protein endogenous to the subject.
77. The isolated peptide of any of embodiments 74-76, wherein the first domain and second domain are, respectively, an extracellular ligand binding domain and a hinge domain, an extracellular ligand binding domain and a transmembrane domain, a transmembrane domain and an intracellular costimulatory signaling domain, and an intracellular costimulatory signaling domain and an ITAM signaling domain.
78. The isolated peptide of any of embodiments 74-77, wherein the first domain is a transmembrane domain or a functional portion thereof and the second domain is a costimulatory signaling domain or a functional portion thereof.
79. The isolated peptide of embodiment 78, wherein the transmembrane domain is a CD28 transmembrane domain or a functional portion or variant thereof and the costimulatory signaling domain is a 4-1BB signaling domain or a functional portion or variant thereof.
80. The isolated peptide of embodiment 79, wherein
   the CD28 transmembrane domain comprises the sequence of amino acids set forth in SEQ ID NO:2, 103 or 104 or a functional portion or variant thereof comprising a sequence that exhibits at least 95% sequence identity to SEQ ID NO:2, 103 or 104; and
   the 4-1BB costimulatory signaling domain comprises the sequence of amino acids set forth in SEQ ID NO:3 or a functional portion or variant thereof comprising a sequence that exhibits at least 95% sequence identity to SEQ ID NO:3.
81. The isolated peptide of embodiment 79 or embodiment 80, wherein the first domain and second domain together comprise the sequence of amino acids set forth in SEQ ID NO:5 or a functional portion or variant thereof comprising a sequence of amino acids that exhibits at least 85%, 86%, 87%, 88%, 89%, 90%, 91%, 92%, 93%, 94%, 95%, 96%, 97%, 98% or 99% sequence identity to SEQ ID NO:5.
82. The isolated peptide of any of embodiments 79-81, wherein the first domain and second domain together comprise the sequence of amino acids set forth in SEQ ID NO:5.
83. The isolated peptide of any of embodiments 74-82, wherein:
   the peptide is between 7 and 30 amino acids, 8 and 20 amino acids, 8 and 15 amino acids, 10 and 17 amino acids, 7 and 13 amino acids or 8 and 10 amino acid; or
   the peptide is or is about 10 amino acids, 11 amino acids, 12 amino acids, 13 amino acids, 14 amino acids, 15 amino acids, 16 amino acids or 17 amino acids.
84. The isolated peptide of any of embodiments 74-83, comprising:
   i) a sequence of amino acids set forth in any of SEQ ID NOS: 6, 7, 8-12, 16-22 and 137 or a variant thereof that retains activity to induce tolerance; or
   ii) a sequence of amino acids of a peptide epitope of a sequence of i) that binds to an HLA molecule.
85. The isolated peptide of any of embodiments 74-84, wherein the peptide has a binding affinity for a human leukocyte antigen (HLA) molecule that is less than 1000 nM, less than 500 nM or less than 50 nM.
86. The isolated peptide of embodiment 85, wherein the binding affinity is an IC50.
87. The isolated peptide of embodiment 85 or embodiment 86, wherein the HLA allele is selected from among HLA-A^{∗}02:01, HLA-A^{∗}03:01, HLA-A^{∗}11:01 and HLA-B^{∗}08:01
88. An isolated peptide, consisting of the sequence of amino acids set forth in SEQ ID NO: 6, 7, 8-12, 16-22 or 137 or a peptidomimetic, analog or variant thereof.
89. A composition, comprising the peptide of any of embodiments 74-88 or a plurality of peptides of any of embodiments 74-88.
90. A composition, comprising a plurality of peptides, wherein each of said peptides overlap in sequence and comprise all or a portion of an immunogenic region of a chimeric receptor.
91. The composition of embodiment 89 or embodiment 90, comprising a pharmaceutically acceptable excipient.
92. A combination, comprising:
   a peptide of any of embodiments 74-88 or a plurality of peptides of any of embodiments 74-88; and
   a chimeric receptor.
93. A kit, comprising the composition of any of embodiments 89-91 or combination of embodiment 92 and, optionally, instructions for use.
94. The composition of any of embodiments 89-91, combination of embodiment 92 or kit of embodiment 93, wherein the chimeric receptor is expressed by cells genetically engineered with nucleic acid encoding the chimeric receptor.
95. The composition, combination or kit of any of embodiments 89-94, wherein the chimeric receptor is a chimeric antigen receptor.

In some of any of the above embodiments, the receptor is a chimeric receptor that binds to an antigen other than that associated with the disease or condition, such as in the context of a receptor expressed on a regulatory T cell or an iCAR, e.g., where it is desired to suppress the cell upon binding to the receptor, such as to avoid side effects such as those induced by non-disease targeted binding. Although the receptor itself is inhibitory, the cells' persistence is generally still desired to maximize or increase exposure, such as in the context of a cell also expressing a disease-targeting and activating receptor. Thus, in some embodiments, the peptide(s) that are administered include those having sequence identity or similarity to a non-activating or non-disease/condition-targeting receptor.

In some embodiments of any of the embodiments, the peptide may be a plurality of peptides.

### VIII. Examples

The following examples are included for illustrative purposes only and are not intended to limit the scope of the invention.

### Example 1: Analysis of Transgene Product-Specific Host Immune Responses

Pre- and post-treatment peripheral blood mononuclear cells (PBMC) samples were obtained from four (4) subjects with B cell malignancies treated with autologous T cells expressing a CD19-specific CAR. The CAR included an anti-CD 19 scFv derived from murine antibody, a hinge domain, a CD28 transmembrane domain, a 4-1BB intracellular signaling domain, and a CD3-zeta intracellular signaling domain. The CAR-expressing T cells also expressed a truncated EGFR (EGFRt) as a surrogate marker by transducing cells with a lentiviral vector containing a nucleic acid encoding the CAR and a nucleic acid encoding the EGFRt surrogate marker, separated by a T2A ribosome switch domain.

Pre- and post (day 42)-infusion PBMCs obtained from the subjects were assessed to detect the presence or absence of specific anti-CAR immune responses essentially as described by Berger et al. Blood. 2006 March; 107(6): 2294-2302, Berger et al. J Virol. 2001 January 75(2): 799-808, Riddell et al. Nature Medicine. 1996 February 2(2): 216-223, Berger et al. Blood. 2005 February 105(4): 1640-1647. Briefly, PBMCs (responders) were stimulated *in vitro* with autologous gamma-irradiated cells transduced with the CAR expressed by the administered cells (stimulators at a 1:1 or 2:1 responder-to-stimulator ratio). The cultures then were assessed in a chromium release assay for cytotoxicity against autologous ⁵¹Cr-labeled CAR-transduced ("CD19 CAR") and non-transduced ("Mock") T cells (targets) at various effector-to-target (E/T) ratios. Following co-incubation, release of chromium was quantified and the percentage of maximum achievable lysis in each sample determined.

The results for samples derived from one exemplary patient are shown in Figure 1, which depicts the cytolytic activity of PBMCs pre-infusion and post-infusion at day 42. Whereas no cytolytic activity specific for CAR-transduced target cells was detected in any pre-infusion PBMC-derived cultures, in two of the four subjects assessed, CAR-specific lytic activity was detected in cultures derived from post-infusion PBMC samples. These results indicate that CAR-specific immune responses can develop following a single infusion of CAR-expressing T cells.

Epitope mapping was carried out to assess region(s) of the CAR recognized by the specific immune responses. Pre- and post-infusion PBMC samples were stimulated in the presence of individual pools of multiple 15-mer peptides, with sequences representing overlapping portions (11 amino acid overlap) of the entire length of an approximately 500 amino acid sequence of the CAR expressed by the administered cells. Cells were stained with antibodies to detect CD8 and CD4 surface expression and intracellular expression of cytokine. Twenty-three (23) pools were assessed, each containing ten (10) peptides each and collectively including 125 individual overlapping peptides, with each peptide represented in at least two of the pools.

This design permitted the generation of an analytic grid to assess responses specific for individual peptides, whereby a peptide present in more than one pool detected as hits in this assay was deemed a potentially immunogenic peptide hit. For the two patients in whom a CAR-specific immune response had been detected, six and three peptide hits, respectively, were identified.

Individual ELISpot assays were performed using an anti-cytokine capture antibody to assess the presence or absence of a specific immune response for each of these individual hits (see Berger et al. (2006); Berger et al. (2001); Riddell et al. (1996); and Berger et al. (2005), supra). The results of an exemplary assay for one patient are shown in FIG. 2. Specific immune responses against peptides with sequences within the V_{H} portion of the scFv of the CAR were detected in both patients assessed (including regions within the FR1, CDR1, and FR2 regions for one patient and within the FR3 for the other). For the first patient, specific immune responses also were detected against two overlapping 15-mer peptides, each containing the junction between the transmembrane domain and costimulatory domain of the CAR (labeled "fusion site" in FIG. 2). These two overlapping 15-mer peptides had the amino acid sequences AFIIFWVKRGRKKLL (SEQ ID NO: 8) and FWVKRGRKKLLYIFK (SEQ ID NO: 9. also set forth in SEQ ID NO:165), respectively. In another study following administration with a different CAR having a murine scFv, CD28 transmembrane and costimulatory domains and a CD3 zeta domain, using similar methods, an immune response also was detected for one subject against a pool containing V_{H} portions of an anti-CD19 scFv and for another subject in a pool containing junction portions.

No specific immune responses were detected in the patients by this assay against peptides within other regions. For example, in this assay, no specific responses were detected against peptides having sequences within other CDRs or framework regions of the scFv, peptides within regions of costimulatory or transmembrane domain but not spanning the junction between the two, or peptides within the EGFRt or CD3-ζ region of the CAR. Specific immune responses were not detected against endogenous sequences.

### Example 2: In Silico Analysis of Peptides Derived from Junction Regions of a CAR for Binding to HLA Class I and HLA Class II

T cell epitope prediction tools, available from the Immune Epitope Database and analysis resource (IEDB), were used for *in silico* analysis to predict MHC-binding affinities and other properties related to potential immunogenicity for each of a series of overlapping peptide sequences within a portion of an exemplary CAR sequence. The portion included a spacer having an immunoglobulin-derived hinge domain, a human CD28 transmembrane domain, a human 4-1BB costimulatory domain, and a human CD3zeta signaling domain. In the portion assessed, the hinge domain was a human IgG4 hinge domain, the CD28 transmembrane domain comprised a sequence set forth in SEQ ID NO:2 and the 4-1BB costimulatory domain contained the sequence set forth in SEQ ID NO:3. This portion thus contained three junctions between different domains derived from human sequences (which junctions may have represented sites of potential immunogenicity against a CAR upon administration to a human subject): the junction between the spacer region and transmembrane domain, the junction between the transmembrane domain and costimulatory domain, and the junction between the costimulatory domain and intracellular signaling domain (see Figures 3A and 3B).

To identify portions of the sequence that may have particular properties making them more likely to be presented to T cells, affinities for binding to 27 individual HLA class I alleles and 56 individual HLA class II alleles were predicted for overlapping peptides along the length of the portion, of 8-14 amino acids in length and of 15 amino acids in length (containing 9-mer binding core), respectively. These alleles, collectively representing HLA alleles present in greater than 99% of the worldwide population, and their approximate frequency in the United States population are listed in Tables 1A and 1B.

| **Table 1A: HLA class I** | | |
|---|---|---|
| **Class I** | **allele** | **Frequency in population** |
| 1 | HLA-A^{∗}01:01 | 12.94 |
| 2 | HLA-A^{∗}02:01 | 42.88 |
| 3 | HLA-A^{∗}02:03 | 0.19 |
| 4 | HLA-A^{∗}02:06 | 1.55 |
| 5 | HLA-A^{∗}03:01 | 13.50 |
| 6 | HLA-A^{∗}11:01 | 11.60 |
| 7 | HLA-A^{∗}23:01 | 8.30 |
| 8 | HLA-A^{∗}24:02 | 22.56 |
| 9 | HLA-A^{∗}26:01 | 5.36 |
| 10 | HLA-A^{∗}30:01 | 6.29 |
| 11 | HLA-A^{∗}30:02 | 5.21 |
| 12 | HLA-A^{∗}31:01 | 6.87 |
| 13 | HLA-A^{∗}32:01 | 3.71 |
| 14 | HLA-A^{∗}33:01 | 2.62 |
| 15 | HLA-A^{∗}68:01 | 6.36 |
| 16 | HLA-A^{∗}68:02 | 4.79 |
| 17 | HLA-B^{∗}07:02 | 12.96 |
| 18 | HLA-B^{∗}08:01 | 9.23 |
| 19 | HLA-B^{∗}15:01 | 6.54 |
| 20 | HLA-B^{∗}35:01 | 13.03 |
| 21 | HLA-B^{∗}40:01 | 9.79 |
| 22 | HLA-B^{∗}44:02 | 7.22 |
| 23 | HLA-B^{∗}44:03 | 8.96 |
| 24 | HLA-B^{∗}51:01 | 8.51 |
| 25 | HLA-B^{∗}53:01 | 7.26 |
| 26 | HLA-B^{∗}57:01 | 3.49 |
| 27 | HLA-B^{∗}58:01 | 4.82 |

| **Table 1B: HLA class II** | | | | | |
|---|---|---|---|---|---|
| **Class II** | **allele** | **Frequency in population** | **Class II** | **allele** | **frequency in population** |
| 1 | HLA-DRB1^{∗}01:01 | 13.62 | 29 | HLA-DQA1^{∗}01:02/DQB1^{∗}05:02 | 21.13 |
| 2 | HLA-DRB1^{∗}15:01 | 22.86 | 30 | HLA-DQA1^{∗}01-02/DQB1^{∗}06-02 | 30.74 |
| 3 | HLA-DRB1^{∗}03:01 | 21.82 | 31 | HLA-DQA1^{∗}03:01/DQB1^{∗}03:02 | 31.56 |
| 4 | HLA-DRB1^{∗}04:01 | 15.54 | 32 | HLA-DQA1^{∗}01:02/DQB1^{∗}06:04 | 19.00 |
| 5 | HLA-DRB1^{∗}11:01 | 10.92 | 33 | HLA-DQA1^{∗}05:01/DQB1^{∗}03:01 | 80.58 |
| 6 | HLA-DRB1^{∗}13:01 | 9.86 | 34 | HLA-DQA1^{∗}02:01/DQB1^{∗}02:02 | 27.99 |
| 7 | HLA-DRB1^{∗}07:01 | 19.84 | 35 | HLA-DQA1^{∗}03:01/DQB1^{∗}03:01 | 49.92 |
| 8 | HLA-DRB1^{∗}01:01 | 4.06 | 36 | HLA-DQA1^{∗}02:01/DQB1^{∗}03:03 | 23.32 |
| 9 | HLA-DRB1^{∗}01:02 | 1.85 | 37 | HLA-DQA1^{∗}03:03/DQB1^{∗}03:03 | 20.22 |
| 10 | HLA-DRB1^{∗}04:02 | 6.28 | 38 | HLA-DPA1^{∗}01:03/DPB1^{∗}01:01 | 99.83 |
| 11 | HLA-DRB1^{∗}04:05 | 1.22 | 39 | HLA-DPA1^{∗}01:03/DPB1^{∗}02:01 | 99.83 |
| 12 | HLA-DRB1^{∗}04:07 | 2.78 | 40 | HLA-DPA1^{∗}01:03/DPB1^{∗}03:01 | 99.82 |
| 13 | HLA-DRB1^{∗}04:08 | 1.26 | 41 | HLA-DPA1^{∗}01:03/DPB1^{∗}04:01 | 99.88 |
| 14 | HLA-DRB1^{∗}08:04 | 0.86 | 42 | HLA-DPA1^{∗}01:03/DPB1^{∗}04:02 | 99.86 |
| 15 | HLA-DRB1^{∗}09:01 | 5.33 | 43 | HLA-DPA1^{∗}01:03/DPB1^{∗}05:01 | 99.81 |
| 16 | HLA-DRB1^{∗}10:01 | 2.78 | 44 | HLA-DPA1^{∗}02:01/DPB1^{∗}01:01 | 23.54 |
| 17 | HLA-DRB1^{∗}11:02 | 0.94 | 45 | HLA-DPA1^{∗}02:01/DPB1^{∗}02:01 | 24.11 |
| 18 | HLA-DRB1^{∗}11:03 | 0.74 | 46 | HLA-DPA1^{∗}02:01/DPB1^{∗}03:01 | 17.63 |
| 19 | HLA-DRB1^{∗}11:04 | 4.76 | 47 | HLA-DPA1^{∗}02:01/DPB1^{∗}04:01 | 46.73 |
| 20 | HLA-DRB1^{∗}15:02 | 0.78 | 48 | HLA-DPA1^{∗}02:01/DPB1^{∗}04:02 | 38.04 |
| 21 | HLA-DRB1^{∗}15:03 | 1.22 | 49 | HLA-DPA1^{∗}02:01/DPB1^{∗}05:01 | 13.24 |
| 22 | HLA-DRB1^{∗}16:01 | 4.06 | 50 | HLA-DPA1^{∗}02:01/DPB1^{∗}06:01 | 8.59 |
| 23 | HLA-DRB1^{∗}16:02 | 0.84 | 51 | HLA-DPA1^{∗}02:01/DPB1^{∗}09:01 | 7.26 |
| 24 | HLA-DRB3^{∗}02:02 | 0.00 | 52 | HLA-DPA1^{∗}02:01/DPB1^{∗}11:01 | 9.98 |
| 25 | HLA-DRB3^{∗}03:01 | 0.00 | 53 | HLA-DPA1^{∗}02:01/DPB1^{∗}13:01 | 11.55 |
| 26 | HLA-DRB5^{∗}01:01 | 0.00 | 54 | HLA-DPA1^{∗}02:01/DPB1^{∗}14:01 | 7.98 |
| 27 | HLA-DQA1^{∗}01:01/DQB1^{∗} 05:01 | 30.57 | 55 | HLA-DPA1^{∗}02:01/DPB1^{∗}15:01 | 7.73 |
| 28 | HLA-DQA1^{∗}05:01/DQB1^{∗} 02:01 | 76.17 | 56 | HLA-DPA1^{∗}02:01/DPB1^{∗}17:01 | 10.40 |

Algorithm-based T cell epitope prediction tools available from the IEDB were used to predict IC50 values for binding to HLA class I molecules for each 8-14 amino acid peptide in the dataset using ANN (Nielsen et al. (2003) Protein Sci., 12:1007-1017 and Lundegaard et al. (2008) NAR, 36:W509-512) and, in some cases, one or more additional prediction usingSMM (Peters and Sette (2005) BMC Bioinformatics, 6:132) and comblib (Sidney et al. (2008) Immunome Res. 4:2, or the Consensus tool (see Kim, et al. (2012) Immune epitope database analysis resource, NAR (combining predictions from any of the foregoing). Predictions for IC50 values for binding to HLA class II for each 15 amino acid peptide in the dataset was made using the NetMHCIIpan method (Karosiene et al. (2013) Immunogenetics 65(10):711; Nielsen et al. (2008) PLoS Comput Biol.4(7)e1000107). For each individual position within the portion of the CAR amino acid sequence, the total number of sequences in the dataset that included the position and was predicted to bind to any of the class I or class II alleles with a predicted IC50 of less than 50 nm was determined. FIGs 4A (HLA class I) and 4B (HLA class II), depict the results for class I and class II alleles, respectively, showing positional coverage along the length of the sequence, based on the determined total number, weighted according to the frequency of the individual HLA alleles in the population. The area under the curve (AUC) across the entire assessed region was approximately 1321 for HLA class I binding and 2943 for HLA class II binding. The AUC for the transmembrane-costimulatory domain region was approximately 931 for HLA class I binding and 2212 for HLA class II binding.

As shown in FIGs. 4A and 4B, certain portions of the sequence were predicted by this method to contain fragments more likely to bind well in MHC complexes and thus be presented as epitopes for potential recognition by T cells. Binding affinity for HLA alleles alone does not necessarily predict immunogenicity. Given that the individual domains (e.g., transmembrane, costimulatory) in this exemplary CAR were human-derived, upon administration to a human subject, immunogenic responses were less likely to develop against an epitope within any one of these individual regions alone (as opposed to an epitope spanning multiple regions not ordinarily associated with one another, and/or including a junction between such regions). For example, even for a peptide predicted to bind well to and be presented in the context of an MHC molecule, if the peptide was derived entirely from an endogenous protein, it may be recognized as "self' and thus may fail to induce a productive immune response. For example, whereas certain regions entirely within a single transmembrane or cytoplasmic domain scored highly on the HLA-binding affinity prediction, in the results described in Example 1, no immune responses were detected against peptide sequences solely within either one of these domains of a similar CAR sequence. Accordingly, while various "hot spots" were observed with respect to predicted HLA-binding affinity, subsequent assessment and alteration focused on those areas that not only had higher predicted IC50 values, but also included potential epitopes that spanned the junction between different domains derived from two different proteins.

In particular, a junction region that includes one or more potential peptide epitopes spanning the junction of the CD28 transmembrane domain and 4-1BB signaling domain of the exemplary CAR was further assessed. With respect to the sequence set forth in SEQ ID NO:5, which includes the exemplary human CD28 transmembrane domain (SEQ ID NO:2) and exemplary human 4-1BB costimulatory domain (SEQ ID NO:3), the assessed junction region contained 13 amino acids on either side of the junction spanning the CD28 transmembrane and 4-1BB costimulatory domains as follows:
FWVLVVVGGVLACY**SLLVTVAFIIFWVKRGRKKLLYIFKQ**PFMRPVQTTQEEDGC SC RFPEEEEGGCEL (SEQ ID NO:5), in which a 26 amino acid junction region is indicated by bold, and the two amino acids just C' and N' of the junction between the domains is indicated by underline. The assessed 26 amino acid junction region is set forth in SEQ ID NO: 137 and corresponds to amino acid residues 15 to 40 of the sequence of amino acids set forth in SEQ ID NO:5.

I*n silico* modeling was carried out to identify one or more amino acid modifications (mutations) within the 26-amino acid junction region set forth in SEQ ID NO: 137 resulting in peptide fragments that were predicted to bind with high IC50 values to class I and class II alleles, and thus that were likely to reduce the potential for inducing immunogenicity against a CAR containing this region. Specifically, predictions were made for variant peptide fragments of the junction region containing one or more mutations at amino acid residue positions corresponding to positions 14, 17 and 20 with numbering with reference to SEQ ID NO:137 (which correspond to one or more mutations at amino acid positions corresponding to positions 28, 31 and 34 with numbering with reference to SEQ ID NO:5). In this exemplary study, these residues were chosen for further analysis following *in silico* mutagenesis and binding predictions of all high affinity epitopes in which all possible single amino acid replacements across that epitope were surveyed for their impact on the predicted IC50 values. Residues that resulted in greater IC50 predictions (decrease in the binding) were identified, which identified the above residues as being sensitive to replacements.

A series of different variant junction regions were assessed, each containing one or more amino acid replacement at the assessed position(s), as compared to the non-mutated junction region within the exemplary CAR sequence. An exemplary subset of amino acid replacements at the identified positions were chosen that may be less disruptive to the structure or function of either the transmembrane region of the costimulatory signaling domain. Also, replacements were chosen that may be able to impact more than one epitope at a time, since the epitopes overlap. Specifically, individual variant junction regions contained the following modifications (amino acid replacements): K28A, K28H, K28L, K28Q, K28S, R31A, R31H, R31L, R31N, R31S, L34A, L34S, K28Q/R31A, K28Q/R31N, K28Q/R31S, K28Q/L34A, K28Q/L34S, R31N/L34A, R31N/L34S, K28Q/R31N/L34A, K28Q/R31N/L34S, with numbering with reference to SEQ ID NO:5.

For the non-variant and variant junction regions, weighted immunogenicity scores were obtained for class I and class II alleles, using the T cell epitope prediction tools available from IEDB. Scores were derived using predicted IC50 values for each of a series of 8-mer to 14-mer overlapping peptides (for each of the 27 HLA class I alleles, individually) and a series of 15-mer overlapping peptides (for each of the 56 HLA class II alleles, individually) within the respective (variant or non-variant) 26-amino acid junction region, and were weighted based on relative frequency in the population of the individual HLA class I and class II alleles. A higher relative score is indicative of a higher degree of predicted binding.

The results are set forth in FIG. 5. The results demonstrate the ability to decrease in the overall predicted HLA class I immunogenicity score within a CAR junction region by modifying amino acids within the region. The results also confirm the ability to reduce predicted HLA class I binding affinity (and hence reduced predicted immunogenicity score) without resulting in a substantial increase in the predicted immunogenicity score for HLA class II binding. Thus, in general, the results showed that amino acid modification(s) within a region spanning a junction between a CD28 transmembrane domain and a 4-1BB costimulatory domain of a CAR could be made and effect an overall reduction in the predicted affinity for human HLA binding, which would be consistent with a reduction in potential for immunogenicity, upon administration to a human subject, of a chimeric receptor identical to a receptor having this region, but containing the modification or combination of modifications in this region.

### Example 3: Comparison of In Silico Analysis and In Vitro Binding of Peptides Derived from Junction Regions of a CAR for Binding to HLA Class I

Actual binding affinities for certain HLA class I alleles (A^{∗}02:01, A^{∗}03:01, A^{∗}11:01, and B^{∗}08:01) were assessed *in vitro* for exemplary overlapping 9 amino acid peptide sequences within a portion of the 26 amino acid junction region spanning the CD28 and 4-1BB junction. Specifically, assessment was of a series of overlapping 9-mer peptides derived from the sequence VAFIIFWVKRGRKKLL (set forth in SEQ ID NO: 7), which contains a portion of the CD28 transmembrane domain and 4-1BB costimulatory domain spanning the junction between the domains (bond joining the two amino acids noted in underline). In addition, a series of overlapping 9-mer peptides of each of a number of different variants of this portion also were assessed, each variant containing a mutation or mutations in this region as described in Example 2.

The various 9-mer overlapping peptides were synthesized and their purity tested by MALDI-TOF Mass Spectrometry. The synthetic peptides were then incubated with recombinant MHC molecules to assess binding properties using the REVEAL Epitope Discovery System, which is a high-throughput binding assay that measures the degree to which each peptide is able to stabilize a ternary MHC-peptide complex (ProImmune, Oxford, United Kingdom). Each peptide was separately tested for this ability with respect to each of the HLA class I alleles, normalized to the degree observed for a positive control (known T cell epitope for the relevant allele). The results are reported as a score, in which the binding was normalized to the positive control peptide set at 100%. In this analysis, a score of greater than 50 generally was considered to represent good or high affinity binding.

The results were compared to predicted binding (IC50) values obtained for binding of the same peptide:MHC complex, using the *in silico* prediction methods as described in Example 2. Since the maximum IC50 value predicted was about 50,000, the IC50 values were log transformed, subtracted from LOG(50000) and divided by LOG(50000) to obtain a normalized *in silico* score ((Log(50000) - logIC50) / Log(50000)). In this analysis, an *in silico* binding prediction score of greater than 2.0 generally was considered to represent predicted good or high affinity binding.

The results are set forth in Table 2A (HLA-A^{∗}02:01 and HLA-A^{∗}03:01) and Table 2B (HLA-A^{∗}11:01 and HLA-B^{∗}08:01). In general, the *in silico* binding predictions were predictive of the actual *in vitro* binding results. In some cases, a relatively higher binding was predicted *in* silico, but not observed in the *in vitro* assay.

The results also were consistent with predicted binding affinity being generally predictive of affinity as measured in the *in vitro* assay. Additionally, the results demonstrated successful reduction of binding affinity to an HLA by modifications within a junction region, and that it was possible to modify the sequence in a way that resulted in a lower predicted or actual binding affinity or score of one of the overlapping potential epitopes, without increasing (or while also reducing) binding affinity or score for another of the overlapping epitopes containing the same residue. In some embodiments, such mutations or modifications may be particularly advantageous. As a non-limiting example of the results, modifications K28L, R31H, L34S and/or L34A, with reference to numbering set forth in SEQ ID NO:5, generally resulted in a reduced predicted or actual binding affinity or score for at least one HLA allele and/or for at least one peptide within the region assessed, without resulting in a higher binding affinity to another HLA allele and/or without resulting in a higher binding affinity for another peptide.

| **Table 2A: In Silico and In Vitro MHC binding of Variant Peptides** | | | | | | | | | |
|---|---|---|---|---|---|---|---|---|---|
| | | | A^{∗}02:01 | | | A^{∗}03:01 | | | |
| Overlapping Referen ce Peptide | Peptide Sequence | Mutati on | IEDB IC50 | In Silico Score | In Vitro Score | IEDB IC50 | In Silico | In Vitro | SEQ ID NO. |
| 1 | V AFIIFWVK | none | 19617 | 0.41 | 0.70 | 262 | 2.28 | 1.50 | 16 |
| 2 | AFIIFWVKR | none | 25314 | 0.30 | 0.50 | 17846 | 0.45 | 5.70 | 17 |
| 3 | FIIFWVKRG | none | 7967 | 0.80 | 10.10 | 23693 | 0.32 | 1.40 | 18 |
| 4 | IIFWVKRGR | none | 24769 | 0.31 | 1.70 | 406 | 2.09 | 24.30 | 19 |
| 5 | IFWVKRGRK | none | 30463 | 0.22 | 4.50 | 3482 | 1.16 | 20.50 | 20 |
| 6 | FWVKRGRKK | none | 28878 | 0.24 | 3.50 | 17327 | 0.46 | 2.20 | 21 |
| 7 | WVKRGRKKL | none | 27956 | 0.25 | 1.40 | 22961 | 0.34 | 13.10 | 22 |
| 1 | VAFIIFWV**S** | K28S | 12273 | 0.61 | 70.60 | 22660 | 0.34 | 0.70 | 23 |
| 2 | AFIIFWV**S**R | K28S | 23924 | 0.32 | 4.50 | 18157 | 0.44 | 0.30 | 24 |
| 3 | FIIFWV**S**RG | K28S | 3382 | 1.17 | 0.20 | 21751 | 0.36 | 0.10 | 25 |
| 4 | IIFWV**S**RGR | K28S | 21442 | 0.37 | 2.60 | 155 | 2.51 | 25.30 | 26 |
| 5 | IFWV**S**RGRK | K28S | 30615 | 0.21 | 1.30 | 1880 | 1.42 | 39.20 | 27 |
| 6 | FWV**S**RGRKK | K28S | 28679 | 0.24 | 1.50 | 17832 | 0.45 | 2.90 | 28 |
| 7 | WV**S**RGRKKL | K28S | 23551 | 0.33 | 2.30 | 22394 | 0.35 | 2.10 | 29 |
| 1 | VAFIIFWV**L** | K28L | 1336 | 1.57 | 0.20 | 20145 | 0.39 | 0.00 | 30 |
| 2 | AFIIFWV**L**R | K28L | 22444 | 0.35 | 5.70 | 15583 | 0.51 | 0.30 | 31 |
| 3 | FIIFWV**L**RG | K28L | 2037 | 1.39 | 7.20 | 20853 | 0.38 | 0.10 | 32 |
| 4 | IIFWV**L**RGR | K28L | 17613 | 0.45 | 8.40 | 238 | 2.32 | 2.80 | 33 |
| 5 | IFWV**L**RGRK | K28L | 30293 | 0.22 | 2.90 | 3675 | 1.13 | 19.50 | 34 |
| 6 | FWV**L**RGRKK | K28L | 28857 | 0.24 | 3.30 | 16996 | 0.47 | 0.40 | 35 |
| 7 | WV**L**RGRKKL | K28L | 19522 | 0.41 | 2.40 | 23063 | 0.34 | 0.70 | 36 |
| 1 | VAFIIFWV**H** | K28H | 23252 | 0.33 | 3.10 | 10359 | 0.68 | 0.30 | 37 |
| 2 | AFIIFWV**H**R | K28H | 22819 | 0.34 | 0.30 | 18506 | 0.43 | 0.30 | 38 |
| 3 | FIIFWV**H**RG | K28H | 1691 | 1.47 | 37.30 | 22930 | 0.34 | 0.00 | 39 |
| 4 | IIFWV**H**RGR | K28H | 23573 | 0.33 | 4.70 | 326 | 2.19 | 30.10 | 40 |
| 5 | IFWV**H**RGRK | K28H | 29930 | 0.22 | 7.10 | 1062 | 1.67 | 37.80 | 41 |
| 6 | FWV**H**RGRKK | K28H | 28189 | 0.25 | 1.60 | 17052 | 0.47 | 3.60 | 42 |
| 7 | WV**H**RGRKKL | K28H | 25035 | 0.30 | 1.60 | 22278 | 0.35 | 3.40 | 43 |
| 1 | VAFIIFWV**A** | K28A | 2733 | 1.26 | 28.50 | 21010 | 0.38 | 0.00 | 44 |
| 2 | AFIIFWV**A**R | K28A | 23072 | 0.34 | 2.20 | 17755 | 0.45 | 0.50 | 45 |
| 3 | FIIFWV**A**RG | K28A | 1902 | 1.42 | | 22486 | 0.35 | | 102 |
| 4 | IIFWV**A**RGR | K28A | 22077 | 0.36 | 9.60 | 206 | 2.39 | 18.60 | 46 |
| 5 | IFWV**A**RGRK | K28A | 30251 | 0.22 | 1.00 | 3893 | 1.11 | 53.70 | 47 |
| 6 | FWV**A**RGRKK | K28A | 28353 | 0.25 | 13.50 | 16210 | 0.49 | 5.00 | 48 |
| 7 | WV**A**RGRKKL | K28A | 22630 | 0.34 | 8.80 | 22548 | 0.35 | 1.30 | 49 |
| 1 | VAFIIFWV**Q** | K28Q | 17921 | 0.45 | 5.30 | 22927 | 0.34 | 0.20 | 50 |
| 2 | AFIIFWV**Q**R | K28Q | 24165 | 0.32 | 0.40 | 19279 | 0.41 | 0.20 | 51 |
| 3 | FIIFWV**Q**RG | K28Q | 4152 | 1.08 | 16.80 | 23136 | 0.33 | 0.40 | 52 |
| 4 | IIFWV**Q**RGR | K28Q | 21783 | 0.36 | 5.80 | 231 | 2.34 | 18.00 | 53 |
| 5 | IFWV**Q**RGRK | K28Q | 30704 | 0.21 | 6.10 | 3177 | 1.20 | 36.70 | 54 |
| 6 | FWV**Q**RGRKK | K28Q | 29501 | 0.23 | 5.20 | 18619 | 0.43 | 6.40 | 55 |
| 7 | WV**Q**RGRKKL | K28Q | 24480 | 0.31 | 8.10 | 23630 | 0.33 | 4.90 | 56 |
| 4 | IIFWVKRG**S** | R31S | 18902 | 0.42 | 13.60 | 19450 | 0.41 | 16.20 | 57 |
| 5 | IFWVKRG**S**K | R31S | 29391 | 0.23 | 2.40 | 3348 | 1.17 | 10.40 | 58 |
| 6 | FWVKRG**S**KK | R31S | 28317 | 0.25 | 1.90 | 14227 | 0.55 | 5.90 | 59 |
| 7 | WVKRG**S**KKL | R31S | 23485 | 0.33 | 3.00 | 22637 | 0.34 | 1.90 | 60 |
| 4 | IIFWVKRG**L** | R31L | 2692 | 1.27 | 82.20 | 16661 | 0.48 | 15.90 | 61 |
| 5 | IFWVKRG**L**K | R31L | 29250 | 0.23 | 2.40 | 1973 | 1.40 | 26.90 | 62 |
| 6 | FWVKRG**L**KK | R31L | 27554 | 0.26 | 9.40 | 14434 | 0.54 | 2.70 | 63 |
| 7 | WVKRG**L**KKL | R31L | 20709 | 0.38 | 24.50 | 22985 | 0.34 | 10.00 | 64 |
| 4 | IIFWVKRG**H** | R31H | 27453 | 0.26 | 5.30 | 1665 | 1.48 | 26.60 | 65 |
| 5 | IFWVKRG**H**K | R31H | 29806 | 0.22 | 2.20 | 3806 | 1.12 | 9.00 | 66 |
| 6 | FWVKRG**H**KK | R31H | 27689 | 0.26 | 2.60 | 16743 | 0.48 | 4.10 | 67 |
| 7 | WVKRG**H**KKL | R31H | 25923 | 0.29 | 3.90 | 22313 | 0.35 | 1.40 | 68 |
| 4 | IIFWVKRG**A** | R31A | 6107 | 0.91 | 85.90 | 16069 | 0.49 | 42.90 | 69 |
| 5 | IFWVKRG**A**K | R31A | 29354 | 0.23 | 13.80 | 3470 | 1.16 | 22.00 | 70 |
| 6 | FWVKRG**A**KK | R31A | 28151 | 0.25 | 4.30 | 17066 | 0.47 | 4.70 | 71 |
| 7 | WVKRG**A**KKL | R31A | 24746 | 0.31 | 4.70 | 22982 | 0.34 | 4.10 | 72 |
| 4 | IIFWVKRG**N** | R31N | 25979 | 0.28 | 9.30 | 18552 | 0.43 | 4.30 | 73 |
| 5 | IFWVKRG**N**K | R31N | 29978 | 0.22 | 3.50 | 2669 | 1.27 | 8.00 | 74 |
| 6 | FWVKRG**N**KK | R31N | 28430 | 0.25 | 8.70 | 17713 | 0.45 | 3.60 | 75 |
| 7 | WVKRG**N**KKL | R31N | 24790 | 0.30 | 2.40 | 22813 | 0.34 | 0.90 | 76 |
| 4 | IIFWV**Q**RG**S** | K28Q/ R31S | 14326 | 0.54 | 22.40 | 17741 | 0.45 | 4.50 | 77 |
| 5 | IFWV**Q**RG**S**K | K28Q/ R31S | 29540 | 0.23 | 33.50 | 3052 | 1.21 | 20.30 | 78 |
| 6 | FWV**Q**RG**S**KK | K28Q/ R31S | 28907 | 0.24 | 0.80 | 15992 | 0.50 | 1.90 | 79 |
| 7 | WV**Q**RG**S**KKL | K28Q/ R31S | 18121 | 0.44 | 4.40 | 23279 | 0.33 | 1.20 | 80 |
| 4 | IIFWV**Q**RG**A** | K28Q/ R31A | 2658 | 1.27 | 95.30 | 13467 | 0.57 | 2.70 | 81 |
| 5 | IFWV**Q**RG**A**K | K28Q/ R31A | 29482 | 0.23 | 11.70 | 3205 | 1.19 | 17.00 | 82 |
| 6 | FWV**Q**RG**A**KK | K28Q/ R31A | 28792 | 0.24 | 2.20 | 18649 | 0.43 | 2.70 | 83 |
| 7 | WV**Q**RG**A**KKL | K28Q/ R31A | 20217 | 0.39 | 3.00 | 23651 | 0.33 | 2.00 | 84 |
| 4 | IIFWV**Q**RG**N** | K28Q/ R31N | 23103 | 0.34 | 13.00 | 16590 | 0.48 | 3.60 | 85 |
| 5 | IFWV**Q**RG**N**K | K28Q/ R31N | 30164 | 0.22 | 16.10 | 2438 | 1.31 | 23.20 | 86 |
| 6 | FWV**Q**RG**N**KK | K28Q/ R31N | 29113 | 0.23 | 3.10 | 19165 | 0.42 | 1.30 | 87 |
| 7 | WV**Q**RG**N**KKL | K28Q/ R31N | 19790 | 0.40 | 4.30 | 23457 | 0.33 | 1.50 | 88 |
| 7 | WVKRGRKK**S** | L34S | 30812 | 0.21 | 3.90 | 25365 | 0.29 | 0.90 | 89 |
| 7 | WVKRGRKK**A** | L34A | 28556 | 0.24 | 4.50 | 24086 | 0.32 | 0.90 | 90 |
| 7 | WV**Q**RGNKK**S** | K28Q/ L34S | 26883 | 0.27 | 1.20 | 25680 | 0.29 | 0.70 | 91 |
| 7 | WV**Q**RGNKK**A** | K28Q/ L34A | 21998 | 0.36 | 1.90 | 24564 | 0.31 | 1.20 | 92 |
| 1 | VAFIIFWV**R** | K28R | 20045 | 0.40 | 1.20 | 5746 | 0.94 | 0.70 | 100 |
| 2 | AFIIFWV**R**R | K28R | 25059 | 0.30 | 1.40 | 17924 | 1.00 | | 101 |

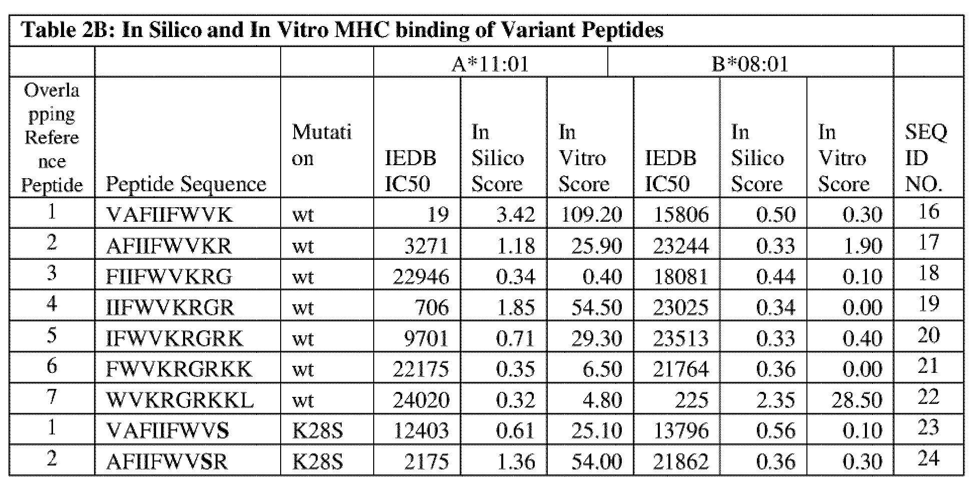

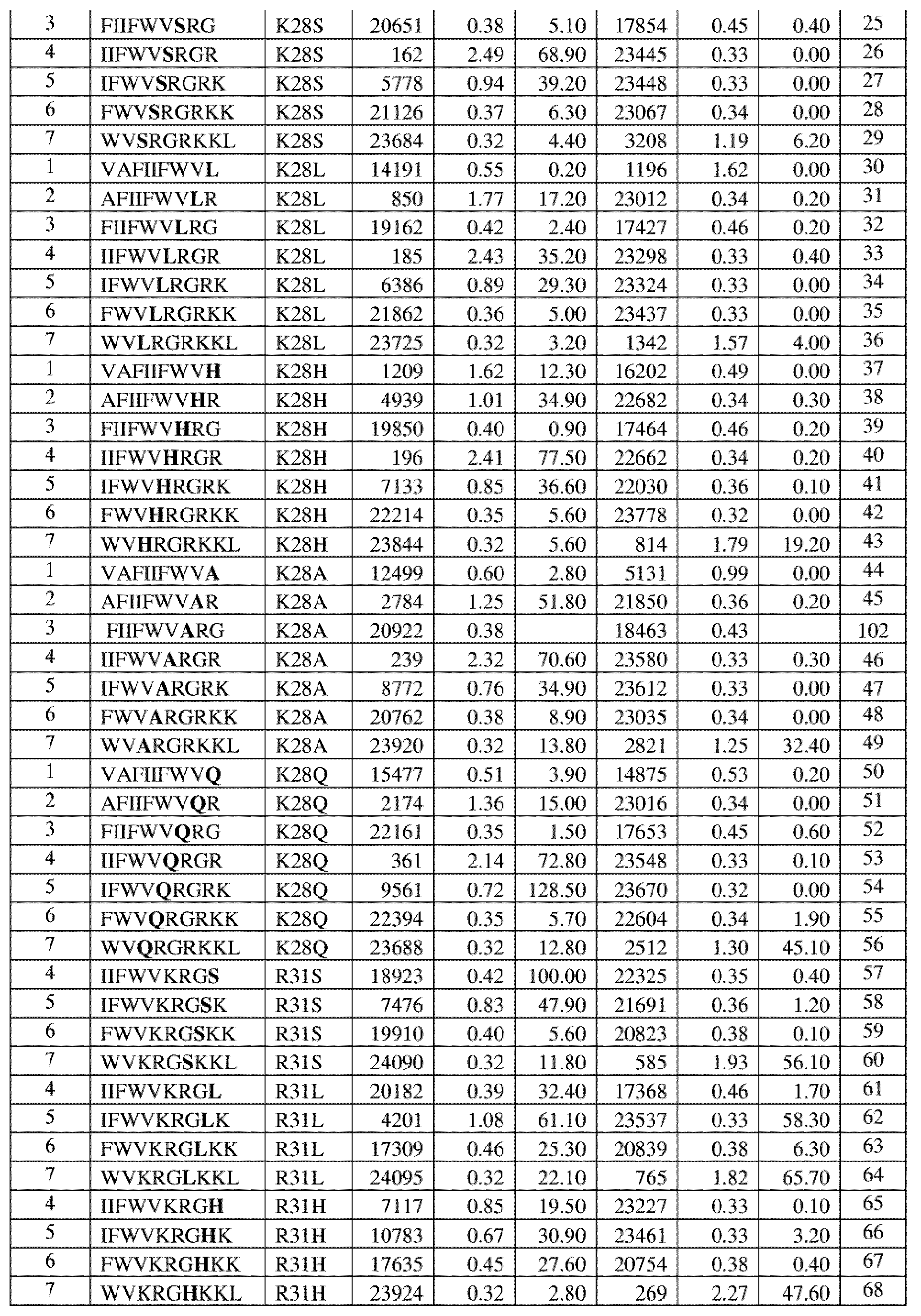

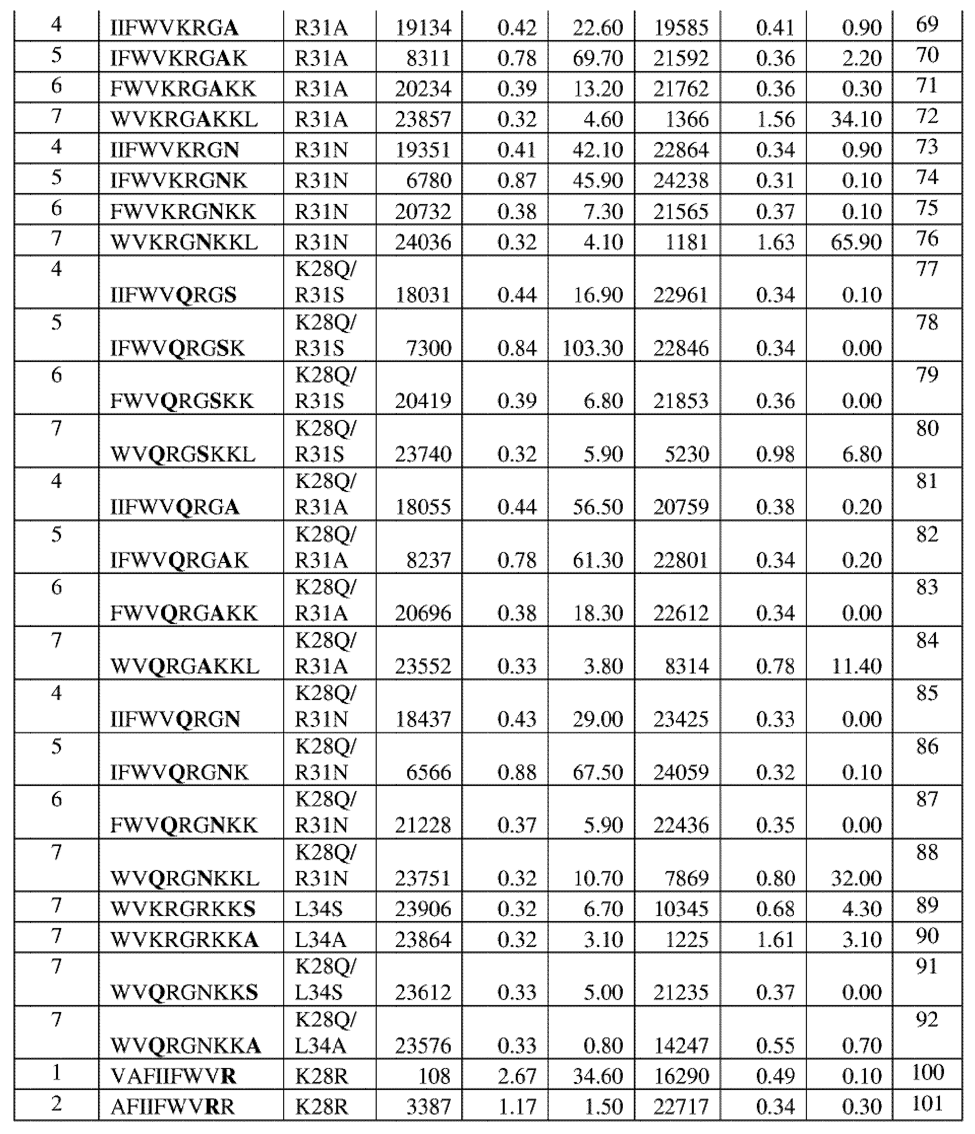

### Example 4: Analysis of Peptides Derived from Junction Region of a CAR for Binding to HLA-A2:01

In order to identify CAR-derived peptides potentially capable of inducing immunogenic responses, a series of overlapping peptides within the non-variant (reference) sequence containing the junction between the CD28 transmembrane domain and 4-1BB costimulatory domain of a CAR were assessed *in silico.* Algorithms were used to predict binding affinities for the peptide groove of a common human MHC class I molecule (HLA-A2:01) using *in silico* analysis to predict affinity for binding. As set forth in FIG. 3, the assessed portion of the CAR had the sequence
CYSLLVTVAFIIFWVKRGRKKLLYIFKQPF (set forth in SEQ ID NO: 6), which contains a portion of the of the CD28 transmembrane domain (set forth in SEQ ID NO:2) and a portion of the 4-1BB costimulatory domain (set forth in SEQ ID NO:3), with the residues spanning the junction of the domains shown by underline. Predicted HLA-A2:01 binding affinity was assessed *in silico* for a series of 140 overlapping peptides of 8-14 amino acids of the sequence set forth in SEQ ID NO:6. Thirty-five (35) of the peptides contained only sequence from the transmembrane domain portion; 35 of the peptides contained only from the costimulatory domain portion, and 70 of the peptides had a junction or fusion region sequence, containing amino acid residues bridging the junction between the domains. For this assessment, peptide fragments predicted to bind to HLA-A2:01 with a dissociation constant of 0 nM to 50 nM were considered predicted to bind with high affinity. Peptide fragments predicted to bind with a dissociation constant of 51 nM to 1000 nM were considered predicted to bind with low affinity. Peptide fragments predicted to bind with a predicted affinity of 1000 nM to 5000 nM were considered predicted to bind with rare affinity. The results are presented in FIG. 3.

As shown in FIG. 3, two of the peptides derived from the reference sequence in this region, each containing a sequence with an overlapping region spanning the junction between the domains were predicted to exhibit low binding affinity for HLA-A2:01. Specifically, a 14-mer peptide having the sequence FIIFWVKRGRKKLL (SEQ ID NO: 10), was predicted to bind with a dissociation constant of 294 nM, and a 13-mer peptide having the sequence of FIIFWVKRGRKKL (SEQ ID NO: 11) was predicted to bind with a dissociation constant of 618 nM. These peptides each included a portion of the 15-mer peptide set forth in SEQ ID NO:8 and identified in Example 1. Shorter 8-mer to 12-mer peptides within this sequence were not predicted to exhibit binding to HLA-A2:01. Another 13-mer peptide containing the amino acid sequence IIFWVKRGRKKLL (SEQ ID NO: 12) was predicted to have a rare binding affinity with a predicted dissociation constant of approximately 3000 nM. None of the remaining fragments that bridged the junction between the two domains were predicted by this assay to exhibit binding affinity for HLA-A2:01 (all had a predicted dissociation constant of far greater than 5000 nM. and in most cases higher than 14,000 nm or 20,000 nM or greater). In each of the peptides predicted to bind to HLAA2:01, neither of the two junction-spanning residues (VK) themselves was predicted to be an anchor residue; rather, such peptides contained these residues in non-flanking positions.

Approximately 15 of the peptides containing sequence derived only from the transmembrane domain were predicted to have a dissociation constant for HLA-A2:01 of less than 5000 nM. Two peptides containing sequence only from the co-stimulatory domain were predicted to have a dissociation constant for HLA-A2:01 binding of less than 5000 nM. The costimulatory domain and transmembrane domain in the assessed sequence are derived from endogenous human sequences, which generally are less likely to be immunogenic to a human subject. For example, in the study described in Example 1, no immune responses were detected that were specific for peptide sequences solely within either one of these domains of the CAR. Accordingly, variants of peptides containing sequence spanning the junction region were assessed.

To generate variant peptides predicted to have reduced binding affinities to HLA-A2:01 and/or reduced immunogenicity in a human subject having this HLA allele, a variant sequence was generated *in silico,* containing mutations in the junction region as compared to the sequence set forth in SEQ ID NO:6. Given that peptides containing the junction-spanning "VK" residues (at non-anchor positions) were predicted to exhibit high binding affinities for HLA-A2:01, two asparagine residues were inserted in the junction between the CD28 transmembrane and 4-1BB co-stimulatory domains. The variant contained the sequence CYSLLVTVAFIIFWV**NN**KRGRKKLLYIFKQPF (set forth in SEQ ID NO: 13, the sequence flanking the junction that was generated by insertion of the asparagine residues is shown in underline). The exemplary variant sequence of SEQ ID NO: 13 was assessed by the same predictive methods. To assess predicted binding affinities for this variant sequence, a series of 154 overlapping fragments of 8-14 amino acids of the sequence set forth in SEQ ID NO: 13 were assessed by *in silico* analysis as described above, whereby 35 peptides had a sequence only in the transmembrane portion, 35 peptides had a sequence only in the costimulatory domain portion and 84 peptides contained a junction region sequence containing amino acids bridging the domains, including one or both of the inserted asparagine residues.

The results are depicted in FIG. 3. As shown, overall, the HLA-A2:01 binding affinities of overlapping peptides within the variant region containing the junction, collectively, were substantially reduced as compared to the non-variant sequence. In particular, the predicted dissociation constant for binding to HLA-A2:01 of peptides in the portion of the junction region previously predicted to be immunogenic was substantially reduced. For example, peptide variants IIFWVNNKRGRKKL (SEQ ID NO: 14) and IIFWVNNKRGRKK (SEQ ID NO: 15), which included asparagine residues in the altered region flanking the junction compared to peptides identified as set forth in SEQ ID NOS:10 and 11, respectively, were predicted to exhibit no detectable binding affinity to HLA-A2:01. Two 14-mer peptides, FIIFWVNNKRGRKK (SEQ ID NO:96) and IFWVNNKRGRKKLL (SEQ ID NO:97), were predicted to exhibit a dissociation constant for binding to this HLA indicating a rare binding affinity, within the range of 1000 nM to 5000 nM. All other peptides containing the modified junction region sequence were predicted to exhibit a dissociation constant of greater than 5000 nM, and in most cases higher than 14,000 nM or 20,000 nM or greater, and thus were not predicted to exhibit binding affinity for HLA-A2:01 by this assessment. Additionally, the modification of the junction region sequence did not create any new peptides predicted to have higher binding affinities for HLA-A2:01 within the costimulatory or transmembrane domain regions.

### Example 5: Administration of anti-CD22 CAR-Expressing Cells to Subjects Previously Treated with anti-CD19 CAR

Six subjects with relapsed/refractory CD22⁺ B cell acute lymphoblastic leukemia (ALL) were administered autologous T cells expressing an anti-CD22 chimeric antigen receptor (CAR). The CAR included a human anti- CD22 scFv antibody, a CD8alpha transmembrane domain, a 4-1BB intracellular signaling domain, and a CD3zeta intracellular signaling domain.

All subjects had previously undergone at least one prior allogeneic hematopoietic stem cell transplant and had received treatment with one of various CD19-directed CAR-T cell therapies. Five of the subjects had relapsed with ALL on which CD19 was not detected ("CD19 neg") and one subject was otherwise a non-responder to the prior CD19 CAR therapy.

**Table 3 summarizes the characteristics of the treated patients.**

| **Table 3: Patient Characteristics** | | | | | | |
|---|---|---|---|---|---|---|
| **ID** | **Age/Sex** | **Prior HCT** | **Prior anti-CD19 CAR** | **CD19 neg relapse** | **CD22 site density** | **Pre-HCT disease burden (% leukemia in aspirate)** |
| 1 | 22/M | Y | Y | Y | 2084 | >95% |
| 2 | 20/F | Y (2) | Y | Y | 13452 | 5% |
| 3 | 22/M | Y | Y | Y | 846 | >90% |
| 4 | 22/M | Y | Y | N | 2589 | 95% |
| 5 | 7/F | Y | Y | Y | 2839 | 32% |
| 6 | 17/F | Y | Y | Y | 2185 | 1% |

| | | | | | | |
|---|---|---|---|---|---|---|
| HCT: hematopoietic cell transplantation. | | | | | | |

Prior to administration of the cells, patients underwent autologous leukapheresis to harvest peripheral blood mononuclear cells (PBMCs). T cells were isolated from the harvested PBMCs by immunoaffinity-based enrichment for CD3 expression and cultured in the presence of anti-CD3/-CD28 beads, followed by transduction with a lentiviral vector encoding the anti-CD22 CAR. The cells were cultured for 7-10 days. Subjects received induction chemotherapy with 25 mg/m² fludarabine on Days -4, -3 and -2 and 900 mg/m² cyclophosphamide on day -2 (cell infusion on Day 0). Each patient received an initial CAR T 5 cell dose of 3 x 10 transduced T-cells/recipient weight (kg) by intravenous infusion. The second subject enrolled developed grade 3 diarrhea, meeting the criteria for dose-limiting toxicity (DLT), which led to dose expansion at the first dose-level to treat a total of 6 subjects. No subsequent DLTs were seen at this dosage. Two subjects developed grade 1 cytokine release syndrome (CRS), one subject developed grade 2 CRS, and in two subjects CRS was not present.

The number of CAR-T cells in peripheral blood, bone marrow or cerebrospinal fluid was determined at certain timepoints post-treatment by incubating cells with CD22-Fc. For patients in which expansion was observed, evidence for CAR-T cell expansion was seen in peripheral blood, bone marrow and cerebrospinal fluid, beginning at about day 7. The maximum or peak CAR-T cell expansion was generally observed between about day 12 and about day 15 post-infusion. Table 7 sets forth the maximum or peak percentage of anti-CD22 CAR-T cells observed in this assessment period as a percentage of total T cells in each sample for the treated subjects. Clinical responses were evaluated at day 28 (+/- 4 days) post-infusion.

As shown in Table 4, the results were consistent with responses being generally correlated to degree of CAR-T cell expansion. For three subjects that exhibited no or low CAR-T cell expansion also showed evidence of disease progression. Two other subjects had stable disease, and one was observed with complete remission with no MRD. Flow cytometric CAR persistence was detected out to 47 days post-infusion in this subject, with remission maintained for 3 months post-infusion. The results demonstrate safe, feasible, and clinically active anti-CD22 CAR T-cell therapy in subjects having undergone (and having become non-responsive to, e.g., due to epitope/antigen loss) previous anti-CD19 CAR therapy.

| **Table 4: Treatment response** | | | | | |
|---|---|---|---|---|---|
| **ID** | **Maximum CAR expansion (flow)** | | | **CRS** | **Best Response** |
| | PB | Marrow | CSF | | |
| 1 | 0 | 0 | n/a | None | PD |
| 2 | 52.3% | 19.5% | 0% | Gr 1 | MRD neg CR |
| 3 | 73% | 36% | 32% | Gr 1 | SD |
| 4 | 6% | 1% | 0% | Gr 2 | SD |
| 5 | 0% | 1.3% | 0% | None | PD |
| 6 | 1.8% | 2% | 0% | None | PD |

| | | | | | |
|---|---|---|---|---|---|
| PB: peripheral blood; CSF: cerebrospinal fluid; CRS: cytokine release syndrome; PD: progressive disease: MRD: minimal residual disease; CR: complete remission; SD: stable disease. | | | | | |

### Example 6: Comparison of In Silico Analysis and In Vitro Binding of Peptides Derived from Junction Regions of a CAR for Binding to HLA Class II

Actual binding affinities for certain HLA class II alleles (DPA1^{∗}01:03:DPB1^{∗}04:01, DRA^{∗}01:01;DRB1^{∗}03:01, DRA^{∗}01:01, DRB1^{∗}15:01, DRA^{∗}01:01;DRB1^{∗}11:01, DPA1^{∗}01:03;DPB1^{∗}04:02, DPA1^{∗}01:03;DPB1^{∗}03:01, DPA1^{∗}01:03;DPB1^{∗}01:01,DPA1^{∗}02:01;DPB1^{∗}01:01,DPA1^{∗}02:01;DPB1^{∗}04:02, DRA^{∗}01:01, DRB1^{∗}11:04, DRA^{∗}01:01, DRB1^{∗}01:02, and DPA1^{∗}02:01;DPB1^{∗}15:01) were assessed *in vitro* for exemplary overlapping 15 amino acid peptide sequences within a portion of the region spanning the junction between the CD28 and 4-1BB-derived sequences of the CAR described in Example 4. Specifically, assessment was of a series of overlapping 15-mer peptides derived from the sequence
CY**SLLVTVAFIIFWVKRGRKKLLYIFKQ**PFMRPVQT (set forth in SEQ ID NO: 160), which contains a portion of the CD28-derived transmembrane domain and 4-1BB-derived costimulatory domain and spans the junction between the domains (bond joining the two amino acids noted in underline). In addition, a series of overlapping 15-mer peptides of each of a number of different variants of this portion also were assessed, each variant containing a mutation or mutations in this region as described in Example 2.

The various 15-mer overlapping peptides were synthesized and their purity tested by MALDI-TOF Mass Spectrometry. The synthetic peptides were then incubated with recombinant MHC molecules to assess binding properties using the REVEAL Epitope Discovery System as described in Example 3. Each peptide was separately tested for this ability with respect to each of the HLA class II alleles, normalized to the degree observed for a positive control (known T cell epitope for the relevant allele). A score was calculated for each allele, in which the binding was normalized to the positive control peptide set at 100%. The potential impact in the population based on these scores was calculated as the sum of the individual allele scores for each peptide.

The results are set forth in FIG. 7. As shown, the results demonstrated that modifications within the junction region resulted in successful reduction for HLA class II binding of at least one peptide within the region.

For overlapping 15-mer peptides present within the native junction region, the results were compared to predicted binding (IC50) values obtained for binding of the same peptide:MHC complex, using the *in silico* prediction methods as described in Example 2. Since the maximum IC50 value predicted was about 50,000, the IC50 values were log transformed, subtracted from LOG(50000) and divided by LOG(50000) to obtain a normalized *in silico* score ((Log(50000) - logIC50) / Log(50000)). Immunogenicity scores (weighted based on relative frequency in the population of the individual HLA class II alleles) were obtained for the normalized *in silico* and actual binding affinities. The results are set forth in FIG. 8. In general, the *in silico* binding predictions were predictive of, and in some cases were over-predictive of, the actual *in vitro* binding results.

### Example 7: Comparison of Expression and Cytolytic Activity of CARs with Modified Junction Regions

T cells were generated that expressed either (1) the original chimeric antigen receptor (CAR) as described in Example 1, containing an anti-CD 19 scFv, a hinge domain, the junction region set forth in SEQ ID NO:5 (including a transmembrane domain derived from the native CD28 and an intracellular signaling domain derived from the native 4-1BB) (deemed the "native" junction region) and a CD3-zeta intracellular signaling domain or (2) one of a number of particular variants thereof, individually containing a modified junction region in which one or more mutations were introduced in the junction region spanning the junction between the CD28 transmembrane domain and 4-1BB costimulatory signaling domain. Each of the particular variant CARs contained a modified junction region having a mutation (with reference to SEQ ID NO:5) selected from among: K28Q/R31N/L34S, K28Q/R31N/L34A, R31N/L34S, R31N/L34A, K28Q/L34A, K28Q/R31S, K28Q/R31N, K28Q/R31A, L34S, L34A, R31S, R31N, R31A, K28S, K28Q, K28L, K28H, and K28A.

Primary human CD4+ and CD8+ T cells were isolated by immunoaffinity-based selection from human PBMC samples obtained from healthy donors. The resulting cells were stimulated by culturing with an anti-CD3/anti-CD28 reagent prior to engineering with the respective CAR. Cells were transduced using a lentiviral vector containing a nucleic acid molecule encoding the CAR and a nucleic acid encoding a truncated EGFR (EGFRt), for use as a surrogate marker for transduction, separated by a sequence encoding a T2A ribosome switch. A mock transduction was used as negative control. Transduced cells were used for expression analysis and cytolytic activity assay.

### A. CAR Expression

Cell surface CAR expression (as indicated via the surrogate marker) was assessed at day 17 post-transduction with nucleic acid encoding the native CAR or each of the modified CARs. Cells were stained with anti-EGFR antibody to detect EGFRt as a surrogate to verify CAR expression and assessed by flow cytometry. CAR expression was similar in cells transduced to express the various variant CARs containing a modified junction region, and was comparable to expression of the CAR containing the native junction region.

### B. Cytolytic Activity

Cells engineered to express the native or variant CARs were assessed for cytolytic activity against K562 target cells expressing the CD19 antigen (K562-CD19). The T cells were incubated with the target cells (K562-CD19) at various effector:target ratios (CAR:Target ratios of 1:1, 2:1 and 4:1) in a well of a culture plate. Lytic activity of the engineered T cells was assessed by measuring the number of caspase-positive target cells per well. The results showed that in this study, the T cells expressing the various variant CARs containing the modified junction region were able to kill CD19-expressing target cells in a target-specific manner, to a similar degree as T cells expressing the CAR containing the "native" junction region.

The present invention is not to be limited in scope by the embodiments disclosed herein, which are intended as single illustrations of individual aspects of the invention, and any that are functionally equivalent are within the scope of the invention. Various modifications to the models and methods of the invention, in addition to those described herein, will become apparent to those skilled in the art from the foregoing description and teachings, and are similarly intended to fall within the scope of the invention. Such modifications or other embodiments can be practiced without departing from the true scope and spirit of the invention.

### Sequences

**Table 5: Sequences**

| **SEQ ID NO** | **Sequence** | **Note** |
|---|---|---|
| **1** | ESKYGPPCPPCP | IgG4 hinge |
| **2** | FWVLWVGGVLACYSLLVTVAFIIFWV | CD28 transmembrane domain |
| **3** | KRGRKKLLYIFKQPFMRPVQTTQEEDGCSCRFPEEEEGGCEL | 4-1BB costimulatory domain (amino acids 214-255 of Q07011.1) Homo sapien |
| **4** | RVKFSRSADAPAYQQGQNQLYNELNLGRREEYDVLDKRRGRDPEMGGKP RRKNPQEGLYNELQKDKMAEAYSEIGMKGERRRGKGHDGLYQGLSTATK DTYDALHMQALPPR | CD3-zeta intracellular signaling domain |
| **5** | FWVLVVVGGVLACYSLLVTVAFIIFWVKRGRKKLLYIFKQPFMRPVQTT QEEDGCSCRFPEEEEGGCEL | CD28-4-1BB |
| **6** | CYSLLVTVAFIIFWVKRGRKKLLYIFKQPF | Peptide |
| **7** | VAFIIFWVKRGRKKLL | Peptide |
| **8** | AFIIFWVKRGRKKLL | Peptide |
| **9** | FWVKRGRKKLLYIFK | Peptide |
| **10** | FIIFWVKRGRKKLL | Peptide |
| **11** | FIIFWVKRGRKKL | Peptide |
| **12** | IIFWVKRGRKKLL | Peptide |
| **13** | CYSLLVTVAFIIFWV**NN**KRGRKKLLYIFKQPF | Variant junction region |
| **14** | IIFWVNNKRGRKKL | Variant peptide |
| **15** | IIFWVNNKRGRKK | Variant peptide |
| **16** | VAFIIFWVK | Synthetic peptide |
| **17** | AFIIFWVKR | Synthetic peptide |
| **18** | FIIFWVKRG | Synthetic peptide |
| **19** | IIFWVKRGR | Synthetic peptide |
| **20** | IFWVKRGRK | Synthetic peptide |
| **21** | FWVKRGRKK | Synthetic peptide |
| **22** | WVKRGRKKL | Synthetic peptide |
| **23** | VAFIIFWVS | Synthetic peptide K28S |
| **24** | AFIIFWVSR | Synthetic peptide K28S |
| **25** | FIIFWVSRG | Synthetic peptide K28S |
| **26** | IIFWVSRGR | Synthetic peptide K28S |
| **27** | IFWVSRGRK | Synthetic peptide K28S |
| **28** | FWVSRGRKK | Synthetic peptide K28S |
| **29** | WVSRGRKKL | Synthetic peptide K28S |
| **30** | VAFIIFWVL | Synthetic peptide K28L |
| **31** | AFIIFWVLR | Synthetic peptide K28L |
| **32** | FIIFWVLRG | Synthetic peptide K28L |
| **33** | IIFWVLRGR | Synthetic peptide K28L |
| **34** | IFWVLRGRK | Synthetic peptide K28L |
| **35** | FWVLRGRKK | Synthetic peptide K28L |
| **36** | WVLRGRKKL | Synthetic peptide K28L |
| **37** | VAFIIFWVH | Synthetic peptide K28H |
| **38** | AFIIFWVHR | Synthetic peptide K28H |
| **39** | FIIFWVHRG | Synthetic peptide K28H |
| **40** | IIFWVHRGR | Synthetic peptide K28H |
| **41** | IFWVHRGRK | Synthetic peptide K28H |
| **42** | FWVHRGRKK | Synthetic peptide K28H |
| **43** | WVHRGRKKL | Synthetic peptide K28H |
| **44** | VAFIIFWVA | Synthetic peptide K28A |
| **45** | AFIIFWVAR | Synthetic peptide K28A |
| **46** | IIFWVARGR | Synthetic peptide K28A |
| **47** | IFWVARGRK | Synthetic peptide K28A |
| **48** | FWVARGRKK | Synthetic peptide K28A |
| **49** | WVARGRKKL | Synthetic peptide K28A |
| **50** | VAFIIFWVQ | Synthetic peptide K28Q |
| **51** | AFIIFWVQR | Synthetic peptide K28Q |
| **52** | FIIFWVQRG | Synthetic peptide K28Q |
| **53** | IIFWVQRGR | Synthetic peptide K28Q |
| **54** | IFWVQRGRK | Synthetic peptide K28Q |
| **55** | FWVQRGRKK | Synthetic peptide K28Q |
| **56** | WVQRGRKKL | Synthetic peptide K28Q |
| **57** | IIFWVKRGS | Synthetic peptide R31S |
| **58** | IFWVKRGSK | Synthetic peptide R31S |
| **59** | FWVKRGSKK | Synthetic peptide R31S |
| **60** | WVKRGSKKL | Synthetic peptide R31S |
| **61** | IIFWVKRGL | Synthetic peptide R31L |
| **62** | IFWVKRGLK | Synthetic peptide R31L |
| **63** | FWVKRGLKK | Synthetic peptide R31L |
| **64** | WVKRGLKKL | Synthetic peptide R31L |
| **65** | IIFWVKRGH | Synthetic peptide R31H |
| **66** | IFWVKRGHK | Synthetic peptide R31H |
| **67** | FWVKRGHKK | Synthetic peptide R31H |
| **68** | WVKRGHKKL | Synthetic peptide R31H |
| **69** | IIFWVKRGA | Synthetic peptide R31A |
| **70** | IFWVKRGAK | Synthetic peptide R31A |
| **71** | FWVKRGAKK | Synthetic peptide R31A |
| **72** | WVKRGAKKL | Synthetic peptide R31A |
| **73** | IIFWVKRGN | Synthetic peptide R31N |
| **74** | IFWVKRGNK | Synthetic peptide R31N |
| **75** | FWVKRGNKK | Synthetic peptide R31N |
| **76** | WVKRGNKKL | Synthetic peptide R31N |
| **77** | IIFWVQRGS | Synthetic peptide K28Q/R31S |
| **78** | IFWVQRGSK | Synthetic peptide K28Q/R31S |
| **79** | FWVQRGSKK | Synthetic peptide K28Q/R31S |
| **80** | WVQRGSKKL | Synthetic peptide K28Q/R31S |
| **81** | IIFWVQRGA | Synthetic peptide K28Q/R31A |
| **82** | IFWVQRGAK | Synthetic peptide K28Q/R31A |
| **83** | FWVQRGAKK | Synthetic peptide K28Q/R31A |
| **84** | WVQRGAKKL | Synthetic peptide K28Q/R31A |
| **85** | IIFWVQRGN | Synthetic peptide K28Q/R31N |
| **86** | IFWVQRGNK | Synthetic peptide K28Q/R31N |
| **87** | FWVQRGNKK | Synthetic peptide K28Q/R31N |
| **88** | WVQRGNKKL | Synthetic peptide K28Q/R31N |
| **89** | WVKRGRKKS | Synthetic peptide L34S |
| **90** | WVKRGRKKA | Synthetic peptide L34A |
| **91** | WVQRGNKKS | Synthetic peptide K28Q/L34S |
| **92** | WVQRGNKKA | Synthetic peptide K28Q/L34A |
| **93** | MGNSCYNIVATLLLVLNFERTRSLQDPCSNCPAGTFCDNNRNQICSPCP PNSFSSAGGQRTCDICRQCKGVFRTRKECSSTSNAECDCTPGFHCLGAG CSMCEQDCKQGQELTKKGCKDCCFGTFNDQKRGICRPWTNCSLDGKSVL VNGTKERDVVCGPSPADLSPGASSVTPPAPAREPGHSPQIISFFLALTS TALLFLLFFLTLRFSVVKRGRKKLLYIFKQPFMRPVQTTQEEDGCSCRF PEEEEGGCEL | 4-1BB costimulatory domain (Accession No. Q07011.1 ) Homo sapien |
| **94** | MLRLLLALNLFPSIQVTGNKILVKQSPMLVAYDNAVNLSCKYSYNLFSR EFRASLHKGLD SAVEVCVVYGNYSQQLQVYSKTGFNCDGKLGNESVTFYLQNLYVNQTDI YFCKIEVMYPP PYLDNEKSNGTIIHVKGKHLCPSPLFPGPSKPFWVLVVVGGVLACYSLL VTVAFIIFWVR SKRSRLLHSDYMNMTPRRPGPTRKHYQPYAPPRDFAAYRS | CD28 transmembrane domain (Accession No. P10747) Homo sapien |
| **95** | MKWKALFTAAILQAQLPITEAQSFGLLDPKLCYLLDGILFIYGVILTAL FLRVKFSRSADAPAYQQGQNQLYNELNLGRREEYDVLDKRRGRDPEMGG KPQRRKNPQEGLYNELQKDKMAEAYSEIGMKGERRRGKGHDGLYQGLST ATKDTYDALHMQALPPR | CD3 zeta chain (Accession No. P20963) Homo sapien |
| **96** | FIIFWVNNKRGRKK | Synthetic peptide |
| **97** | IFWVNNKRGRKKLL | Synthetic peptide |
| **98** | FIIFWVNNKRGRKK | Synthetic peptide |
| **99** | IFWVNNKRGRKKLL | Synthetic peptide |
| **100** | VAFIIFWVR | Synthetic peptide K28R |
| **101** | AFIIFWVRR | Synthetic peptide K28R |
| **102** | FIIFWV**A**RG | Synthetic peptide K28A |
| **103** | MFWVLVWGGVLACYSLLVTVAF11FWV | CD28 transmembrane domain |
| | | (amino acids 153-179 of Accession No. P10747) Homo sapien |
| **104** | IEVMYPPPYLDNEKSNGTIIHVKGKHLCPSPLFPGPSKPFWVLVVVGGV LACYSLLVTVAFIIFWV | CD28, including transmembrane (amino acids 114-179 of Accession No. P10747) Homo sapien |
| **105** | RVKFSRSAEPPAYQQGQNQLYNELNLGRREEYDVLDKRRGRDPEMGGKP RRKNPQEGLYNELQKDKMAEAYSEIGMKGERRRGKGHDGLY QGLSTATKDTYDALHMQALPPR | CD3 zeta Homo sapien |
| **106** | GAATCTAAGTACGGACCGCCCTGCCCCCCTTGCCCT | spacer (IgG4hinge) (nt) homo sapien |
| **107** | ESKYGPPCPPCPGQPREPQVYTLPPSQEEMTKNQVSLTCLVKGFYPSDI AVEWESNGQPENNYKTTPPVLDSDGSFFLYSRLTVDKSRWQEGNVFSCS VMHEALHNHYTQKSLSLSLGK | Hinge-CH3 spacer Homo sapien |
| **108** | ESKYGPPCPPCPAPEFLGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK | Hinge-CH2-CH3 spacer Homo sapien |
| **109** | RWPESPKAQASSVPTAQPQAEGSLAKATTAPATTRNTGRGGEEKKKEKE KEEQEERETKTPECPSHTQPLGVYLLTPAVQDLWLRDKATFTCFVVGSD LKDAHLTWEVAGKVPTGGVEEGLLERHSNGSQSQHSRLTLPRSLWNAGT SVTCTLNHPSLPPQRLMALREPAAQAPVKLSLNLLASSDPPEAASWLLC EVSGFSPPNILLMWLEDQREVNTSGFAPARPPPQPGSTTFWAWSVLRVP APPSPQPATYTCVVSHEDSRTLLNASRSLEVSYVTDH | IgD-hinge-Fc Homo sapien |
| **110** | LEGGGEGRGSLLTCGDVEENPGPR | T2A artificial |
| **111** | MLLLVTSLLLCELPHPAFLLIPRKVCNGIGIGEFKDSLSINATNIKHFK NCTSISGDLHILPVAFRGDSFTHTPPLDPQELDILKTVKEITGFLLIQA WPENRTDLHAFENLEIIRGRTKQHGQFSLAVVSLNITSLGLRSLKEISD GDVIISGNKNLCYANTINWKKLFGTSGQKTKIISNRGENSCKATGQVCH ALCSPEGCWGPEPRDCVSCRNVSRGRECVDKCNLLEGEPREFVENSECI QCHPECLPQAMNITCTGRGPDNCIQCAHYIDGPHCVKTCPAGVMGENNT LVWKYADAGHVCHLCHPNCTYGCTGPGLEGCPTNGPKIPSIATGMVGAL LLLLVVALGIGLFM | tEGFR artificial |
| **112** | RSKRSRLLHSDYMNMTPRRPGPTRKHYQPYAPPRDFAAYRS | CD28 cytoplasmic domain (amino acids 180-220 of P10747) Homo sapien |
| **113** | RSKRSRGGHSDYMNMTPRRPGPTRKHYQPYAPPRDFAAYRS | CD28 cytoplasmic domain variant (LL to GG) Homo sapien |
| **114** | FWVLVVVGGVLACYSLLVTVAFIIFWVARGRKKLLYIFKQPFMRPVQTT QEEDGCSCRFPEEEEGGCEL | CD28-4-1BB K28A variant |
| **115** | FWVLVVVGGVLACYSLLVTVAFIIFWVHRGRKKLLYIFKQPFMRPVQTT QEEDGCSCRFPEEEEGGCEL | CD28-4-1BB K28H variant |
| **116** | FWVLVVVGGVLACYSLLVTVAFIIFWVLRGRKKLLYIFKQPFMRPVQTT QEEDGCSCRFPEEEEGGCEL | CD28-4-1BB |
| | | K28L variant |
| **117** | FWVLVVVGGVLACYSLLVTVAFIIFWVQRGRKKLLYIFKQPFMRPVQTT QEEDGCSCRFPEEEEGGCEL | CD28-4-1BB K28Q variant |
| **118** | FWVLVVVGGVLACYSLLVTVAFIIFWVSRGRKKLLYIFKQPFMRPVQTT QEEDGCSCRFPEEEEGGCEL | CD28-4-1BB K28S variant |
| **119** | FWVLVVVGGVLACYSLLVTVAFIIFWVKRGAKKLLYIFKQPFMRPVQTT QEEDGCSCRFPEEEEGGCEL | CD28-4-1BB R31A variant |
| **120** | FWVLVVVGGVLACYSLLVTVAFIIFWVKRGHKKLLYIFKQPFMRPVQTT QEEDGCSCRFPEEEEGGCEL | CD28-4-1BB R31H variant |
| **121** | FWVLVVVGGVLACYSLLVTVAFIIFWVKRGLKKLLYIFKQPFMRPVQTT QEEDGCSCRFPEEEEGGCEL | CD28-4-1BB R31L variant |
| **122** | FWVLVVVGGVLACYSLLVTVAFIIFWVKRGNKKLLYIFKQPFMRPVQTT QEEDGCSCRFPEEEEGGCEL | CD28-4-1BB R31N variant |
| **123** | FWVLVVVGGVLACYSLLVTVAFIIFWVKRGRKKALYIFKQPFMRPVQTT QEEDGCSCRFPEEEEGGCEL | CD28-4-1BB L34A variant |
| **124** | FWVLVVVGGVLACYSLLVTVAFIIFWVKRGRKKSLYIFKQPFMRPVQTT QEEDGCSCRFPEEEEGGCEL | CD28-4-1BB L34S variant |
| **125** | FWVLVVVGGVLACYSLLVTVAFIIFWVQRGAKKLLYIFKQPFMRPVQTT QEEDGCSCRFPEEEEGGCEL | CD28-4-1BB K28Q/R31A variant |
| **126** | FWVLVVVGGVLACYSLLVTVAFIIFWVQRGNKKLLYIFKQPFMRPVQTT QEEDGCSCRFPEEEEGGCEL | CD28-4-1BB K28Q/R31N variant |
| **127** | FWVLVVVGGVLACYSLLVTVAFIIFWVQRGSKKLLYIFKQPFMRPVQTT QEEDGCSCRFPEEEEGGCEL | CD28-4-1BB K28Q/R31S variant |
| **128** | FWVLVVVGGVLACYSLLVTVAFIIFWVQRGRKKALYIFKQPFMRPVQTT QEEDGCSCRFPEEEEGGCEL . | CD28-4-1BB K28Q/L34A variant |
| **129** | FWVLVVVGGVLACYSLLVTVAFIIFWVQRGRKKSLYIFKQPFMRPVQTT QEEDGCSCRFPEEEEGGCEL | CD28-4-1BB K28Q/L34S variant |
| **130** | FWVLVVVGGVLACYSLLVTVAFIIFWVKRGNKKALYIFKQPFMRPVQTT QEEDGCSCRFPEEEEGGCEL | CD28-4-1BB R31N/L34A variant |
| **131** | FWVLVVVGGVLACYSLLVTVAFIIFWVKRGNKKSLYIFKQPFMRPVQTT QEEDGCSCRFPEEEEGGCEL | CD28-4-1BB R31N/L34S variant |
| **132** | FWVLVVVGGVLACYSLLVTVAFIIFWVQRGNKKALYIFKQPFMRPVQTT QEEDGCSCRFPEEEEGGCEL | CD28-4-1BB K28Q/R31N/L34A variant |
| **133** | FWVLVVVGGVLACYSLLVTVAFIIFWVQRGNKKSLYIFKQPFMRPVQTT QEEDGCSCRFPEEEEGGCEL | CD28-4-1BB K28Q/R31N/L34S variant |
| **134** | FWVLVVVGGVLACYSLLVTVAFIIFWVNNKRGRKKLLYIFKQPFMRPVQ TTQEEDGCSCRFPEEEEGGCEL | CD28-4-1BB with variant junction region with NN insertion. |
| **135** | MFWVLVVVGGVLACYSLLVTVAFIIFWVKRGRKKLLYIFKQPFMRPVQT | CD28-4-1BB |
| | TQEEDGCSCRFPEEEEGGCEL | |
| **136** | IEVMYPPPYLDNEKSNGTIIHVKGKHLCPSPLFPGPSKPFWVLVVVGGV LACYSLLVTVAFIIFWVKRGRKKLLYIFKQPFMRPVQTTQEEDGCSCRF PEEEEGGCEL | CD28-4-1BB |
| **137** | SLLVTVAFIIFWV**K**RG**R**KK**L**LYIFKQ | CD28-4-1BB junction region |
| **138** | SLLVTVAFIIFWV**A**RG**R**KK**L**LYIFKQ | CD28-4-1BB junction region K14A variant |
| **139** | SLLVTVAFIIFWV**H**RG**R**KK**L**LYIFKQ | CD28-4-1BB junction region K14H variant |
| **140** | SLLVTVAFIIFWV**L**RG**R**KK**L**LYIFKQ | CD28-4-1BB junction region K14L variant |
| **141** | SLLVTVAFIIFWV**Q**RG**R**KK**L**LYIFKQ | CD28-4-1BB junction region K14Q variant |
| **142** | SLLVTVAFIIFWV**S**RG**R**KK**L**LYIFKQ | CD28-4-1BB junction region K14S variant |
| **143** | SLLVTVAFIIFWV**K**RG**A**KK**L**LYIFKQ | CD28-4-1BB junction region R17A variant |
| **144** | SLLVTVAFIIFWV**K**RG**H**KK**L**LYIFKQ | CD28-4-1BB junction region R17H variant |
| **145** | SLLVTVAFIIFWV**K**RG**L**KK**L**LYIFKQ | CD28-4-1BB junction region R17L variant |
| **146** | SLLVTVAFIIFWV**K**RG**N**KK**L**LYIFKQ | CD28-4-1BB junction region R17N variant |
| **147** | SLLVTVAFIIFWV**K**RG**R**KK**A**LYIFKQ | CD28-4-1BB junction region L20A variant |
| **148** | SLLVTVAFIIFWV**K**RG**R**KK**S**LYIFKQ | CD28-4-1BB junction region L20S variant |
| **149** | SLLVTVAFIIFWV**Q**RG**A**KK**L**LYIFKQ | CD28-4-1BB junction region K14Q/R17A variant |
| **150** | SLLVTVAFIIFWV**Q**RG**N**KK**L**LYIFKQ | CD28-4-1BB junction region K14Q/R17N variant |
| **151** | SLLVTVAFIIFWV**Q**RG**S**KK**L**LYIFKQ | CD28-4-1BB junction region |
| | | K14Q/R17S variant |
| **152** | SLLVTVAFIIFWV**Q**RG**R**KK**A**LYIFKQ | CD28-4-1BB junction region K14Q/L20A variant |
| **153** | SLLVTVAFIIFWV**Q**RG**R**KK**S**LYIFKQ | CD28-4-1BB junction region K14Q/L20S variant |
| **154** | SLLVTVAFIIFWV**K**RG**N**KK**A**LYIFKQ | CD28-4-1BB junction region R17N/L20A variant |
| **155** | SLLVTVAFIIFWV**K**RG**N**KK**S**LYIFKQ | CD28-4-1BB junction region R17N/L20S variant |
| **156** | SLLVTVAFIIFWV**Q**RG**N**KK**A**LYIFKQ | CD28-4-1BB junction region K14Q/R 17N/L20A variant |
| **157** | SLLVTVAFIIFWV**Q**RG**N**KK**S**LYIFKQ | CD28-4-1BB junction region K14Q/R 17N/L20S variant |
| **158** | G A A T C T A A G T A C G G A C C G C C C T G C C C C C C T T G  C C C T | spacer (IgG4hinge) (nucleotide) homo sapien |
| **159** | RVKFSRSADAPAYKQGQNQLYNELNLGRREEYDVLDKRRGRDPEMGGKP RRKNPQEGLYNELQKDKMAEAYSEIGMKGERRRGKGHDGLYQGLSTATK DTYDALHMQALPPR | CD3 Zeta |
| **160** | CYSLLVTVAFIIFWVKRGRKKLLYIFKQPFMRPVQT | peptide |
| **161** | CYSLLVTVAFIIFWV | Synthetic peptide |
| **162** | LLVTVAFIIFWVKRG | Synthetic peptide |
| **163** | TVAFIIFWVKRGRKK | Synthetic peptide |
| **164** | FIIFWVKRGRKKLLY | Synthetic peptide |
| **165** | FWVKRGRKKLLYIFK | Synthetic peptide |
| **166** | KRGRKKLLYIFKQPF | Synthetic peptide |
| **167** | RKKLLYIFKQPFMRP | Synthetic peptide |
| **168** | LLYIFKQPFMRPVQT | Synthetic peptide |
| **169** | TVAFIIFWVKRGHKK | Synthetic peptide R31H |
| **170** | FIIFWVKRGHKKLLY | Synthetic peptide R31H |
| **171** | FWVKRGHKKLLYIFK | Synthetic peptide R31H |
| **172** | KRGHKKLLYIFKQPF | Synthetic peptide |
| | | R31H |
| **173** | HKKLLYIFKQPFMRP | Synthetic peptide R31H |
| **174** | TVAFIIFWVKRGNKK | Synthetic peptide R31N |
| **175** | FIIFWVKRGNKKLLY | Synthetic peptide R31N |
| **176** | FWVKRGNKKLLYIFK | Synthetic peptide R31N |
| **177** | KRGNKKLLYIFKQPF | Synthetic peptide R31N |
| **178** | NKKLLYIFKQPFMRP | Synthetic peptide R31N |
| **179** | FIIFWVKRGRKKALY | Synthetic peptide L34A |
| **180** | FWVKRGRKKALYIFK | Synthetic peptide L34A |
| **181** | KRGRKKALYIFKQPF | Synthetic peptide L34A |
| **182** | RKKALYIFKQPFMRP | Synthetic peptide L34A |
| **183** | FWVLVVVGGVLACYSLLVTVAFIIFWVKRGSKKLLYIFKQPFMRPVQTT QEEDGCSCRFPEEEEGGCEL | CD28-4-1BB R31S variant |
| **184** | SLLVTVAFIIFWVKRGSKKLLYIFKQ | CD28-4-1BB junction region R31S variant |

### Embodiments of the invention

Embodiment 1. A method of inducing tolerance to a chimeric receptor, comprising administering to a subject at least one peptide comprising all or a portion of an immunogenic region of said chimeric receptor, said peptide being administered under conditions that induces tolerance in the subject to the chimeric receptor.

Embodiment 2. The method of embodiment 1, wherein the chimeric receptor is expressed by cells genetically engineered with nucleic acid encoding the chimeric receptor.

Embodiment 3. The method of embodiment 1, further comprising administering to the subject the chimeric receptor and/or administering cells expressing the chimeric receptor for treating a disease or condition in the subject.

Embodiment 4. The method of any of embodiments 1-3, wherein the chimeric receptor is a chimeric antigen receptor.

Embodiment 5. A method of treatment, comprising:
a) administering to a subject at least one peptide comprising all or a portion of an immunogenic region of a chimeric antigen receptor, said peptide being administered under conditions that induce tolerance in the subject to the chimeric antigen receptor; and
b) administering to the subject cells expressing the chimeric antigen receptor, wherein the chimeric antigen receptor specifically binds to an antigen associated with a disease or condition in the subject.

Embodiment 6. The method of any of embodiments 3-5, wherein the peptide is administered prior to, subsequently or intermittently from administration of the chimeric receptor.

Embodiment 7. The method of any of embodiments 3-6, wherein the peptide is administered prior to administration of the chimeric receptor.

Embodiment 8. The method of any of embodiments 3-7, wherein the peptide is administered from or from about 7 to 28 days before administration of the chimeric receptor.

Embodiment 9. The method of any of embodiments 1-8, wherein the peptide is administered in the absence of an adjuvant.

Embodiment 10. The method of any of embodiments 1-8, wherein the method further comprises administering a tolerogenic agent.

Embodiment 11. The method of embodiment 10, wherein the tolerogenic agent targets one or more molecules that is expressed on T cells selected from among CD4, CD40, CD40L, OX40, OX40L, CD26, CD44, CD28, PD1, BTLA, B7-1, ICOS, CTLA-4, B7-2 family, CD99, CD137 (4-1BBL), CD2, LFA3, CD27, CD70, CD3, CD8, ICAM1, LFA1, MHC class II and MHC class I molecule, or targets one or more molecules that are expressed on non-T cells, such as macrophages or dendritic cells, that serve as the ligand/receptor pair for the molecules identified above on the T cells.

Embodiment 12. The method of embodiment 11, wherein the tolerogenic agent is an antibody or fragment thereof or is a soluble fusion protein.

Embodiment 13. The method of any of embodiments 1-8 and 10-12, wherein the peptide is administered in the presence of adjuvant.

Embodiment 14. The method of any of embodiments 1-13, wherein the peptide is administered by, intravenous, intraperitoneal, intranasal, oral or intrathymic administration.

Embodiment 15. The method of embodiment 14, wherein the peptide is administered by intraperitoneal injection.

Embodiment 16. The method of any of embodiments 1-15, wherein the peptide is administered in an amount that is from or from about 0.1 mg to 1000 mg.

Embodiment 17. The method of any of embodiments 1-16, wherein the peptide is administered in an amount that is from or from about 100 mg to 1000 mg.

Embodiment 18. The method of any of embodiments 1-16, wherein the peptide is administered in an amount that is from or from about 0.1 mg to 5 mg.

Embodiment 19. The method of any of embodiments 1-18, wherein the peptide is administered a plurality of times by repeated administration.

Embodiment 20. The method of embodiment 19, wherein the peptide is administered at least two times, at least three times, at least four times, at least five times, at least six times or at least seven times.

Embodiment 21. The method of any of embodiments 1-20, wherein the peptide is administered at least once daily for two days, three days, four days, five days, six days or seven days.

Embodiment 22. The method of any of embodiments 19-21, wherein each administration of the peptide occurs prior to administration of the chimeric receptor.

Embodiment 23. The method of any of embodiments 1-22, wherein the peptide comprises a plurality of peptides, each of said peptides comprising all or a portion of an immunogenic region of said chimeric receptor.

Embodiment 24. The method of embodiment 23, wherein the plurality of peptides are peptides of two or more different immunogenic regions of said chimeric receptor.

Embodiment 25. The method of embodiment 24, wherein the plurality of peptides are peptides of the same immunogenic region of said chimeric receptor, each of said peptides overlapping in sequence and comprising a portion of said immunogenic region.

Embodiment 26. The method of any of embodiments 23-25, wherein the plurality of peptides comprises at least two, three, four, five, six, seven, eight, nine or ten different peptides.

Embodiment 27. The method of any of embodiments 1-26, wherein said immunogenic region comprises a T cell epitope.

Embodiment 28. The method of embodiment 27, wherein the T cell epitope is an MHC class I or an MHC class II epitope.

Embodiment 29. The method of embodiment 27, wherein the T cell epitope is an MHC class I epitope.

Embodiment 30. The method of any of embodiments 1-29, wherein the subject is positive for an HLA allele that specifically binds to the administered peptide.

Embodiment 31. The method of any of embodiments 1-30, comprising prior to administering the peptide, selecting a subject that is positive for an HLA allele that specifically binds to the administered peptide.

Embodiment 32. The method of any of embodiments 28-31, wherein:
the peptide binds an MHC class I and the HLA allele is selected from among HLA-A^{∗}02:01, HLA-A^{∗}03:01, HLA-A^{∗}11:01 and HLA-B^{∗}08:01; or
the peptide binds an MHC class II and the HLA allele comprises and alpha and/or beta chain selected from HLA-DPA1^{∗}0103, HLA-DPA1^{∗}0201, HLA-DPB1^{∗}0101, HLA-DPB1^{∗}0301, HLA-DPB 1 ^{∗}0401, HLA-DPB ^{∗}0402, HLA-DPB1^{∗}1501, HLA-DRA^{∗} 0101, HLA-DRB1^{∗}1101.

Embodiment 33. The method of any of embodiments 1-32, wherein the immunogenic region is or comprises a region within one or more portions selected from the group consisting of an scFv portion, a linker portion, an amino acid sequence not endogenous to the subject, a sequence derived from a different species than that of the subject, and/or a junction between two CAR domains; and/or where the region of the chimeric receptor is a junction region comprising amino acids on each side of a junction between two domains.

Embodiment 34. The method of any of embodiments 1-33, wherein:
the region comprises a framework region (FR) within the scFv portion,
the region comprises a heavy chain FR sequence
the region comprises a heavy chain CDR sequence,
the region comprises a light chain FR sequence, and/or
the region comprises a light chain CDR sequence.

Embodiment 35. The method of any of embodiments 1-33, wherein the immunogenic region comprises a contiguous sequence of amino acids of a junction region of the chimeric receptor, wherein the junction region comprises up to 15 contiguous amino acids directly C-terminal of a junction that joins a first domain and a second domain of the chimeric receptor and/or up to 15 contiguous amino acids directly N-terminal of the junction, and optionally further comprises the junction.

Embodiment 36. The method of embodiment 35, wherein the first domain and second domain are directly linked or are indirectly linked via a linker or linkers.

Embodiment 37. The method of embodiment 35 or embodiment 36, wherein each of the first and second domain comprise domain sequences of a protein derived from the same species as the subject and/or domain sequences of a protein endogenous to the subject.

Embodiment 38. The method of any of embodiments 1-37, wherein the subject is a human.

Embodiment 39. The method of any of embodiments 35-38, wherein the peptide comprises a contiguous sequence of amino acids of the junction region.

Embodiment 40. The method of any of embodiments 1-39, wherein the peptide is between 7 and 30 amino acids, 8 and 20 amino acids, 8 and 15 amino acids, 10 and 17 amino acids, 7 and 13 amino acids or 8 and 10 amino acids.

Embodiment 41. The method of any of embodiments 1-38, wherein the peptide is between 10 and 17 amino acids.

Embodiment 42. The method of any of embodiments 1-41, wherein the peptide is or is about 10 amino acids, 11 amino acids, 12 amino acids, 13 amino acids, 14 amino acids, 15 amino acids, 16 amino acids or 17 amino acids.

Embodiment 43. The method of any of embodiments 1-42, wherein the chimeric receptor is a CAR and the CAR comprises an extracellular antigen-recognition domain that specifically binds to a target antigen and an intracellular signaling domain comprising an activating cytoplasmic signaling domain.

Embodiment 44. The method of embodiment 43, wherein the extracellular antigen-recognition domain comprises an antibody or antigen-binding fragment.

Embodiment 45. The method of embodiment 43 or embodiment 44, wherein the extracellular antigen-recognition domain comprises an scFv.

Embodiment 46. The method of any of embodiments 43-45, wherein:
the activating cytoplasmic signaling domain comprises a T cell receptor (TCR) component and/or comprise an immunoreceptor tyrosine-based activation motif (ITAM); and/or
the activating cytoplasmic signaling domain comprises an intracellular domain of a CD3-zeta (CD3ζ) chain.

Embodiment 47. The method of any of embodiments 43-46, further comprising a transmembrane domain linking the extracellular domain and the intracellular signaling domain.

Embodiment 48. The method of embodiment 47, wherein the transmembrane domain comprises a transmembrane portion of CD28.

Embodiment 49. The method of any of embodiments 43-48, wherein the intracellular signaling domain further comprises an intracellular costimulatory signaling domain of a T cell costimulatory molecule.

Embodiment 50. The method of embodiment 49, wherein the T cell costimulatory molecule is selected from the group consisting of CD28 and 41BB.

Embodiment 51. The method of any of embodiments 35-50, wherein the first domain and second domain are, respectively, an extracellular ligand binding domain and a hinge domain, an extracellular ligand binding domain and a transmembrane domain, a transmembrane domain and an intracellular costimulatory signaling domain, and an intracellular costimulatory signaling domain and an ITAM signaling domain.

Embodiment 52. The method of any of embodiments 35-51, wherein the first domain is a transmembrane domain or a functional portion thereof and the second domain is a costimulatory signaling domain or a functional portion thereof.

Embodiment 53. The method of embodiment 52, wherein the transmembrane domain is a CD28 transmembrane domain or a functional portion or variant thereof and the costimulatory signaling domain is a 4-1BB signaling domain or a functional portion or variant thereof.

Embodiment 54. The method of embodiment 53, wherein
the CD28 transmembrane domain comprises the sequence of amino acids set forth in SEQ ID NO:2, 103 or 104 or a functional portion or variant thereof comprising a sequence that exhibits at least 95% sequence identity to SEQ ID NO:2, 103 or 104; and
the 4-1BB costimulatory signaling domain comprises the sequence of amino acids set forth in SEQ ID NO:3 or a functional portion or variant thereof comprising a sequence that exhibits at least 95% sequence identity to SEQ ID NO:3.

Embodiment 55. The method of embodiment 53 or embodiment 54, wherein the first domain and second domain together comprise the sequence of amino acids set forth in SEQ ID NO:5 or a functional portion or variant thereof comprising a sequence of amino acids that exhibits at least 85%, 86%, 87%, 88%, 89%, 90%, 91%, 92%, 93%, 94%, 95%, 96%, 97%, 98% or 99% sequence identity to SEQ ID NO:5.

Embodiment 56. The method of any of embodiments 53-54, wherein the first domain and second domain together comprise the sequence of amino acids set forth in SEQ ID NO:5.

Embodiment 57. The method of any of embodiments 1-56, wherein the peptide has a binding affinity for a human leukocyte antigen (HLA) molecule that is less than 1000 nM, less than 500 nM or less than 50 nM.

Embodiment 58. The method of embodiment 57, wherein the binding affinity is an IC50.

Embodiment 59. The method of any of embodiments 57-58, wherein:
the peptide binds an MHC class I and the HLA allele is selected from among HLA-A^{∗}02:01, HLA-A^{∗}03:01, HLA-A^{∗}11:01 and HLA-B^{∗}08:01; or
the peptide binds an MHC class II and the HLA allele comprises an alpha and/or beta chain selected from HLA-DPA1^{∗}0103, HLA-DPA1^{∗}0201, HLA-DPB1^{∗}0101, HLA-DPB1^{∗}0301, HLA-DPB1^{∗}0401, HLA-DPB^{∗}0402, HLA-DPB1^{∗}1501, HLA-DRA^{∗}0101, HLA-DRB1^{∗}1101.

Embodiment 60. The method of any of embodiments 1-59, wherein the peptide is administered no more than one week prior to administering the chimeric receptor.

Embodiment 61. The method of any of embodiments 1-60, further comprising administering an immunosuppressive compound.

Embodiment 62. The method of any of embodiments 3-61, wherein the chimeric antigen receptor specifically binds to an antigen associated with the disease or condition.

Embodiment 63. The method of embodiment 62, wherein the disease or condition is a cancer, and autoimmune disease or disorder, or an infectious disease.

Embodiment 64. Use of a peptide for formulation of a medicament for reducing an immune response associated with treatment with a chimeric receptor, wherein said peptide is formulated for administration to induce tolerance in a subject to the chimeric receptor.

Embodiment 65. A pharmaceutical composition, comprising a peptide for use in reducing an immune response associated with treatment with a chimeric receptor, wherein said peptide is formulated for administration to induce tolerance in a subject to the chimeric receptor.

Embodiment 66. The use of embodiment 64 or pharmaceutical composition of embodiment 65, wherein the peptide is formulated for administration in the absence of an adjuvant.

Embodiment 67. Use of a chimeric receptor for formulation of a medicament for treating a disease or condition in a subject that has received a peptide to induce tolerance to the chimeric receptor.

Embodiment 68. A pharmaceutical composition, comprising a chimeric receptor for use in treating a disease or condition in a subject that has received a peptide to induce tolerance to the chimeric receptor.

Embodiment 69. The use or pharmaceutical composition of any of embodiments 64-68, wherein the chimeric receptor is a chimeric antigen receptor.

Embodiment 70. The use or pharmaceutical composition of any of embodiments 64-69, wherein the chimeric receptor is expressed by cells genetically engineered with nucleic acid encoding the chimeric receptor.

Embodiment 71. The use or pharmaceutical composition of any of embodiments 64-70, wherein the chimeric receptor binds to an antigen associated with the disease or condition.

Embodiment 72. The use or pharmaceutical composition of embodiment 71, wherein the disease or condition is a cancer, and autoimmune disease or disorder, or an infectious disease.

Embodiment 73. An isolated peptide, comprising a contiguous sequence of amino acids of a junction region of a chimeric receptor, wherein the junction region comprises up to 15 contiguous amino acids directly C-terminal of a junction that joins a first domain of the chimeric receptor and a second domain of the chimeric receptor and/or up to 15 contiguous amino acids directly N-terminal of the junction, and optionally further comprises the junction.

Embodiment 74. The isolated peptide of embodiment 73, wherein the first domain and second domain are directly linked or are indirectly linked via a linker or linkers.

Embodiment 75. The isolated peptide of embodiment 73 or embodiment 74, wherein each of the first and second domain comprise domain sequences of a protein derived from the same species as the subject and/or domain sequences of a protein endogenous to the subject.

Embodiment 76. The isolated peptide of any of embodiments 73-75, wherein the first domain and second domain are, respectively, an extracellular ligand binding domain and a hinge domain, an extracellular ligand binding domain and a transmembrane domain, a transmembrane domain and an intracellular costimulatory signaling domain, and an intracellular costimulatory signaling domain and an ITAM signaling domain.

Embodiment 77. The isolated peptide of any of embodiments 73-76, wherein the first domain is a transmembrane domain or a functional portion thereof and the second domain is a costimulatory signaling domain or a functional portion thereof.

Embodiment 78. The isolated peptide of embodiment 77, wherein the transmembrane domain is a CD28 transmembrane domain or a functional portion or variant thereof and the costimulatory signaling domain is a 4-1BB signaling domain or a functional portion or variant thereof.

Embodiment 79. The isolated peptide of embodiment 78, wherein
the CD28 transmembrane domain comprises the sequence of amino acids set forth in SEQ ID NO:2, 103 or 104 or a functional portion or variant thereof comprising a sequence that exhibits at least 95% sequence identity to SEQ ID NO:2, 103 or 104; and
the 4-1BB costimulatory signaling domain comprises the sequence of amino acids set forth in SEQ ID NO:3 or a functional portion or variant thereof comprising a sequence that exhibits at least 95% sequence identity to SEQ ID NO:3.

Embodiment 80. The isolated peptide of embodiment 78 or embodiment 79, wherein the first domain and second domain together comprise the sequence of amino acids set forth in SEQ ID NO: 5 or a functional portion or variant thereof comprising a sequence of amino acids that exhibits at least 85%, 86%, 87%, 88%, 89%, 90%, 91%, 92%, 93%, 94%, 95%, 96%, 97%, 98% or 99% sequence identity to SEQ ID NO:5.

Embodiment 81. The isolated peptide of any of embodiments 78-80, wherein the first domain and second domain together comprise the sequence of amino acids set forth in SEQ ID NO:5.

Embodiment 82. The isolated peptide of any of embodiments 73-81, wherein:
the peptide is between 7 and 30 amino acids, 8 and 20 amino acids, 8 and 15 amino acids, 10 and 17 amino acids, 7 and 13 amino acids or 8 and 10 amino acid; or
the peptide is or is about 10 amino acids, 11 amino acids, 12 amino acids, 13 amino acids, 14 amino acids, 15 amino acids, 16 amino acids or 17 amino acids.

Embodiment 83. The isolated peptide of any of embodiments 73-82, wherein the peptide has a binding affinity for a human leukocyte antigen (HLA) molecule that is less than 1000 nM, less than 500 nM or less than 50 nM.

Embodiment 84. The isolated peptide of embodiment 83, wherein the binding affinity is an IC50.

Embodiment 85. The isolated peptide of any of embodiments 73-84, wherein:
the peptide binds an MHC class I and the HLA allele is selected from among HLA-A^{∗}02:01, HLA-A^{∗}03:01, HLA-A^{∗}11:01 and HLA-B^{∗}08:01; or
the peptide binds an MHC class II and the HLA allele comprises and alpha and/or beta chain selected from HLA-DPA1^{∗}0103, HLA-DPA1^{∗}0201, HLA-DPB1^{∗}0101, HLA-DPB 1^{∗}0301, HLA-DPB1^{∗}0401, HLA-DPB^{∗}0402, HLA-DPB1^{∗}1501, HLA-DRA^{∗}0101, BLA-DRB1^{∗}1101

Embodiment 86. A composition, comprising the peptide of any of embodiments 73-85 or a plurality of peptides of any of embodiments 73-85.

Embodiment 87. A composition, comprising a plurality of peptides, wherein each of said peptides overlap in sequence and comprise all or a portion of an immunogenic region of a chimeric receptor.

Embodiment 88. The composition of embodiment 86 or embodiment 87, comprising a pharmaceutically acceptable excipient.

Embodiment 89. A combination, comprising:
a peptide of any of embodiments 73-85 or a plurality of peptides of any of embodiments 73-85; and
a chimeric receptor.

Embodiment 90. A kit, comprising the composition of any of embodiments 86-88 or combination of embodiment 89 and, optionally, instructions for use.

Embodiment 91. The composition of any of embodiments 86-88, combination of embodiment 89 or kit of embodiment 90, wherein the chimeric receptor is expressed by cells genetically engineered with nucleic acid encoding the chimeric receptor.

Embodiment 92. The composition, combination or kit of any of embodiments 86-91, wherein the chimeric receptor is a chimeric antigen receptor.

## Claims

1. An isolated peptide, comprising a contiguous sequence of amino acids of a junction region of a chimeric receptor, wherein the junction region comprises up to 15 contiguous amino acids directly C-terminal of a junction that joins a first domain of the chimeric receptor and a second domain of the chimeric receptor and/or up to 15 contiguous amino acids directly N-terminal of the junction, wherein the first domain and second domain are directly linked, wherein:
i) the first domain is a CD28 transmembrane domain comprising the sequence of amino acids set forth in SEQ ID NO:2, 103 or 104 or a functional portion or variant thereof comprising a sequence that exhibits at least 95% sequence identity to SEQ ID NO:2, 103 or 104, and
ii) the second domain is a 4-1BB signaling domain comprising the sequence of amino acids set forth in SEQ ID NO:3 or a functional portion or variant thereof comprising a sequence that exhibits at least 95% sequence identity to SEQ ID NO:3, and
wherein the peptide is between 7 and 40 amino acids in length.

2. The isolated peptide of claim 1, wherein the isolated peptide comprises the junction.

3. The isolated peptide of claim 1 or claim 2, wherein the first domain and second domain together comprise the sequence of amino acids set forth in SEQ ID NO:5 or a functional portion or variant thereof comprising a sequence of amino acids that exhibits at least 85%, 86%, 87%, 88%, 89%, 90%, 91%, 92%, 93%, 94%, 95%, 96%, 97%, 98% or 99% sequence identity to SEQ ID NO:5.

4. The isolated peptide of any one of claims 1-3, wherein the peptide:
a) comprises or consists of the sequence of amino acids set forth in any of SEQ ID NOS: 6, 7, 8-12, 16-22, 137, 163, 166, 167 or 168;
b) is or comprises the sequence of amino acids set forth in any of SEQ ID NOS: 8-12, 16-22, 163, 166, 167 or 168 or a portion thereof, wherein the portion specifically binds to an MHC molecule and is capable of inducing tolerance to a co-administered chimeric receptor; or
c) comprises a sequence of amino acids of a peptide epitope of a sequence of amino acids set forth in any of SEQ ID NOS: 6, 7, 8-12, 16-22, and 137 that binds to an HLA molecule.

5. The isolated peptide of any one of claims 1-4, wherein peptide is between 10 and 30 amino acids and comprises or consists of the sequence of amino acids set forth in any of SEQ ID NOs 7-12, 16-22, 163, or 165.

6. The isolated peptide of any one of claims 1-5, wherein the peptide is capable of binding to a major histocompatibility complex (MHC) molecule, optionally an MHC class I or MHC class II protein.

7. The isolated peptide of claim 6, wherein the peptide exhibits a binding affinity for an MHC molecule that is:
a) from 0.1 nM to 25,000 nM, from 0.1 nM to 5,000 nM, from 0.1 nM to 1,000 nM, from 0.1 nM to 500 nM, from 0.1 nM to 250 nM, from 0.1 nM to 100 nM, from 0.1 nM to 50 nM, from 0.1 nM to 25 nM, or from 0.1 nM to 10 nM, and/or
b) less than 1000 nM, less than 500 nM or less than 50 nM.

8. The isolated peptide of claim 6 or claim 7, wherein the MHC molecule is a human leukocyte antigen (HLA) molecule.

9. The isolated peptide of claim 8, wherein the HLA allele:
a) is selected from among HLA-A*02:01, HLA-A*03:01, HLA-A*11:01 and HLA-B*08:01; or
b) comprises and alpha and/or beta chain selected from HLA-DPA1*0103, HLA-DPA1*0201, HLA-DPB1*0101, HLA-DPB1*0301, HLA-DPB1*0401, HLA-DPB*0402, HLA-DPB1*1501, HLA-DRA*0101, and HLA-DRB1*1101.

10. The isolated peptide of any one of claims 1-9, wherein the peptide is:
a) between 7 and 30 amino acids, 8 and 20 amino acids, 8 and 15 amino acids, 10 and 17 amino acids, 7 and 13 amino acids, or 8 and 10 amino acids; or
b) 10 amino acids, 11 amino acids, 12 amino acids, 13 amino acids, 14 amino acids, 15 amino acids, 16 amino acids or 17 amino acids.

11. A composition comprising a peptide of any one of claims 1-10.

12. A composition comprising a plurality of peptides, each according to any one of claims 1-10.

13. The composition of claim 12, wherein each of the peptides overlap in sequence.

14. The composition of claim 12 or 13, wherein the plurality of peptides comprises 2, 3, 4, 5, 6 ,7, 8, 9, 10, 11, 12, 13, 14, 15, 16, 17, 18, 19, 20 or more peptides.

15. The isolated peptide of any one of claims 1-10 or the composition of any one of claims 11-14, for use as a medicament.
